(19) Europäisches Patentamt / European Patent Office / Office européen des brevets

(11) **EP 4 386 759 A1**

(12) **EUROPEAN PATENT APPLICATION**

(43) Date of publication:
**19.06.2024   Bulletin 2024/25**

(21) Application number: **23216667.8**

(22) Date of filing: **14.12.2023**

(51) International Patent Classification (IPC):
**G16B 5/00** (2019.01)     **G16B 20/00** (2019.01)

(52) Cooperative Patent Classification (CPC):
**G16B 20/00; G16B 5/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.12.2022   KR 20220174921**
**30.11.2023   KR 20230171559**

(71) Applicants:
• **POSTECH Research and Business Development Foundation**
**Pohang-si, Gyeongsangbuk-do 37666 (KR)**

• **Immunobiome Inc.**
**Pohang-si, Gyeongsangbuk-do 37666 (KR)**

(72) Inventors:
• **LEE, Kwang Hwan**
**02861 Seoul (KR)**
• **KIM, Sang Uk**
**37673 Pohang-si (KR)**
• **KIM, In Hae**
**37666 Pohang-si (KR)**
• **LEE, Ju Hun**
**37673 Pohang-si (KR)**

(74) Representative: **Manitz Finsterwald**
**Patent- und Rechtsanwaltspartnerschaft mbB**
**Martin-Greif-Strasse 1**
**80336 München (DE)**

(54) **A METHOD OF DISCOVERING NOVEL ANTICANCER DRUG USING CO-ESSENTIALITY NETWORK, AND AN APPARATUS THEREOF**

(57)     In the present disclosure, the present inventors validated the effectiveness of the co-essentiality network, constructed from the gene essentiality profile across cancer cells, as a robust platform for identifying anticancer targets. Furthermore, the co-essentiality network facilitated the drug repurposing not previously addressed by conventional molecular networks. These findings underline the value of co-essentiality networks in advancing precision oncology, offering new potential therapeutic avenues.

Fig. 1

**Description**

**TECHNICAL FIELD**

The present disclosure relates to a method of drawing novel anticancer drug using co-essentiality network, and an apparatus thereof.

**BACKGROUND**

Over the past few years, various anticancer drugs have been developed for diverse cancer types. However, the overall clinical efficacy of approved drugs remains limited. Thus, identifying targetable alterations is urgently needed for the success of anticancer therapy. To achieve precision oncology, diverse tasks must be performed, such as identifying driver genes and discovering drug targets in specific cancer types.

Network-based approaches support precision oncology to identify robust anticancer targets or biomarkers linked to known disease genes, since genes related to disease phenotypes cooperate and cluster in the network. The present inventors recently identified biomarkers of chemotherapy and immunotherapy by propagating the relatedness of therapeutic agents from drug targets to their neighbors in a protein-protein interaction (PPI) network. Cheng et al. developed an in-silico cancer drug repurposing framework using network modules derived from gene co-expression and PPI networks.

However, it remains to be seen which network is suitable for precision oncology, even though the choice of network is crucial to limit the performance of network-based approaches. It has been shown that network topology is a critical factor in improving the identification of disease genes. Huang et al. showed that the performance gap could be > 5,000× between the networks, from the highest to the lowest performance. Buphamalai et al. also reported that each network is relevance to specific tasks.

The Chinese patent No. 111128299 has provided a disclosure to diagnose colon cancer by constructing network using gene expression information, and the Chinese patent No. 110473591 has provided a disclosure to construct a network using gene co-expression information. Also, there were some cases of using co-essentiality network, such as finding proteome, but there have been no cases of using the same network to draw new anticancer drugs (including repurposing of conventional drugs).

**SUMMARY**

The problem to be solved by present disclosure includes, providing a method for drawing novel anticancer based on a co-essentiality network, which is more precise than another method based on another network. Rather than relying on a one-size-fits-all network, the present inventors designed and evaluated a network that performs purpose task better than a one-size-fits-all network.

The present disclosure provides a method of drawing novel anticancer using co-essentiality network thorough a computing device, comprising: (1) a process of collecting gene genome data, and constructing co-essentiality network through measuring the similarity between two genes; (2) a process of identifying cancer driver module from the co-essentiality network; and (3) a process of discovering novel anticancer using the cancer driver module.

The present disclosure also provides a device of discovering novel anticancer using co-essentiality network thorough a computing device, comprising: a collecting unit configured to collect gene genome data; a constructing unit configured to construct co-essentiality network through measuring the similarity between two genes; an identifying unit configured to identify cancer driver module from the co-essentiality network; and a discovering unit configured to discover novel anticancer using the cancer driver module.

The present inventors find that the co-essentiality network was able to prioritize cancer-type-specific therapeutic targets and discover drug-repurposing candidates. The co-essentiality links formed highly clustered network modules of potential therapeutic targets. Moreover, the co-essentiality network predicted more precise drug responses in cancer cells than other molecular networks. The present inventors anticipate that the co-essentiality network will be a valuable resource for precision oncology and provide new therapeutic opportunities for cancer patients.

**BRIEF DESCRIPTION OF THE DRAWINGS**

**Fig. 1** shows (A) a schematic illustration of constructing the co-essentiality network and its validation, (B) enrichment of the co-essentiality links to KEGG pathways. (C) enrichment of network links to 31 KEGG cancer-related pathways (CRPs) for four networks: the co-essentiality network, PPI network (BioGRID), co-expression network, and co-methylation network. (D) relative modularity of 15 pathways overlapped between the 31 CRPs and the 41 co-essentiality specific KEGG pathways.

**Fig. 2** shows curated gene set enrichment of 6 co-essentiality links.

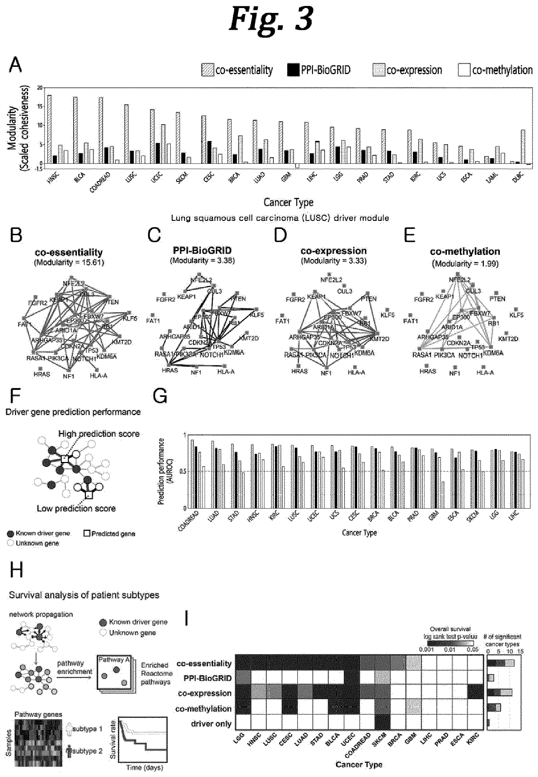

**Fig. 3** shows **(A)** modularity calculated from subnetwork of driver genes across 19 TCGA cancer types in four networks: co-essentiality, PPI-BioGRID, co-expression, and co-methylation. **(B-E)** illustrations of subnetwork of lung squamous cell carcinoma (LUSC) driver genes in each network. **(F)** a schematic illustration of driver gene identification using the co-essentiality network. **(G)** driver gene identification performance in four networks: co-essentiality; PPI-BioGRID; co-expression; co-methylation. **(H)** a schematic illustration of TCGA patient stratification using the co-essentiality network. **(I)** results for patient stratification using four networks and cancer drivers.

**Fig. 4** shows empirical distribution of modularity of the degree controlled random nodes for LUSC driver genes.

**Fig. 5** shows modularity of CGC cancer drivers in the four networks.

**Fig. 6** shows scaled clustering coefficient of cancer driver genes in the four networks.

**Fig. 7** shows correlation between the modularity of driver genes in the co-essentiality network and the number of cell lines used to construct the co-essentiality network.

**Fig. 8** shows performance of the co-essentiality network for cancer driver gene identification compared with that of seven different PPI networks.

**Fig. 9** shows performance of the co-essentiality network for cancer driver gene identification compared with that of another co-essentiality network and the genetic interaction network.

**Fig. 10** shows **(A)** schematic view of Hotnet2 algorithm. **(B)** a heatmap showing the rank of performance for driver gene identification among the 13 networks measured using MCC.

**Fig. 11** shows **(A)** schematic illustration of the uKIN algorithm. **(B)** A heatmap showing the rank of performance for driver gene identification among the 13 networks measured using ROAUC.

**Fig. 12** shows survival plot of patient subgroups in 16 cancer types stratified by the co-essentiality network.

**Fig. 13** shows survival plot of patient subgroups in 16 cancer types stratified by the PPI-BioGRID.

**Fig. 14** shows survival plot of patient subgroups in 16 cancer types stratified by the co-expression network.

**Fig. 15** shows survival plot of patient subgroups in 16 cancer types stratified by the co-methylation network.

**Fig. 16** shows survival plot of patient subgroups in 16 cancer types stratified by driver genes of each cancer type.

**Fig. 17** shows **(A)** subnetwork of the co-essentiality network for signaling by nonreceptor tyrosine kinase pathway. **(B)** heatmap of LUSC gene expression in Signaling by Non-Receptor Tyrosine Kinases pathway. **(C)** survival plot of LUSC patient subgroups stratified by gene expression of signaling by the nonreceptor tyrosine kinase pathway. **(D)** survival plot of LUSC patient subgroups stratified by LUSC driver genes.

**Fig. 18** shows **(A)** A schematic view of drug target prioritization using co-essentiality network and validation of prioritized targets. **(B)** Normalized enrichment score (NES) from GSEA of four networks: co-essentiality, PPI-BioGRID, co-expression, co-methylation. **(C)** Subnetwork of five approved targets(*CDKN1A, ATM, CRKL, SOX10,* and *RAF1*) in SKCM and driver genes in their first neighbors in the co-essentiality network. **(D)** first neighbors of *SOX10* in the four networks.

**Fig. 19** shows performance of the co-essentiality network for approved anticancer target prioritization compared with that of seven different PPI networks.

**Fig. 20** shows performance of the co-essentiality network for approved anticancer target prioritization compared with that of another co-essentiality network and genetic interaction network.

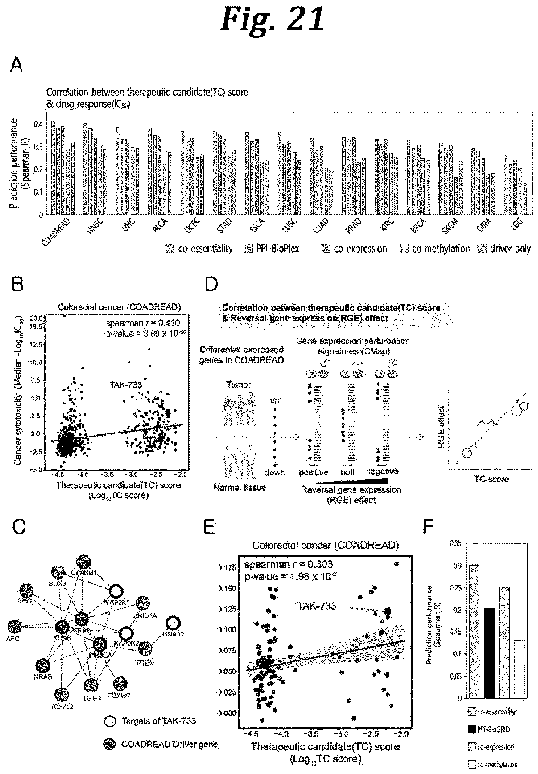

**Fig. 21** shows **(A)** prediction performance of drug response measured using the spearman correlation coefficient between TC score and median $-\log_{10}(IC_{50})$ for four networks: co-essentiality, PPI-BioGRID, co-expression, and co-methylation. **(B)** scatter plot of drug response and TC score of colorectal cancer in the co-essentiality network and data point of candidate drug: TAK-733. **(C)** a subnetwork of seven target genes of TAK-733 and COADREAD driver genes which are connected to them in the co-essentiality network. **(D)** schematic diagram of drug's reversal gene expression (RGE) effect on COADREAD. **(E)** scatter plot of drug's RGE effect and TC score of COADREAD in the co-essentiality network. **(F)** performance of the four networks for predicting drug RGE effect in COADREAD.

**Fig. 22** shows performance of the co-essentiality network for drug response prediction compared with that of seven different PPI networks.

**Fig. 23** shows performance of the co-essentiality network for drug response prediction compared with that of another co-essentiality network and genetic interaction network.

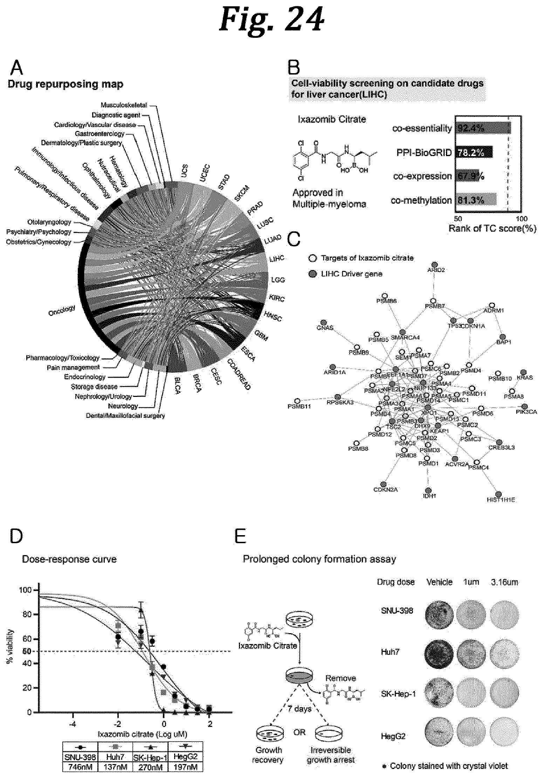

**Fig. 24** shows **(A)** a chord plot illustrating a global view of potential anticancer indications for 333 approved drugs across 17 cancer types. **(B)** rank percentile of TC score for ixazomib citrate in four networks: co-essentiality, PPI-BioGRID, co-expression, and co-methylation. **(C)** the co-essentiality links between target genes of ixazomib citrate and LIHC driver genes. **(D)** dose-response curves of ixazomib citrate in four LIHC cell lines (SNU-398, Huh7, SK-Hep-1 and HepG2). **(E)** schematic view of prolonged colony assay (Left). Negative effect of ixazomib citrate on the colony formation assay in four LIHC cell lines: SNU-398, Huh7, SK-Hep-1, and HepG2 (Right).

**Fig. 25** shows links between the targets of ixazomib citrate and LIHC driver genes in the four networks.

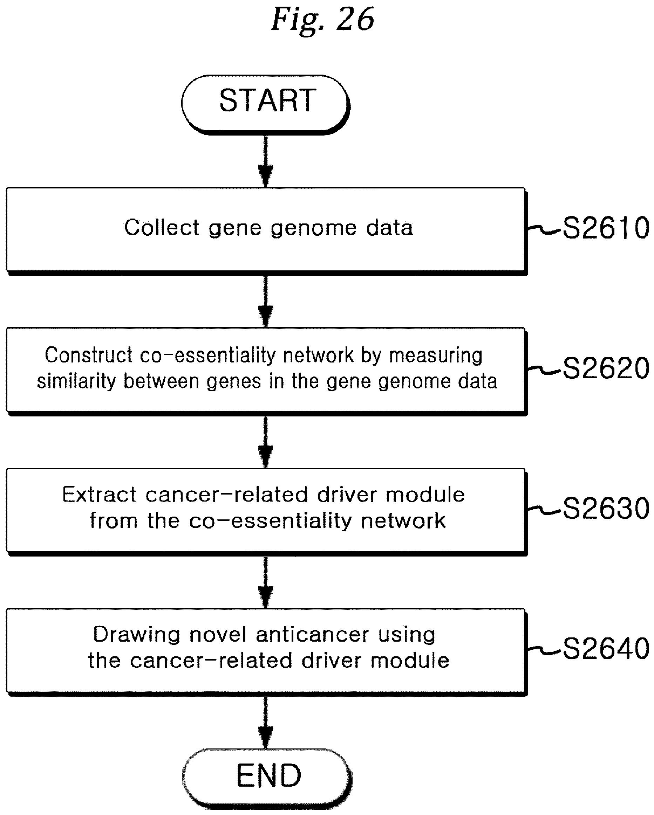

**Fig. 26** is a flow chart of a method according to present disclosure.

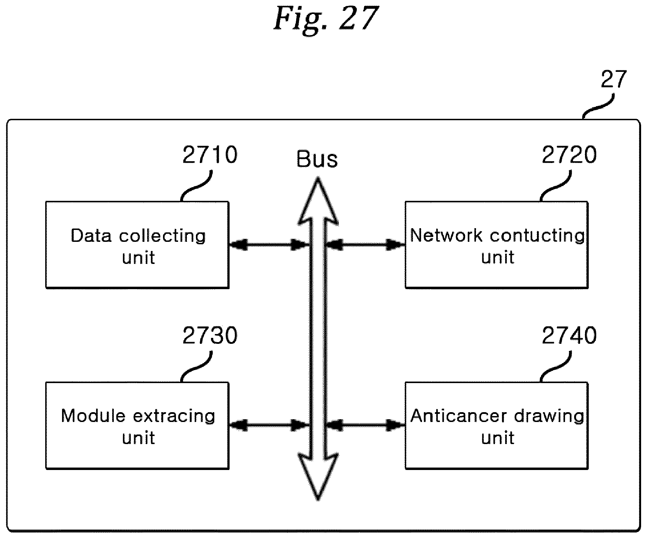

**Fig. 27** is a block diagram of an apparatus according to present disclosure.

## DETAILED DESCRIPTION

A Hereafter, embodiments of the present disclosure will be described in detail with reference to the accompanying drawings so that the present disclosure may be readily implemented by a person with ordinary skill in the art. However, it is to be noted that the present disclosure is not limited to the embodiments but may be embodied in various other ways. In drawings, parts irrelevant to the description are omitted for the simplicity of explanation, and like reference numerals denote like parts through the whole document.

Throughout the present document, when a member is positioned "on" another member, this includes not only when the member is in contact with the other member, but also when another member is present between the two members.

Throughout the present document, when a part "comprises" a component, this means that other components may be further included rather than excluding the other components unless there is a particular contrary description.

The terms "approximately" and "substantially" used throughout the document are used in or close to the figure when manufacturing and material tolerances unique to the mentioned meaning are presented and are used to prevent unscrupulous infringers from unfairly using the disclosure. The term "~(doing) step" or "step of~" to the extent used throughout the present specification does not mean "step for~".

Throughout this document, the term "their combination(s)" in the expression of the Markush type refers to one or more mixtures or combinations selected from the group of components described in the Markush type expression.

Throughout the present specification, the description of "A and/or B" means "A or B, or A and B".

Throughout the present specification, the term "essential" means the degree of perturbation on gene. For instance, when a gene is removed at a specific level, such as cell group or organism group, if it negatively affects the survival of that group, the gene can be expressed as "essential" for that group.

Throughout the present specification, the term "co-essentiality network" means a network comprising genes with similar knockout essentiality profiles across various cancer cell lines. While several co-essentiality networks have been used for gene function prediction, the benefit of using co-essentiality networks in drug discovery still needs to be assessed. For example, it is reported that if two genes have similar essentiality profiles, they tend to have similar biological functions. Co-essentiality networks have been applied to discover new functions of genes and to infer genes into the same functional complexes or metabolic pathways. However, attempts to identify cancer drug targets using co-essentiality networks have been limited to discovering surrogate targets for several challenging target protein cases, despite the therapeutic opportunity that the essentiality phenotype might possess.

In the present disclosure, the present inventors aimed to assess diverse in-silico frameworks using co-essentiality networks to identify novel anticancer drugs for specific cancer types and investigate the advantages of this network compared with conventional molecular networks. The present inventors find that the co-essentiality network was able to prioritize cancer-type-specific therapeutic targets and discover drug-repurposing candidates. The co-essentiality links formed highly clustered network modules of potential therapeutic targets. Moreover, the co-essentiality network predicted more precise drug responses in cancer cells than other molecular networks. The present inventors anticipate that the co-essentiality network will be a valuable resource for precision oncology and provide new therapeutic opportunities for cancer patients.

Throughout the present specification, the term "drug-repurposing" means a change of an use of conventional drug, which was for treatment of other disease, to treatment of another disease.

Throughout the present specification, the term "module" means a set of highly related nodes in a network, and "driver module" means a module which comprises cancer driver genes in co-essentiality network.

Throughout the present specification, the term "reversal gene expression" means that a pattern of gene expression of cell changes in reverse due to some factors, such as a drug.

Throughout the present specification, the term "prioritization" means process of setting priority to data or etc., according to its importance.

Throughout the present specification, the term "enrichment" means that a gene is furthermore expressed for a specific phenotype.

A Hereafter, embodiments of the present disclosure will be described in detail with reference to the accompanying drawings. However, it is to be noted that the present disclosure is not limited to the embodiments but may be embodied in various other ways.

**Example 1. Data, Constructing network, etc.**

**1-1. Resources and network information**

For all 13 networks used in the present disclosure, nodes in the networks were converted to HUGO symbols, while only the nodes and links included in the largest connected component were selected. The numbers of nodes and links of the final networks used in the present disclosure are reported in **table 1.**

[Table 1]

| Network | Node number | Link number |
|---|---|---|
| co-essentiality | 18,119 | 8,105,180 |
| co-expression | 19,120 | 12,759,793 |
| co-methylation | 16,333 | 10,193,089 |
| BioGRID | 18,708 | 434,527 |
| BioPlex | 14,523 | 167,384 |
| GPSnet | 15,124 | 167,854 |
| HURI | 8,124 | 51,816 |
| InBioMap | 17,421 | 608,161 |
| iRefIndex | 14,955 | 152,147 |
| Pathway Commons | 19,082 | 1,040,194 |
| STRING | 12,910 | 359,564 |
| Benchmark co-essentiality(PMID: 33859415) | 9,480 | 56,070 |
| cSLnet | 8,150 | 21,534 |

**1-2. Gene essentiality data**

To build a co-essentiality network, the present inventors used a dataset consisting of the genome-wide CRISPR screenings from the Achilles project 20q2 in the dependency map (DepMap) project. Using the dataset and cell line, the present inventors obtained a growth data that identified the effect caused in cell line growth, when some functions of a gene was knocked out. In here, the growth data means a data about essentiality of a gene. The present inventors used the data to construct co-essentiality network.

**1-3. Gene expression data**

To construct a co-expression network, the present inventors used CCLE expression data quantified from RNA-seq files using GTEx pipelines [18]. The dataset contains the gene expression data of 19,144 genes in 1,305 cell lines from 34 distinct lineages. Among the 19,144 genes, 23 with zero expression values across the cell lines were removed.

**1-4. Gene methylation data**

To construct a co-methylation network, the present inventors used CCLE DNA methylation reduced representation bisulfite sequencing data (promoter 1 kb upstream of TSS). The dataset consists of the methylation data of 21,337 loci covering 17,182 gene promoter regions in 843 cell lines. Due to the many missing values in the data matrix, the present inventors only held cell lines with methylation data of at least 17,000 loci and loci with methylation data in at least 644 cell lines. Finally, the present inventors incorporated 20,198 methylation loci from 805 cell lines into the network construction steps.

**1-5. Constructing the gene correlation-based network**

To construct three networks (a co-essentiality network, co-expression network, and co-methylation network) from the corresponding dataset, the present inventors measured the similarity in essentiality, expression, and methylation between two genes using corrected correlation and used this value as the link weight in the network. After correcting the data, if two genes had a link weight of 0, the present inventors filtered out that link. This procedure consists of three steps:

(i) For all missing values in the datasets, the present inventors conducted a k-nearest neighbor (KNN) imputation with k = 10 using the impyute Python module.

(ii) To measure the correlation between genes, the present inventors calculated the Pearson correlation coefficient (PCC) for all pairs of genes in the datasets and used the absolute value of PCC to capture both directions of correlation between genes.

(iii) For the absolute value of PCC, the present inventors applied the context likelihood relatedness (CLR) algorithm, which conducts adaptive background correction to eliminate false correlations and indirect influences. In particular,

$$CLR_{ij} = \sqrt{z_i^2 + z_j^2}$$

the PCC value between gene i and $j$, $r_{ij}$ was adjusted with the score where $z_i =$

$$\begin{cases} \frac{r_{ij}-\mu_i}{\sigma_i}, & \frac{r_{ij}-\mu_i}{\sigma_i} \geq t \\ 0, & \frac{r_{ij}-\mu_i}{\sigma_i} < t \end{cases}$$

, t is the threshold value for the $z_i$. The $\mu_i$ and $\sigma_i$ are, respectively the sample mean and standard deviation of the empirical distribution of $r_{ik}$, k = 1,...,n (n is the number of genes). To reduce the computation cost of network analyses without losing significant performance for identifying cancer driver genes, The present inventors selected $t$ = 2.0 among six threshold values from 0.0 to 5.0 **(Table 2).**

[Table 2]

| Treshold t | co-essentiality | co-expression | co-methylation |
|---|---|---|---|
| 0 | 0.815 | 0.737 | 0.583 |
| 1 | 0.823 | 0.747 | 0.583 |
| 2 | 0.814 | 0.758 | 0.583 |

(continued)

| Treshold t | co-essentiality | co-expression | co-methylation |
|---|---|---|---|
| 3 | 0.773 | 0.681 | 0.556 |
| 4 | 0.711 | 0.539 | 0.516 |
| 5 | 0.662 | 0.516 | 0.502 |

## 1-6. Preparation of PPI network

Eight human protein-protein interaction (PPI) networks were used: BioGRID [20], BioPlex, GPSnet, HURI, Inbiomap, iRefIndex, Pathway Commons, and STRING. For all PPI networks, the values of link weights were set to 1.0.

## 1-7. BioGRID

The present inventors downloaded the BioGRID interactome from https://thebiogrid.org/ under BIOGRID-4.1.190. The present inventors used the interactions between both proteins from Homo sapiens.

## 1-8. BioPlex

The present inventors downloaded the following BioPlex interactomes: BioPlex 3.0 Interactions (293T Cells) and BioPlex HCT116 v. 1.0 (HCT116 cells) (https://bioplex.hms.harvard.edu/interactions.php under release BioPlex 3.0. The present inventors constructed a single network from the union of both interactomes.

## 1-9. GPSnet

The present inventors used the GPSnet interactome previously constructed by Cheng et al., which assembled 15 commonly used databases with multiple experimental sources of evidence and an in-house systematic human protein-protein interactome. The interactome is publicly available at https://github.com/ChengF-Lab/GPSnet/tree/master/Data mat and file 'Net PPI.mat'. GPSnet was originally an *in-silico* framework for drug repurposing, and in the present disclosure, the present inventors called the PPI network used in this framework GPSnet.

## 1-10. HURI

The present inventors downloaded the HURI interactome from http://www.interactome-atlas.org/ and the file 'HuRI.tsv'.

## 1-11. InBioMap

The present inventors downloaded the InBioMap interactome from http://www.intomics.com/inbio/map and the file 'InBio_Map core_2016 09_12'.

## 1-12. iRefIndex

The present inventors downloaded the iRefIndex interactome from the web interface to the Interaction Reference Index repository (iRefWeb, https://wodaklab.org/iRefWeb/search/index) under the release iRefIndex version 13.0. The present inventors used four searching options: 'single organism interaction; 'Homo Sapiens', 'experimental', and 'physical'.

## 1-13. PathwayCommons

The present inventors downloaded the PathwayCommons interactome from http://www.pathwaycommons.org/archives/PC2/v12/ and the file 'PathwayCommons12.All.hgnc.txt'.

## 1-14. STRING

The present inventors downloaded the STRING interactome from https://string-db.org/ under the release v11.0.

To avoid co-citation information in STRING, the present inventors removed the text-mining scores for all links and recalculated the confidence score of STRING. Next, links with confidence scores > 700 were considered to leverage the high-confidence PPIs.

**1-15. Genetic interaction network**

To compare the co-essentiality network with the genetic interaction network based on a synthetic-lethal relationship, the present inventors used a clinically relevant synthetic lethality network built by the 'identification of clinically relevant synthetic lethality (ISLE)' approach. The present inventors downloaded 'clinically relevant synthetic lethality network (cSLnet)' interactome from https://github.com/jooslee/ISLE/tree/main/networks and the file 'ISLE_clinical_SL_network_FDR_0.2.cys'.

**1-16. Curated gene set enrichment analysis (GSEA) of co-essentiality network**

The present inventors calculated the enrichment of co-essentiality links in six curated gene sets. The present inventors downloaded six curated gene sets: molecular pathways (KEGG [1], REACTOME [2]) and Gene Ontology annotations [3] [GO: BP (Biological Process), MF (Molecular Function), and CC (Cellular Components)] from Molecular signatures database(MSigDB) [4], and human core protein complex from CORUM [5].

In the co-essentiality network, gene pairs were ranked by the link weights and grouped into cumulative bins of 10,000 pairs, and the enrichment was calculated using the ratio of pairs annotated with the same biological modules. The present inventors also measured enrichment expected by chance as the probability of finding the gene pairs within the same biological modules without being informed by co-essentiality links. Similar to the approach of Lee et al. [6], for the expected ratio, the present inventors changed the denominator from the number of co-essentiality links to all possible pairs between the genes given a bin.

**1-17. Modularity calculation**

The present inventors used two types of modularity measures as shown in the following function:

$$\text{cohesiveness} = \frac{\sum W_{in}}{\sum W_{all}} \ [34]$$

$$\text{clustering coefficient} = \frac{1}{n} \sum_{v \in G} \frac{2T_v}{k_v(k_v-1)}$$

where $W_{in}$ is the weight of links within query genes,

$W_{all}$ is the weight of all links connected to query genes,

n is the number of query genes,

$T_v$ is the number of triangles through node, and

$k_v$ is the degree of node v.

Since both modularity measures can be affected by the degree centrality of query nodes, the present inventors applied normalization to the modularity measures, which removes the degree bias. Similar to the approach of Guney et al. [7], the present inventors created a reference modularity distribution that corresponds to the expected modularity of 100 randomly selected groups of genes matching the size and degrees of query genes in the network. Next, modularity was normalized as the z-score of the observed modularity of genes computed from the reference distribution of modularity from random groups.

**1-18. Network enrichment to cancer related pathways (CRPs)**

Among the 186 KEGG pathways from MSigDB, the present inventors used 31 as CRPs, which included the related pathways of 'Pathways in cancer'(Pathway ID: hsa05200) and the pathways in 'Cancer: specific types'. A list of the 31 KEGG pathways is shown in Table 3.

[Table 3]

| Term | co-esse ntiali ty | ppi-Bio GRI D | co-expr essi on | co-meth ylati on | cancer related pathway(C RP) |
|---|---|---|---|---|---|
| KEGG_ABC_TRANSPORTERS | 0.26 3440 86 | 0.26 486 486 5 | 0.27 419 354 8 | 0.331 5217 39 | 0 |
| KEGG_ACUTE_MYELOID_LEUKE MIA | 0.79 5698 925 | 0.30 270 270 3 | 0.45 698 924 7 | 0.239 1304 35 | 1 |
| KEGG_ADHERENS_JUNCTION | 0.83 3333 333 | 0.4 | 0.39 247 311 8 | 0.847 8260 87 | 1 |
| KEGG_ADIPOCYTOKINE_SIGNALI NG_PATHWAY | 0.37 0967 742 | 0.69 729 729 7 | 0.17 204 301 1 | 0.179 3478 26 | 0 |
| KEGG_ALANINE_ASPARTATE_AN D_ GLUTAMATE_METABOLISM | 0.45 6989 247 | 0.28 108 108 1 | 0.05 913 978 5 | 0.108 6956 52 | 0 |
| KEGG_ALDOSTERONE_REGULAT ED_SODIUM_ REABSORPTION | 0.43 5483 871 | 0.49 189 189 2 | 0.16 129 032 3 | 0.119 5652 17 | 0 |
| KEGG_ALLOGRAFT_REJECTION | 0.58 0645 161 | 0.80 540 540 5 | 0.95 161 290 3 | 0.434 7826 09 | 0 |
| KEGG_ALPHA_LINOLENIC_ACID_ METABOLISM | 0.43 0107 527 | 0.05 405 405 4 | 0.09 677 419 4 | 0.206 5217 39 | 0 |
| KEGG_ALZHEIMERS_DISEASE | 0.97 3118 28 | 0.94 054 054 1 | 0.96 236 559 1 | 0.885 8695 65 | 0 |
| KEGG_AMINO_SUGAR_AND_NU CLEOTIDE_ SUGAR_METABOLISM | 0.79 0322 581 | 0.41 621 621 6 | 0.63 440 860 2 | 0.782 6086 96 | 0 |
| KEGG_AMINOACYL_TRNA_BIOSY NTHESIS | 0.86 0215 054 | 0.84 324 324 3 | 0.86 021 505 4 | 0.869 5652 17 | 0 |
| KEGG_AMYOTROPHIC_LATERAL_ SCLEROSIS_ ALS | 0.06 9892 473 | 0.33 513 513 5 | 0.04 838 709 7 | 0.684 7826 09 | 0 |
| KEGG_ANTIGEN_PROCESSING_A ND_ PRESENTATION | 0.91 9354 839 | 0.63 783 783 8 | 0.88 172 043 | 0.744 5652 17 | 0 |
| KEGG_APOPTOSIS | 0.68 2795 699 | 0.79 459 459 5 | 0.55 376 344 1 | 0.760 8695 65 | 1 |

(continued)

| Term | co-essentiality | ppi-BioGRID | co-expression | co-methylation | cancer related pathway(CRP) |
|---|---|---|---|---|---|
| KEGG_ARACHIDONIC_ACID_MET ABOLISM | 0.17 2043 011 | 0.40 540 540 5 | 0.60 752 688 2 | 0.798 9130 43 | 0 |
| KEGG_ARGININE_AND_PROLINE _METABOLISM | 0.31 7204 301 | 0.18 378 378 4 | 0.51 075 268 8 | 0.576 0869 57 | 0 |
| KEGG_ARRHYTHMOGENIC_RIGH T_ VENTRICULAR_CARDIOMYOPA THY_ARVC | 0.33 8709 677 | 0.45 945 945 9 | 0.5 | 0.826 0869 57 | 0 |
| KEGG_ASCORBATE_AND_ALDAR ATE_ METABOLISM | 0.5 | 0.43 243 243 2 | 0.66 666 666 7 | 0.402 1739 13 | 0 |
| KEGG_ASTHMA | 0.52 1505 376 | 0.42 702 702 7 | 0.90 322 580 6 |  | 0 |
| KEGG_AUTOIMMUNE_THYROID _DISEASE | 0.74 1935 484 | 0.93 513 513 5 | 0.91 935 483 9 | 0.282 6086 96 | 0 |
| KEGG _ AXON _ GUIDANCE | 0.47 3118 28 | 0.62 702 702 7 | 0.68 279 569 9 | 0.934 7826 09 | 0 |
| KEGG_B_CELL_RECEPTOR_SIGNA LING_ PATHWAY | 0.72 0430 108 | 0.56 756 756 8 | 0.73 655 914 | 0.597 8260 87 | 0 |
| KEGG_BASAL_CELL_CARCINOMA | 0.15 0537 634 | 0.50 270 270 3 | 0.50 537 634 4 | 0.945 6521 74 | 1 |
| KEGG_BASAL_TRANSCRIPTION_F ACTORS | 0.89 2473 118 | 0.97 297 297 3 | 0.46 774 193 5 | 0.505 4347 83 | 0 |
| KEGG_BASE_EXCISION_REPAIR | 0.45 1612 903 | 0.64 324 324 3 | 0.74 193 548 4 | 0.777 1739 13 | 0 |
| KEGG_BETA_ALANINE_METABOL ISM | 0.05 9139 785 | 0.21 081 081 1 | 0.18 817 204 3 | 0.070 6521 74 | 0 |
| KEGG_BIOSYNTHESIS_OF_UNSAT URATED_ FATTY_ACIDS | 0.41 3978 495 | 0.08 108 108 1 | 0.29 032 258 1 | 0.728 2608 7 | 0 |
| KEGG_BLADDER_CANCER | 0.60 2150 538 | 0.15 135 135 1 | 0.11 290 322 6 | 0.663 0434 78 | 1 |
| KEGG_BUTANOATE_METABOLIS M | 0.10 7526 882 | 0.20 540 540 5 | 0.40 860 215 1 | 0.027 1739 13 | 0 |

(continued)

| Term | co-essentiali ty | ppi-Bio GRI D | co-expr essi on | co-meth ylati on | cancer related pathway(C RP) |
|------|------|------|------|------|------|
| KEGG_CALCIUM_SIGNALING_PA THWAY | 0.37 6344 086 | 0.55 675 675 7 | 0.52 688 172 | 0.967 3913 04 | 1 |
| KEGG_CARDIAC_MUSCLE_CONT RACTION | 0.70 4301 075 | 0.86 486 486 5 | 0.72 580 645 2 | 0.048 9130 43 | 0 |
| KEGG_CELL_ADHESION_MOLEC ULES_CAMS | 0.39 2473 118 | 0.61 081 081 1 | 0.87 634 408 6 | 0.923 9130 43 | 0 |
| KEGG_CELL_CYCLE | 0.94 0860 215 | 0.76 756 756 8 | 0.91 397 849 5 | 0.972 8260 87 | 1 |
| KEGG_CHEMOKINE_SIGNALING_ PATHWAY | 0.73 1182 796 | 0.74 054 054 1 | 0.85 483 871 | 0.619 5652 17 | 0 |
| KEGG_CHRONIC_MYELOID_LEUK EMIA | 0.87 0967 742 | 0.32 432 432 4 | 0.30 645 161 3 | 0.793 4782 61 | 1 |
| KEGG_CIRCADIAN_RHYTHM_MA MMAL | 0.10 2150 538 | 0.83 783 783 8 | 0.37 096 774 2 | 0.75 | 0 |
| KEGG_CITRATE_CYCLE_TCA_CYC LE | 0.92 4731 183 | 0.8 | 0.77 956 989 2 | 0.614 1304 35 | 0 |
| KEGG_COLORECTAL_CANCER | 0.68 8172 043 | 0.41 081 081 1 | 0.31 182 795 7 | 0.831 5217 39 | 1 |
| KEGG_COMPLEMENT_AND_COA GULATION_ CASCADES | 0.18 2795 699 | 0.95 675 675 7 | 0.94 623 655 9 | 0.815 2173 91 | 0 |
| KEGG_CYSTEINE_AND_METHION INE_ METABOLISM | 0.34 4086 022 | 0.30 810 810 8 | 0.06 989 247 3 | 0.489 1304 35 | 0 |
| KEGG_CYTOKINE_CYTOKINE_REC EPTOR_ INTERACTION | 0.80 1075 269 | 0.92 972 973 | 0.97 311 828 | 0.983 6956 52 | 1 |
| KEGG_CYTOSOLIC_DNA_SENSIN G_PATHWAY | 0.69 3548 387 | 0.91 891 891 9 | 0.68 817 204 3 | 0.608 6956 52 | 0 |
| KEGG_DILATED_CARDIOMYOPAT HY | 0.24 7311 828 | 0.34 594 594 6 | 0.62 365 591 4 | 0.864 1304 35 | 0 |
| KEGG_DNA_REPLICATION | 0.93 5483 871 | 0.75 135 135 1 | 0.89 247 311 8 | 0.695 6521 74 | 0 |

(continued)

| Term | co-essentiality | ppi-BioGRID | co-expression | co-methylation | cancer related pathway(CRP) |
|---|---|---|---|---|---|
| KEGG_DORSO_VENTRAL_AXIS_F ORMATION | 0.38 1720 43 | 0.10 810 810 8 | 0.04 301 075 3 | 0.146 7391 3 | 0 |
| KEGG_DRUG_METABOLISM_CYT OCHROME_ P450 | 0.65 5913 978 | 0.85 945 945 9 | 0.83 333 333 3 | 0.092 3913 04 | 0 |
| KEGG_DRUG_METABOLISM_OTH ER_ENZYMES | 0.42 4731 183 | 0.64 864 864 9 | 0.49 462 365 6 | 0.032 6086 96 | 0 |
| KEGG_ECM_RECEPTOR_INTERAC TION | 0.23 1182 796 | 0.77 297 297 3 | 0.83 870 967 7 | 0.907 6086 96 | 1 |
| KEGG_ENDOCYTOSIS | 0.72 5806 452 | 0.82 162 162 2 | 0.54 838 709 7 | 0.581 5217 39 | 0 |
| KEGG_ENDOMETRIAL_CANCER | 0.88 7096 774 | 0.22 702 702 7 | 0.33 333 333 3 | 0.472 8260 87 | 1 |
| KEGG_EPITHELIAL_CELL_SIGNALI NG_IN_ HELICOBACTER_PYLORI_I NFECTION | 0.58 6021 505 | 0.72 432 432 4 | 0.32 795 698 9 | 0.516 3043 48 | 0 |
| KEGG_ERBB_SIGNALING_PATHW AY | 0.75 8064 516 | 0.59 459 459 5 | 0.21 505 376 3 | 0.494 5652 17 | 0 |
| KEGG_ETHER_LIPID_METABOLIS M | 0.16 1290 323 | 0.12 972 973 | 0.03 763 440 9 | 0.260 8695 65 | 0 |
| KEGG_FATTY_ACID_ METABOLIS M | 0.08 0645 161 | 0.31 891 891 9 | 0.60 215 053 8 | 0.396 7391 3 | 0 |
| KEGG_FC_EPSILON_RI_SIGNALIN G_PATHWAY | 0.67 7419 355 | 0.73 513 513 5 | 0.43 548 387 1 | 0.646 7391 3 | 0 |
| KEGG_FC_GAMMA_R_MEDIATE D_ PHAGOCYTOSIS | 0.65 0537 634 | 0.78 378 378 4 | 0.58 602 150 5 | 0.407 6086 96 | 0 |
| KEGG_FOCAL_ADHESION | 0.84 9462 366 | 0.71 351 351 4 | 0.79 032 258 1 | 0.755 4347 83 | 1 |
| KEGG_FOLATE_BIOSYNTHESIS | 0.17 7419 355 | 0.16 216 216 2 | 0.03 225 806 5 | 0.423 9130 43 | 0 |
| KEGG_FRUCTOSE_AND_MANNO SE_ METABOLISM | 0.27 9569 892 | 0.39 459 459 5 | 0.41 935 483 9 | 0.478 2608 7 | 0 |

(continued)

| Term | co-essentiali ty | ppi-Bio GRI D | co-expr essi on | co-meth ylati on | cancer related pathway(C RP) |
|------|------|------|------|------|------|
| KEGG_GALACTOSE_METABOLIS M | 0.12 3655 914 | 0.31 351 351 4 | 0.19 892 473 1 | 0.271 7391 3 | 0 |
| KEGG_GAP_JUNCTION | 0.49 4623 656 | 0.45 405 405 4 | 0.22 043 010 8 | 0.842 3913 04 | 0 |
| KEGG_GLIOMA | 0.80 6451 613 | 0.42 162 162 2 | 0.10 752 688 2 | 0.336 9565 22 | 1 |
| KEGG_GLUTATHIONE_METABOLI SM | 0.46 2365 591 | 0.6 | 0.51 612 903 2 | 0.163 0434 78 | 0 |
| KEGG_GLYCEROLIPID_METABOLI SM | 0.36 0215 054 | 0.04 324 324 3 | 0.10 215 053 8 | 0.5 | 0 |
| KEGG_GLYCEROPHOSPHOLIPID_ METABOLISM | 0.22 0430 108 | 0.07 027 027 | 0.13 978 494 6 | 0.190 2173 91 | 0 |
| KEGG_GLYCINE_SERINE_AND_TH REONINE_ METABOLISM | 0.00 5376 344 | 0.12 432 432 4 | 0.40 322 580 6 | 0.016 3043 48 | 0 |
| KEGG_GLYCOLYSIS_GLUCONEOG ENESIS | 0.40 8602 151 | 0.81 621 621 6 | 0.66 129 032 3 | 0.304 3478 26 | 0 |
| KEGG_GLYCOSAMINOGLYCAN_BI OSYNTHESIS_ CHONDROITIN_SUL FATE | 0.20 4301 075 | 0.53 513 513 5 | 0.56 989 247 3 | 0.733 6956 52 | 0 |
| KEGG_GLYCOSAMINOGLYCAN_BI OSYNTHESIS_ HEPARAN_SULFATE | 0.77 9569 892 | 0.08 648 648 6 | 0.12 903 225 8 | 0.554 3478 26 | 0 |
| KEGG_GLYCOSAMINOGLYCAN_BI OSYNTHESIS_ KERATAN_SULFATE | 0.05 3763 441 | 0.11 351 351 4 | 0.11 827 957 | 0.135 8695 65 | 0 |
| KEGG_GLYCOSAMINOGLYCAN_D EGRADATION | 0.19 3548 387 | 0.2 | 0.48 924 731 2 | 0.173 9130 43 | 0 |
| KEGG_GLYCOSPHINGOLIPID_BIO SYNTHESIS_ GANGLIO_SERIES | 0.18 8172 043 | 0.13 513 513 5 | 0.18 279 569 9 | 0.233 6956 52 | 0 |
| KEGG_GLYCOSPHINGOLIPID_BIO SYNTHESIS_ GLOBO_SERIES | 0.11 2903 226 | 0.09 189 189 2 | 0.06 451 612 9 | 0.141 3043 48 | 0 |
| KEGG_GLYCOSPHINGOLIPID_BIO SYNTHESIS_ LACTO_AND_NEOLA CTO_SERIES | 0.30 1075 269 | 0.01 621 621 6 | 0.31 720 430 1 | 0.184 7826 09 | 0 |

(continued)

| Term | co-esse ntiali ty | ppi- Bio GRI D | co-expr essi on | co-meth ylati on | cancer related pathway(C RP) |
|---|---|---|---|---|---|
| KEGG_GLYCOSYLPHOSPHATIDYLI NOSITOL_ GPI_ANCHOR_BIOSYN THESIS | 0.96 2365 591 | 0.92 432 432 4 | 0.52 150 537 6 | 0.461 9565 22 | 0 |
| KEGG_GLYOXYLATE_AND_DICAR BOXYLATE_ METABOLISM | 0.16 6666 667 | 0.06 486 486 5 | 0.20 430 107 5 | 0.521 7391 3 | 0 |
| KEGG_GNRH_SIGNALING_PATH WAY | 0.41 9354 839 | 0.49 729 729 7 | 0.08 064 516 1 | 0.315 2173 91 | 0 |
| KEGG_GRAFT_VERSUS_HOST_DI SEASE | 0.63 4408 602 | 0.83 243 243 2 | 0.93 548 387 1 | 0.559 7826 09 | 0 |
| KEGG_HEDGEHOG_SIGNALING_ PATHWAY | 0.14 5161 29 | 0.52 972 973 | 0.41 397 849 5 | 0.951 0869 57 | 1 |
| KEGG_HEMATOPOIETIC_CELL_LI NEAGE | 0.32 7956 989 | 0.55 135 135 1 | 0.94 086 021 5 | 0.896 7391 3 | 0 |
| KEGG_HISTIDINE_METABOLISM | 0.09 1397 849 | 0.14 594 594 6 | 0.15 591 397 8 | 0.364 1304 35 | 0 |
| KEGG_HOMOLOGOUS_RECOMBI NATION | 0.84 4086 022 | 0.70 270 270 3 | 0.82 795 698 9 | 0.592 3913 04 | 0 |
| KEGG_HUNTINGTONS_DISEASE | 0.96 7741 935 | 0.89 729 729 7 | 0.96 774 193 5 | 0.940 2173 91 | 0 |
| KEGG_HYPERTROPHIC_CARDIO MYOPATHY_ HCM | 0.32 2580 645 | 0.32 972 973 | 0.64 516 129 | 0.788 0434 78 | 0 |
| KEGG_INOSITOL_PHOSPHATE_M ETABOLISM | 0.29 5698 925 | 0.09 729 729 7 | 0.15 053 763 4 | 0.445 6521 74 | 0 |
| KEGG_INSULIN_SIGNALING_PAT HWAY | 0.63 9784 946 | 0.68 648 648 6 | 0.38 709 677 4 | 0.543 4782 61 | 0 |
| KEGG_INTESTINAL_IMMUNE_NE TWORK_FOR_ IGA_PRODUCTION | 0.39 7849 462 | 0.37 297 297 3 | 0.90 860 215 1 | 0.679 3478 26 | 0 |
| KEGG_JAK_STAT_SIGNALING_PAT HWAY | 0.69 8924 731 | 0.88 648 648 6 | 0.80 107 526 9 | 0.527 1739 13 | 1 |
| KEGG_LEISHMANIA_INFECTION | 0.52 6881 72 | 0.48 648 648 6 | 0.84 408 602 2 | 0.565 2173 91 | 0 |

(continued)

| Term | co-essentiality | ppi-BioGRID | co-expression | co-methylation | cancer related pathway(CRP) |
|---|---|---|---|---|---|
| KEGG_LEUKOCYTE_TRANSENDO THELIAL_MIGRATION | 0.537634409 | 0.675675676 | 0.672043011 | 0.673913043 | 0 |
| KEGG_LIMONENE_AND_PINENE DEGRADATION | 0.016129032 | 0.032432432 | 0.284946237 | 0.043478261 | 0 |
| KEGG _LINOLEIC_ACID_METABOL ISM | 0.306451613 | 0.891891892 | 0.559139785 | | 0 |
| KEGG_LONG_TERM_DEPRESSIO N | 0.284946237 | 0.383783784 | 0.134408602 | 0.880434783 | 0 |
| KEGG_LONG_TERM_POTENTIATI ON | 0.365591398 | 0.27027027 | 0.209677419 | 0.413043478 | 0 |
| KEGG_LYSINE_DEGRADATION | 0.064516129 | 0.156756757 | 0.241935484 | 0.60326087 | 0 |
| KEGG_LYSOSOME | 0.387096774 | 0.762162162 | 0.930107527 | 0.690217391 | 0 |
| KEGG_MAPK_SIGNALING_PATH WAY | 0.715053763 | 0.708108108 | 0.462365591 | 0.625 | 0 |
| KEGG_MATURITY_ONSET_DIABE TES_OF_THE_ YOUNG | 0.215053763 | 0.172972973 | 0.720430108 | 0.820652174 | 0 |
| KEGG_MELANOGENESIS | 0.274193548 | 0.248648649 | 0.252688172 | 0.918478261 | 0 |
| KEGG_MELANOMA | 0.736655914 | 0.362162162 | 0.086021505 | 0.456521739 | 1 |
| KEGG_METABOLISM_OF_XENOB IOTICS_BY_ CYTOCHROM_E_P450 | 0.564516129 | 0.827027027 | 0.887096774 | 0.326086957 | 0 |
| KEGG_MISMATCH_REPAIR | 0.629032258 | 0.789189189 | 0.758064516 | 0.722826087 | 0 |
| KEGG_MTOR_SIGNALING_PATH WAY | 0.897849462 | 0.691891892 | 0.177419355 | 0.10326087 | 1 |
| KEGG_N_GLYCAN_BIOSYNTHESIS | 0.951612903 | 0.259459459 | 0.731182796 | 0.570652174 | 0 |

(continued)

| Term | co-essentiality | ppi-BioGRID | co-expression | co-methylation | cancer related pathway(CRP) |
|---|---|---|---|---|---|
| KEGG_NATURAL_KILLER_CELL_M EDIATED_ CYTOTOXICITY | 0.76 8817 204 | 0.77 837 837 8 | 0.86 559 139 8 | 0.804 3478 26 | 0 |
| KEGG_NEUROACTIVE_LIGAND_R ECEPTOR_ INTERACTION | 0.54 3010 753 | 0.43 783 783 8 | 0.84 946 236 6 | 1 | 0 |
| KEGG_NEUROTROPHIN_SIGNALI NG_PATHWAY | 0.81 1827 957 | 0.65 405 405 4 | 0.61 827 957 | 0.668 4782 61 | 0 |
| KEGG_NICOTINATE_AND_NICOTI NAMIDE_ METABOLISM | 0.04 8387 097 | 0.01 081 081 1 | 0.02 150 537 6 | 0.201 0869 57 | 0 |
| KEGG_NITROGEN_METABOLISM | 0.20 9677 419 | 0.04 864 864 9 | 0.09 139 784 9 | 0.266 3043 48 | 0 |
| KEGG_NOD_LIKE_RECEPTOR_SIG NALING_ PATHWAY | 0.50 5376 344 | 0.67 027 027 | 0.59 139 784 9 | 0.369 5652 17 | 0 |
| KEGG_NON_HOMOLOGOUS_EN D_JOINING | 0.35 4838 71 | 0.38 918 918 9 | 0.26 344 086 | 0.451 0869 57 | 0 |
| KEGG_NON_SMALL_CELL_LUNG _CANCER | 0.82 2580 645 | 0.44 324 324 3 | 0.33 870 967 7 | 0.391 3043 48 | 1 |
| KEGG_NOTCH_SIGNALING_PATH WAY | 0.60 7526 882 | 0.50 810 810 8 | 0.14 516 129 | 0.548 9130 43 | 1 |
| KEGG_NUCLEOTIDE_EXCISION_R EPAIR | 0.74 7311 828 | 0.87 027 027 | 0.70 967 741 9 | 0.836 9565 22 | 0 |
| KEGG_O_GLYCAN_BIOSYNTHESIS | 0.03 7634 409 | 0.24 324 324 3 | 0.44 623 655 9 | 0.195 6521 74 | 0 |
| KEGG_OLFACTORY_TRANSDUCTI ON | 0.97 8494 624 | 0.23 243 243 2 | 0.92 473 118 3 | 0.956 5217 39 | 0 |
| KEGG_ONE_CARBON_POOL_BY_ FOLATE | 0.61 8279 57 | 0.11 891 891 9 | 0.38 172 043 | 0.358 6956 52 | 0 |
| KEGG_OOCYTE_MEIOSIS | 0.66 1290 323 | 0.56 216 216 2 | 0.77 419 354 8 | 0.385 8695 65 | 0 |
| KEGG_OTHER_GLYCAN_DEGRAD ATION | 0.33 3333 333 | 0.28 648 648 6 | 0.61 290 322 6 | 0.086 9565 22 | 0 |

(continued)

| Term | co-essentiality | ppi-BioGRID | co-expression | co-methylation | cancer related pathway(CRP) |
|---|---|---|---|---|---|
| KEGG_OXIDATIVE_PHOSPHORYL ATION | 1 | 0.98 378 378 4 | 0.99 462 365 6 | 0.913 0434 78 | 0 |
| KEGG_P53_SIGNALING_PATHWA Y | 0.51 0752 688 | 0.57 837 837 8 | 0.53 763 440 9 | 0.711 9565 22 | 1 |
| KEGG_PANCREATIC_CANCER | 0.70 9677 419 | 0.37 837 837 8 | 0.34 946 236 6 | 0.706 5217 39 | 1 |
| KEGG_PANTOTHENATE_AND_CO A_ BIOSYNTHESIS | 0.11 8279 57 | 0.02 162 162 2 | 0.01 075 268 8 | 0.211 9565 22 | 0 |
| KEGG_PARKINSONS_DISEASE | 0.98 9247 312 | 0.96 216 216 2 | 0.98 924 731 2 | 0.902 1739 13 | 0 |
| KEGG_PATHOGENIC_ESCHERICHI A_COLI_ INFECTION | 0.64 5161 29 | 0.29 729 729 7 | 0.36 559 139 8 | 0.309 7826 09 | 0 |
| KEGG_PATHWAYS_IN_CANCER | 0.83 8709 677 | 0.54 594 594 6 | 0.65 591 397 8 | 0.853 2608 7 | 1 |
| KEGG_PENTOSE_AND_GLUCURO NATE_ INTERCONVERSIONS | 0.48 3870 968 | 0.71 891 891 9 | 0.63 978 494 6 | 0.255 4347 83 | 0 |
| KEGG_PENTOSE_PHOSPHATE_PA THWAY | 0.55 9139 785 | 0.68 108 108 1 | 0.39 784 946 2 | 0.277 1739 13 | 0 |
| KEGG_PEROXISOME | 0.88 1720 43 | 0.90 810 810 8 | 0.56 451 612 9 | 0.635 8695 65 | 0 |
| KEGG_PHENYLALANINE_METAB OLISM | 0.07 5268 817 | 0.19 459 459 5 | 0.19 354 838 7 | 0.157 6086 96 | 0 |
| KEGG_PHOSPHATIDYLINOSITOL_ SIGNALING_ SYSTEM | 0.40 3225 806 | 0.21 621 621 6 | 0.27 956 989 2 | 0.168 4782 61 | 0 |
| KEGG_PORPHYRIN_AND_CHLOR OPHYLL_ METABOLISM | 0.77 4193 548 | 0.03 783 783 8 | 0.57 526 881 7 | 0.380 4347 83 | 0 |
| KEGG_PPAR_SIGNALING_PATHW AY | 0.25 2688 172 | 0.17 837 837 8 | 0.59 677 419 4 | 0.429 3478 26 | 1 |
| KEGG_PRIMARY_BILE_ACID_BIO SYNTHESIS | 0.24 1935 484 | 0.22 162 162 2 | 0.23 655 914 | 0.130 4347 83 | 0 |

(continued)

| Term | co-esse ntiali ty | ppi-Bio GRI D | co-expr essi on | co-meth ylati on | cancer related pathway(C RP) |
|---|---|---|---|---|---|
| KEGG_PRIMARY_IMMUNODEFIC IENCY | 0.13 9784 946 | 0.51 351 351 4 | 0.87 096 774 2 | 0.809 7826 09 | 0 |
| KEGG_PRION_DISEASES | 0.02 1505 376 | 0.23 783 783 8 | 0.23 118 279 6 | 0.152 1739 13 | 0 |
| KEGG_PROGESTERONE_MEDIAT ED_OOCYTE_ MATURATION | 0.55 3763 441 | 0.74 594 594 6 | 0.43 010 752 7 | 0.065 2173 91 | 0 |
| KEGG_PROPANOATE_METABOLIS M | 0.15 5913 978 | 0.66 486 486 5 | 0.53 225 806 5 | 0.021 7391 3 | 0 |
| KEGG_PROSTATE_CANCER | 0.85 4838 71 | 0.27 567 567 6 | 0.25 806 451 6 | 0.532 6086 96 | 1 |
| KEGG_PROTEASOME | 0.94 6236 559 | 0.99 459 459 5 | 0.95 698 924 7 | 0.858 6956 52 | 0 |
| KEGG_PROTEIN_EXPORT | 0.81 7204 301 | 0.35 675 675 7 | 0.70 430 107 5 | 0.641 3043 48 | 0 |
| KEGG_PROXIMAL_TUBULE_BICA RBONATE_ RECLAMATION | 0.02 6881 72 | 0.16 756 756 8 | 0.07 526 881 7 | 0.114 1304 35 | 0 |
| KEGG_PURINE_METABOLISM | 0.61 2903 226 | 0.94 594 594 6 | 0.69 354 838 7 | 0.244 5652 17 | 0 |
| KEGG_PYRIMIDINE_METABOLIS M | 0.82 7956 989 | 0.95 135 135 1 | 0.80 645 161 3 | 0.766 3043 48 | 0 |
| KEGG_PYRUVATE_METABOLISM | 0.13 4408 602 | 0.58 918 918 9 | 0.45 161 290 3 | 0.320 6521 74 | 0 |
| KEGG_REGULATION_OF_ACTIN_ CYTOSKELETON | 0.91 3978 495 | 0.81 081 081 1 | 0.62 903 225 8 | 0.483 6956 52 | 0 |
| KEGG_REGULATION_OF_AUTOP HAGY | 0.87 6344 086 | 0.96 756 756 8 | 0.34 408 602 2 | 0.005 4347 83 | 0 |
| KEGG_RENAL_CELL_CARCINOMA | 0.90 3225 806 | 0.34 054 054 1 | 0.48 387 096 8 | 0.510 8695 65 | 1 |
| KEGG_RENIN_ANGIOTENSIN_SYS TEM | 0.03 2258 065 | 0.35 135 135 1 | 0.02 688 172 | 0.440 2173 91 | 0 |

(continued)

| Term | co-essentiality | ppi-BioGRID | co-expression | co-methylation | cancer related pathway(CRP) |
|---|---|---|---|---|---|
| KEGG_RETINOL_METABOLISM | 0.623655914 | 0.913513514 | 0.811827957 | 0.07608697 | 0 |
| KEGG_RIBOFLAVIN_METABOLIS M | 0.0967741 94 | 0.102702703 | 0.0161290 32 | 0.097826087 | 0 |
| KEGG_RIBOSOME | 0.9946236 56 | 0.989189189 | 1 | 0.961956522 | 0 |
| KEGG_RIG_I_LIKE_RECEPTOR_SI GNALING_ PATHWAY | 0.9086021 51 | 0.72972973 | 0.7473118 28 | 0.65217391 3 | 0 |
| KEGG_RNA_DEGRADATION | 0.9301075 27 | 0.8810810 81 | 0.76344086 | 0.9782608 7 | 0 |
| KEGG_RNA_POLYMERASE | 0.8655913 98 | 1 | 0.6774193 55 | 0.53804347 8 | 0 |
| KEGG_SELENOAMINO_ACID_ME TABOLISM | 0.3494623 66 | 0.36756756 8 | 0.2258064 52 | 0.28804347 8 | 0 |
| KEGG_SMALL_CELL_LUNG_CANC ER | 0.763440 86 | 0.29189189 2 | 0.29569892 5 | 0.65760869 6 | 1 |
| KEGG_SNARE_INTERACTIONS_IN _VESICULAR_ TRANSPORT | 0.2365591 4 | 0.978378378 | 0.4408602 15 | 0.29347826 1 | 0 |
| KEGG_SPHINGOLIPID_METABOLI SM | 0.2258064 52 | 0.02702702 7 | 0.3225806 45 | 0.34239130 4 | 0 |
| KEGG_SPLICEOSOME | 0.9569892 47 | 0.902702703 | 0.9784946 24 | 0.98913043 5 | 0 |
| KEGG_STARCH_AND_SUCROSE_ METABOLISM | 0.5161290 32 | 0.62162162 2 | 0.58064516 1 | 0.29891304 3 | 0 |
| KEGG_STEROID_BIOSYNTHESIS | 0.6666666 67 | 0.005405405 | 0.79569892 5 | 0.05978260 9 | 0 |
| KEGG_STEROID_HORMONE_BIO SYNTHESIS | 0.4408602 15 | 0.854054054 | 0.69892473 1 | 0.46739130 4 | 0 |
| KEGG_SULFUR_METABOLISM | 0.1989247 31 | 0.572972973 | 0.16666666 7 | 0.71739130 4 | 0 |

(continued)

| Term | co-esse ntiali ty | ppi-Bio GRI D | co-expr essi on | co-meth ylati on | cancer related pathway(C RP) |
|---|---|---|---|---|---|
| KEGG_SYSTEMIC_LUPUS_ERYTH EMATOSUS | 0.98 3870 968 | 0.65 945 945 9 | 0.98 387 096 8 | 0.994 5652 17 | 0 |
| KEGG_T_CELL_RECEPTOR_SIGNA LING_ PATHWAY | 0.47 8494 624 | 0.84 864 864 9 | 0.76 881 720 4 | 0.701 0869 57 | 0 |
| KEGG_TASTE_TRANSDUCTION | 0.59 1397 849 | 0.48 108 108 1 | 0.75 268 817 2 | 0.586 9565 22 | 0 |
| KEGG_TAURINE_AND_HYPOTAU RINE_ METABOLISM | 0.01 0752 688 | | 0.00 537 634 4 | 0.038 0434 78 | 0 |
| KEGG_TERPENOID_BACKBONE_ BIOSYNTHESIS | 0.54 8387 097 | 0.18 918 918 9 | 0.65 053 763 4 | 0.739 1304 35 | 0 |
| KEGG_TGF_BETA_SIGNALING_PA THWAY | 0.48 9247 312 | 0.63 243 243 2 | 0.47 311 828 | 0.418 4782 61 | 1 |
| KEGG_THYROID_CANCER | 0.56 9892 473 | 0.05 945 945 9 | 0.05 376 344 1 | 0.228 2608 7 | 1 |
| KEGG_TIGHT_JUNCTION | 0.59 6774 194 | 0.47 567 567 6 | 0.54 301 075 3 | 0.875 | 0 |
| KEGG_TOLL_LIKE_RECEPTOR_SIG NALING_ PATHWAY | 0.75 2688 172 | 0.87 567 567 6 | 0.81 720 430 1 | 0.630 4347 83 | 0 |
| KEGG_TRYPTOPHAN_METABOLIS M | 0.08 6021 505 | 0.14 054 054 1 | 0.35 483 871 | 0.217 3913 04 | 0 |
| KEGG_TYPE_I_DIABETES_MELLIT US | 0.57 5268 817 | 0.61 621 621 6 | 0.89 784 946 2 | 0.347 8260 87 | 0 |
| KEGG_TYPE_II_DIABETES_MELLI TUS | 0.29 0322 581 | 0.54 054 054 1 | 0.24 731 182 8 | 0.25 | 0 |
| KEGG_TYROSINE_METABOLISM | 0.12 9032 258 | 0.25 405 405 4 | 0.37 634 408 6 | 0.054 3478 26 | 0 |
| KEGG_UBIQUITIN_MEDIATED_P ROTEOLYSIS | 0.78 4946 237 | 0.51 891 891 9 | 0.78 494 623 7 | 0.929 3478 26 | 0 |
| KEGG_VALINE_LEUCINE_AND_IS OLEUCINE_ BIOSYNTHESIS | 0.26 8817 204 | 0.07 567 567 6 | 0.30 107 526 9 | 0.125 | 0 |

(continued)

| Term | co-essentiali ty | ppi-Bio GRI D | co-expr essi on | co-meth ylati on | cancer related pathway(C RP) |
|---|---|---|---|---|---|
| KEGG_VALINE_LEUCINE_AND_IS OLEUCINE_ DEGRADATION | 0.04 3010 753 | 0.58 378 378 4 | 0.71 505 376 3 | 0.375 | 0 |
| KEGG_VASCULAR_SMOOTH_MU SCLE_ CONTRACTION | 0.25 8064 516 | 0.60 540 540 5 | 0.36 021 505 4 | 0.771 7391 3 | 0 |
| KEGG_VASOPRESSIN_REGULATE D_WATER_ REABSORPTION | 0.31 1827 957 | 0.52 432 432 4 | 0.26 881 720 4 | 0.010 8695 65 | 0 |
| KEGG_VEGF_SIGNALING_PATHW AY | 0.53 2258 065 | 0.46 486 486 5 | 0.12 365 591 4 | 0.353 2608 7 | 1 |
| KEGG_VIBRIO_CHOLERAE_INFEC TION | 0.67 2043 011 | 0.75 675 675 7 | 0.47 849 462 4 | 0.222 8260 87 | 0 |
| KEGG_VIRAL_MYOCARDITIS | 0.46 7741 935 | 0.44 864 864 9 | 0.82 258 064 5 | 0.081 5217 39 | 0 |
| KEGG_WNT_SIGNALING_PATHW AY | 0.44 6236 559 | 0.47 027 027 | 0.42 473 118 3 | 0.891 3043 48 | 1 |

For 186 KEGG pathways, the present inventors calculated relative modularity by converting scaled modularity calculated using cohesiveness to rank percentile scores in four networks: the co-essentiality network, PPI network (BioGRID), co-expression network, and co-methylation network. Next, the present inventors defined network-specific pathways by assigning the type of network to each of the 186 KEGG pathways, according to which network showed the highest relative modularity. The relative modularity values are presented in **Table 3.**

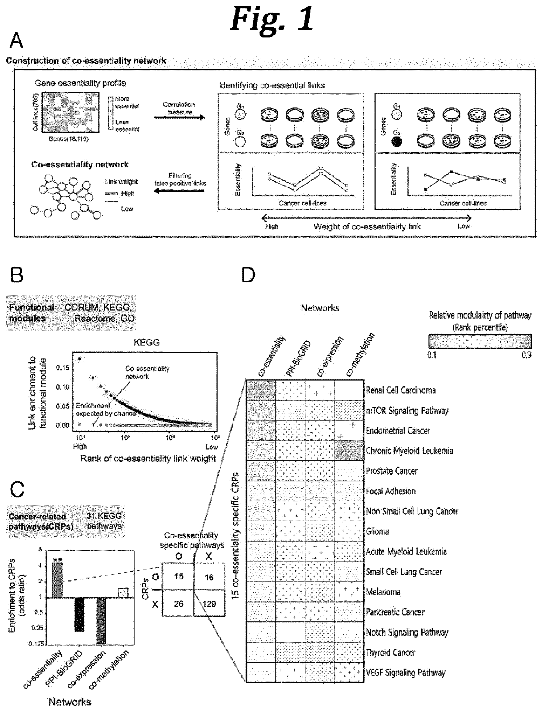

Finally, for each network, the present inventors constructed a 2×2 contingency table with four types of pathways namely network-specific CRPs, non-network-specific CRPs, network-specific non-CRPs, and non-network-specific non-CRPs. From the contingency table, the present inventors calculated the odds ratio of network enrichment to CRPs and determined the statistical significance of enrichment by calculating the p-value from Fisher's exact test. The contingency table of the co-essentiality network is shown in the **Fig. 1C.**

### 1-19. Driver genes of each cancer type

(1) Bailey et al. [8]

The present inventors retrieved driver genes of each cancer type from Bailey et al., who reported 299 driver genes across 33 cancer types. For modularity analysis, the present inventors selected 19 cancer types with more than 10 reported driver genes included in the co-essentiality network: bladder urothelial carcinoma (BLCA), breast invasive carcinoma (BRCA), cervical squamous cell carcinoma and endocervical adenocarcinoma (CESC), Colorectal adenocarcinoma (COADREAD), lymphoid neoplasm diffuse large B-cell lymphoma (DLBC), esophageal carcinoma (ESCA), glioblastoma multiforme (GBM), head and neck squamous cell carcinoma (HNSC), kidney renal clear cell carcinoma (KIRC), acute myeloid leukemia (LAML), brain lower grade glioma (LGG), liver hepatocellular carcinoma(LIHC), lung adenocarcinoma (LUAD), lung squamous cell carcinoma (LUSC), prostate adenocarcinoma (PRAD), skin cutaneous melanoma (SKCM), stomach adenocarcinoma (STAD), Uterine Corpus Endometrial carcinoma (UCEC).

(2) Cancer Gene Census (CGC)

For further validation of the high modularity of driver genes in the co-essentiality network, the present inventors used the experimentally validated cancer driver gene set from Cancer Gene Census (CGC) database. For cancer type specific analysis, the present inventors manually mapped Tier 1 driver genes in CGC to TCGA cancer types according to their tumor type information. A total of 470 driver genes were mapped to 22 TCGA cancer types. Similarly, for modularity analysis, the present inventors selected 19 cancer types with more than 10 reported driver genes included in the co-essentiality network: bladder urothelial carcinoma (BLCA), breast invasive carcinoma (BRCA), Colon adenocarcinoma (COAD), lymphoid neoplasm diffuse large B-cell lymphoma (DLBC), head and neck squamous cell carcinoma (HNSC), Kidney Chromophobe (KICH), kidney renal clear cell carcinoma (KIRC), acute myeloid leukemia (LAML), brain lower grade glioma (LGG), liver hepatocellular carcinoma (LIHC), lung adenocarcinoma (LUAD), lung squamous cell carcinoma (LUSC), Ovarian serous cystadenocarcinoma (OV), Pancreatic adenocarcinoma (PAAD), prostate adenocarcinoma (PRAD), skin cutaneous melanoma (SKCM), stomach adenocarcinoma (STAD), Thyroid carcinoma (THCA), and Uterine Corpus Endometrial carcinoma (UCEC).

The driver genes mapped to cancer types are listed in below:

**TCGA_ACC:** TP53

**TCGA_GBM:** ACVR1, ATRX

**TCGA_ESCA:** KDM6A, LRP1B, SFRP4, SOX2

**TCGA_BLCA:** DROSHA, ERBB3, FGFR3, HRAS, KDM6A, LRP1B, MDM4, MTOR, NOTCH1, NOTCH2, STAG2, TERT, TSC1

**TCGA_LGG:** ATM, AXIN1, DDX3X, EPAS1, KDM6A, KMT2C, KMT2D, NBN, PALB2, PMS2: PTCH1, QKI, SUFU, TERT

**TCGA_HNSC:** BCORL1, CTCF, ERBB3, FAT1, FAT4, FES, MET, MTOR, NFE2L2, NOTCH1, PTK6, PTPRT, TGFBR2, TP63, TSC2

**TCGA_LIHC:** APC, ARID1B, ARID2, AXIN1, AXIN2, CASP8, CTNNB1, DNAJB1, FAT4, HNF1A, IL6ST, PRKACA, PTPN13, SMAD2, TERT

**TCGA_SKCM:** BLM, BRAF, DDB2, ERBB3, ERCC2, ERCC3, ERCC4, ERCC5, FAS, LATS1, NOTCH1, POLE, PTCH1, RECQL4, SMO, STAT5B, TBX3, TERT, XPA, XPC

**TCGA_STAD:** ACVR2A, ATR, AXIN2, BCL9L, BRAF, CDH1, ERBB2, ERBB3, ERBB4, FGFR2, GRIN2A, PIK3CA, POLE, PTK6, PTPN13, PTPRT, RHOA, SFRP4, UBR5, ZFHX3

**TCGA_PAAD:** ACVR2A, AKT2, APC, ATRX, BRAF, BRCA2, CDKN2A, DAXX, EP300, FAT1, FAT4, GNAS, HIF1A, KRAS, MAP2K4, MEN1, PREX2, RNF43, SMAD4, SND1, STK11

**TCGA_PRAD:** ACSL3, AR, AXIN1, BRAF, CANT1, DDX5, ELK4, ERG, ETV1, ETV4, ETV5, FOXA1, HERPUD1, HNRNPA2B1, KLF6, KLK2, NCOR2, NDRG1, PTEN, RAF1, SALL4, SLC45A3, SPOP, TMPRSS2, ZFHX3

**TCGA_UCEC:** ATR, AXIN1, BARD1, BCL9L, CHD4, CTCF, CUX1, FBXW7, FGFR2, HIF1A, MAX, MED12, MLH1, MSH2, MSH6, MTOR, NUTM2B, NUTM2D, PMS2, POLE, PTEN, RAD21, RAD51B, SPOP, SRC, SUZ12, YWHAE, ZFHX3, TCGA_THCA, BRAF, CCDC6, CDC73, CDKN1B, DICER1, ERC1, GOLGAS, HMGA1, HOOK3, KRAS, KTN1, MEN1, MTOR, NCOA4, NRAS, NTRK1, PAX8, PCM1, PPARG, PRKAR1A, RET, STRN, TFG, TPM3, TPR, TRIM27, TRIM33, TSHR, ZNF331, TCGA_KICH, AMER1, ARID1A, BAP1, CLTC, DICER1, DROSHA, FH, FLCN, GPC3, HIF1A, KDM6A, KMT2D, MET, MYCN, NF2, NONO, PALB2, PRCC, PTK6, SFPQ, SIX1, SMARCB1, TFE3, TFEB, TMEM127, TP53, TSC1, TSC2, VHL, WT1

**TCGA_OV:** AKT1, AKT2, ARID1A, ARID1B, ATR, BARD1, BRAF, BRCA1, BRCA2, CCNE1, CDK12, CTNNB1, ERBB2, FES, FOXL2, GOPC, LRP1B, MAPK1, MLH1, MSH2, MSH6, PIK3R1, PMS2, PPM1D, PPP2R1A, PTK6, RNF43, ROS1, SMARCA4 STK11

**TCGA_KIRC:** AMER1, ARID1A, BAP1, CLTC, DICER1, DROSHA, FH, FLCN, GPC3, HIF1A, KDM6A, KMT2D, MET, MTOR, MYCN, NF2, NONO, PALB2, PRCC, PTK6, SFPQ, SIX1, SMARCB1, TFE3, TFEB, TMEM127, TP53, TSC1, TSC2, VHL, WT1

**TCGA_BRCA:** AKT1, APOBEC3B, ARID1A, ARID1B, BAP1, BARD1, BRCA1, BRCA2, BRIP1, CASP8, CCND1, CDH1, CDKN1B, CHEK2, CTCF, EP300, ERBB2, ESR1, ETV6, FOXA1, GATA3, IRS4, KEAP1, MAP2K4, MAP3K1, MAP3K13, NCOR1, NOTCH1, NTRK3, PALB2, PBRM1, PIK3CA, POLQ, PPM1D, RB1, SALL4, SMARCD1, TBX3, TP53

**TCGA_COAD:** AKT1, APC, AXIN1, AXIN2, B2M, BAX, BCL9L, BRAF, CTNNB1, CUX1, EIF3E, EP300, ERBB3, FBXW7, GRIN2A, HIF1A, KRAS, MAP2K1, MAP2K4, MAX, MDM2, MLH1, MSH2, MSH6, MUTYH, PIK3CA, PIK3R1, PMS2, POLD1, POLE, PTPRK, PTPRT, QKI, RAD21, RSPO2, RSPO3, SALL4, SFRP4, SMAD2, SMAD3, SMAD4, SRC, TBL1XR1, TCF7L2, TGFBR2, TP53, UBRS, WDCP

**TCGA_LUAD:** AKT1, ALK, BAP1, BRAF, CCDC6, CD74, DDR2, DICER1, DROSHA, EGFR, EML4, ERBB2, ERBB4, EZR, FGFR2, GRIN2A, HIF1A, HIP1, KDR, KEAP1, KIF5B, KRAS, LRIG3, MAP2K1, MAP2K2, MYCL, NFE2L2, NKX2-1, NOTCH1, NRG1, PIK3CB, PTPN13, PTPRT, RAD21, RB1, RBM10, RET, ROS1, SDC4, SLC34A2, SMARCA4, SOX2, STK11, STRN, TFG, TP53, TP63, TPM3, TPR

**TCGA_LUSC:** AKT1, ALK, BAP1, BRAF, CCDC6, CD74, DDR2, DICER1, DROSHA, EGFR, EML4, ERBB2, ERBB4, EZR, FGFR2, GRIN2A, HIF1A, HIP1, KDR, KEAP1, KIF5B, KRAS, LRIG3, MAP2K1, MAP2K2, MYCL, NFE2L2, NKX2-1, NOTCH1, NRG1, PIK3CB, PTPN13, PTPRT, RAD21, RB1, RBM10, RET, ROS1, SDC4, SLC34A2, SMARCA4, SOX2, STK11, STRN, TFG, TP53, TP63, TPM3, TPR

**TCGA_DLBC:** ALK, ARHGAP26, ASXL1, ATIC, ATM, ATR, B2M, BCL10, BCL2, BCL6, BCL7A, BIRC3, BIRC3, BLM, BTK, CALR, CARD11, CARS, CBL, CCND2, CCND3, CD274, CD79A, CD79B, CDKN2C, CEBPA, CIITA, CLTC, CLTCL1, CNTRL, CNTRL, CREBBP, CSF3R, CUX1, DDX6, EIF4A2, EP300, ETV6, EZH2, FAS, FAT4, FBXO11, FCRL4, FGFR1, FGFR1, FGFR1OP, FGFR1OP, FGFR3, FGFR3, HIST1H4I, HSP90AA1, HSP90AB1, IGH, IGH, IGK, IGL, IKBKB, IKZF1, IL2, IL21R, IRF4, ITK, JAK2, KDM6A, KDSR, LEF1, MAF, MAFB, MALT1, MECOM, MN1, MPL, MSN, MUC1, MYC, MYD88, MYH9, NBN, NFKB2, NIN, NOTCH2, NPM1, NRAS, NSD2, PAFAH1B2, PAXS, PCM1, PDCD1LG2, PDE4DIP, PDGFRB, PIM1, POU2AF1, PRDM1, PRDM16, PRF1, PTPN11, REL, RHOA, RHOH, RMI2, RNF213, SBDS, SETBP1, SF3B1, SH2B3, SOCS1, SPEN, SRSF2, SRSF3, STAG2, SYK, SYK, TBL1XR1, TENTSC, TET2, TFG, TNFAIP3, TNFRSF14, TNFRSF17, TP63, TPM3, TPM4, U2AF1, UBRS, WAS, ZMYM2, ZMYM2, ZRSR2

**TCGA_LAML:** ABI1, ABL1, ABL2, AFDN, AFF1, AFF3, AFF4, ARHGAP26, ARHGAP26, ARHGEF12, ARNT, ASXL1, ASXL1, ATM, ATR, BCL11A, BCL11B, BCL2, BCL3, BCL6, BCL9, BCOR, BCORL1, BCR, BIRC3, BIRC3, BLM, BRCA2, BRIP1, BTG1, BTK, CALR, CBFA2T3, CBFB, CBL, CBL, CBLB, CBLC, CCDC6, CCND1, CCND2, CCND3, CDK6, CDKN2C, CDX2, CEBPA, CEBPA, CNOT3, CNTRL, CREBBP, CRLF2, CSF3R, CSF3R, CUX1, CUX1, DDX10, DDX3X, DEK, DNM2, DNMT3A, ELF4, ELL, EP300, EPS15, ERG, ETNK1, ETV6, EWSR1, FANCA, FANCC, FANCD2, FANCE, FANCF, FANCG, FAT1, FBXW7, FCGR2B, FGFR1, FGFR1OP, FGFR3, FLT3, FOXO3, FOXO4, FOXP1, FSTL3, FUS, GAS7, GATA1, GATA2, GPHN, HIP1, HLF, HOXA11, HOXA13, HOXA9, HOXC11, HOXC13, HOXD11, HOXD13, IGH, IGH, IKZF1, IL7R, IRF4, IRS4, JAK1, JAK2, JAK2, JAK3, KAT6A, KAT6B, KDMSA, KDM6A, KDM6A, KIT, KMT2A, KNL1, KRAS, LASP1, LCK, LEF1, LMO1, LMO2, LPP, LRP1B, LYL1, MAF, MAFB, MAPK1, MECOM, MECOM, MLF1, MLLT1, MLLT10, MLLT11, MLLT3, MLLT6, MN1, MN1, MPL, MRTFA, MSI2, MTCP1, MYC, MYH11, NCOA2, NFKBIE, NIN, NOTCH1, NPM1, NRAS, NRAS, NSD1, NSD2, NSD3, NT5C2, NUMA1, NUP214, NUP98, OLIG2, P2RY8, PALB2, PAX5, PBX1, PCM1, PCM1, PDE4DIP, PDGFRB, PDGFRB, PER1, PHF6, PICALM, PML, POT1, PRDM16, PRDM16, PRF1, PRRX1, PSIP1, PTPN11, PTPN11, PTPRC, RABEP1, RAD21, RAP1GDS1, RARA, RBM15, RPL10, RPL22, RPL5, RPN1, RUNX1, RUNX1T1, SBDS, SBDS, SET, SETBP1, SETBP1, SF3B1, SH2B3, SH2B3, SH3GL1, SRSF2, SRSF2, STAG2, STAT3, STAT5B, STIL, SYK, TAF15, TAL1, TAL2, TBL1XR1, TCF3, TCL1A, TENTSC, TET1, TET2, TLX1, TLX3, TOP1, TRA, TRB, TRD, TRIM24, TRIP11, U2AF1, U2AF1, XPO1, ZBTB16, ZMYM2, ZNF384, ZNF521, ZRSR2, ZRSR2

### 1-20. Network propagation with driver genes of each cancer type

To prioritize genes in the network according to their distance from the driver gene, the present inventors conducted network propagation using the page-rank algorithm from the NetworkX Python module. Among the 19 cancer types, the present inventors conducted network propagation for 17 cancer types, where the co-essentiality network had the highest modularity. For each cancer type, the present inventors assigned one for driver genes and zero for other genes in the network as input for the personalization parameter in the page-rank algorithm. All other page-rank algorithm parameters were adjusted to their default values (damping factor = 0.85).

## 1-21. Patient stratification and survival analysis

To identify driver modules capable of differentiating patient survival, the present inventors identified biological pathways located proximal to driver genes using network propagation scores of the genes in a network. To calculate the propagation score of the genes included in each pathway, the present inventors conducted a GSEA using the GSEApy Python module. Through GSEA on network propagation score, the present inventors selected pathways significantly enriched in genes with high network propagation scores using an FDR of < 0.001 and NES of > 0 as driver modules.

The transcriptome and clinical tables of patients used in the present disclosure were downloaded using the TCGAbiolinks R package. For the pre-processing of gene expression data of TCGA patients, the present inventors computed the gene expression levels using read counts, which were normalized by gene length corrected trimmed mean of M-values calculated using the edgeR R package. For statistical significance, the present inventors selected cancer types that contained at least 100 samples with survival data among 17 cancer types, where the co-essentiality network had the highest modularity of the driver genes. This resulted in 16 cancer types, including 7,259 tumor samples.

The present inventors stratified the patients from TCGA into two groups according to their gene expression levels in the driver modules. The present inventors conducted single-sample gene set enrichment analysis (ssGSEA) on the gene expression of the selected driver modules for each patient. According to NES of each patient, the present inventors defined the top 50% of patients as the upregulated group and the bottom 50% of patients as the downregulated group. To determine whether a survival difference existed between the upregulated and downregulated groups, the present inventors used the log-rank test and obtained statistical significance. To compare the maximum capability of patient subtyping across the networks, the driver module from each network whose patient grouping had the lowest p-value from the log-rank test was selected, and its p-value is shown in **Fig. 3I**.

## 1-22. Drug-gene association data

The present inventors obtained drug-gene association data from PanDrugs which assembled 18 resources with data curated by experts and drug-gene associations collected from experimental drug screenings. The source data of PanDrugs was provided by the original authors. The present inventors used two drug-gene association types, "direct target" and "biomarker", as drug targets. Finally, the present inventors considered 43,909 drug-target associations across 9,090 drugs.

## 1-23. Performance of prioritizing approved anticancer targets

To determine whether network propagation results can prioritize the targets of FDA-approved drugs in each cancer type, the present inventors performed GSEA on the propagation results of driver genes from the query cancer type using a target gene list of approved drugs. The present inventors conducted a GSEA using the GSEApy Python module. The present inventors collected 273 FDA-approved drugs across 17 cancer types from TCGA(https://www.cancer.gov/about-cancer/treatment/drugs) and PanDrugs databases. For each cancer type, a list of genes that had drug-target associations with approved drugs was used for GSEA. The performance of the network in prioritizing approved anticancer targets was measured using the NES value of the GSEA results. The FDA-approved anticancer drugs and their targets used in this analysis are listed in below:

[Approved Drugs]

**BLCA** ERDAFITINIB|ENFORTUMAB VEDOTIN-EJFV|MITOMYCIN|VALSTAR|ATEZOLIZUMAB|KEYTRUDA|METHOTREXATE SODIUM|BALVERSA|OPDIVO|BAVENCIO|VALRUBICIN|IMFINZI|PEMBROLIZUMAB|TECEN TRIQ|PADCEV|METHOTREXATE|DOXORUBICIN HYDROCHLORIDE|DURVALUMAB|CISPLATIN|THIOTEPA|NIVOLUMAB||JELMYTO|AVELUM AB

**BRCA** EXEMESTANE|FEMARA|ERIBULIN|ADO-TRASTUZUMAB EMTANSINE|EPIRUBICIN|LYNPARZA|IBRANCEITRASTUZUMAB AND HYALURONIDASE-OYSK|ATEZOLIZUMAB|TAXOTERE|METHOTREXATE SODIUM|PERJETA|PHESGO|ZOLADEX|TALZENNA|KISQALI|KADCYLA|METHOTREXATE|EL LENCE|TAMOXIFEN CITRATE|TUKYSA|ABRAXANE|VINBLASTINE|NERATINIB MALEATE|FARESTON|PIQRAY|VERZENIO|AFINITOR|HERCEPTIN|TOREMIFENE|ANASTROZ OLE|PACLITAXEL|TAMOXIFENAFINITOR DISPERZ|AREDIA|GOSERELIN ACETATE|TREXALL|PERTUZUMAB|MEGESTROL ACETATE|ENHERTU|SACITUZUMAB GOVITECAN-HZIY|5-FU|OLAPARIB|PALBOCICLIB|THIOTEPA|LAPATINIB DITOSYLATE|AROMASIN|PACLITAXEL ALBUMIN-STABILIZED NANOPARTICLE FORMULATION|TALAZOPARIB TOSYLATE|RUCAPARIB (CAMSYLATE)|IXEMPRA|HERCEPTIN HYLECTA|FAM-TRASTUZUMAB DERUXTECAN-NXKI|ALPELISIB|ARIMIDEX|MITOXANTRONE|EPIRUBICIN HYDROCHLORIDE|FULVESTRANT|GEMZAR|XELODA|GEMCITABINE HYDROCHLORIDE|RUCAPARIB|TECENTRIQ|TUCATINIB|IXABEPILONE|PAMIDRONATE DISODIUM|RIBOCICLIB|NERLYNX|VINBLASTINE SULFATE|EVEROLIMUS|FASLODEX|TOREMIFENE CITRATE|ABEMACICLIB|TRODELVY|HALAVEN|DOCETAXEL|CAPECITABINE|ERIBULIN MESYLATE|LAPATINIB|CYCLOPHOSPHAMIDE|MITOXANTRONE HYDROCHLORIDE|MELPHALAN|DOXORUBICIN HYDROCHLORIDE|LETROZOLE|PERTUZUMAB, TRASTUZUMAB, AND HYALURONIDASE-ZZXF|NERATINIB|RIBOCICLIB SUCCINATE|TRASTUZUMAB|FLUOROURACIL INJECTION|TYKERB

**CESC** PEMBROLIZUMAB|MVASI|AVASTIN|KEYTRUDA|BEVACIZUMAB|TOPOTECAN|TOP OTECAN HYDROCHLORIDE|BLEOMYCIN SULFATE|HYCAMTIN

**COADREAD** YERVOY|AVASTIN|LONSURF|ZIV-AFLIBERCEPT|TIPIRACIL HYDROCHLORIDE|TRIFLURIDINE AND TIPIRACIL HYDROCHLORIDE|KEYTRUDA|IRINOTECAN HYDROCHLORIDE|PILIMUMAB|VECTIBIX|IRINOTECAN|ERBITUX|OPDIVO|CAPECITABINE |PANITUMUMAB|5-FU|BEVACIZUMAB|XELODA|CAMPTOSAR|PEMBROLIZUMAB|MITOMYCIN C|TIPIRACIL|LEUCOVORIN CALCIUM|OXALIPLATIN|CYRAMZA|MVASI|CETUXIMAB|RAMUCIRUMAB|ELOXATIN|ZALT RAP|NIVOLUMAB|STIVARGA|REGORAFENIB|FLUOROURACIL INJECTION

**ESCA** KEYTRUDA|RAMUCIRUMAB|OPDIVO|TRASTUZUMAB

**GBM** MVASI|AVASTIN|AFINITOR DISPERZ|GLIADEL WAFER|PROCARBAZINE|TEMODAR|CARMUSTINE|AFINITOR|TEMOZOLOMIDE|EVEROLI MUS|CARMUSTINE IMPLANT|BICNU|BEVACIZUMAB|LOMUSTINE

**HNSC** CETUXIMAB|KEYTRUDA|TREXALL|HYDREA|TAXOTERE|PEMBROLIZUMAB|NIVOL UMAB|HYDROXYUREA|METHOTREXATE|ERBITUX|OPDIVO|DOCETAXEL|BLEOMYCIN SULFATE

**KIRC** AVASTIN|AFINITOR|IL-2|SORAFENIB|INTERLEUKIN-2|PROLEUKIN|NEXAVAR|EVEROLIMUS |PAZOPANIB HYDROCHLORIDE|KEYTRUDA|IPILIMUMAB|SUNITINIB|SUNITINIB MALATE|AFINITOR DISPERZ|OPDIVO|BAVENCIO|TEMSIROLIMUS|YERVOY|RELATED RESOURCES|TORISEL|BEVACIZUMAB|AXITINIB|PEMBROLIZUMAB|MITOMYCIN|PAZOPAN IB|ALDESLEUKIN|JELMYTO|SUTENT|MVASI|CABOMETYX|CABOZANTINIB-S-MALATE|LENVIMA|NIVOLUMAB|SORAFENIB TOSYLATE|VOTRIENT|INLYTA|AVELUMAB|LENVATINIB MESYLATE

**LGG** MVASI|AVASTIN|AFINITOR DISPERZ|GLIADEL WAFER|PROCARBAZINE|TEMODAR|CARMUSTINE|AFINITOR|TEMOZOLOMIDE|EVEROLI MUS|CARMUSTINE IMPLANT|BICNU|BEVACIZUMAB|LOMUSTINE

**LIHC** PEMIGATINIB|CYRAMZA|REGORAFENIB|ATEZOLIZUMAB|CABOMETYX|KEYTRUD A|BEVACIZUMAB|STIVARGA|CABOZANTINIB-S-MALATE|LENVIMA|PEMBROLIZUMAB|AVASTIN|TECENTRIQ|SORAFENIB TOSYLATE|FLOXURIDINE|RAMUCIRUMAB|NIVOLUMAB|NEXAVAR|OPDIVO|PEMAZYRE|L ENVATINIB MESYLATE

**LUAD** CYRAMZA|IRESSA|AVASTIN|TARCEVA|OSIMERTINIB MESYLATE|AFINITOR|ALECTINIB HYDROCHLORIDE|PORTRAZZA|SELPERCATINIB|VINORELBINE|TOPOTECAN HYDROCHLORIDE|PORFIMER|METHOTREXATE SODIUM|ENTRECTINIB|IPILIMUMAB|CERITINIB|IMFINZI|GILOTRIF|TAFINLAR|ALIMTA|ER LOTINIB HYDROCHLORIDE|XALKORI|EVEROLIMUS|ALECENSA|GEMCITABINE|MEKINIST|PACLITAXEL|TAXOTERE|TRAMETINIB|CARBOPLATIN|P"RAPL'T|AFINITOR DISPERZ|DOCETAXEL|DABRAFENIB MESYLATE|OPDIVO|TAGRISSO|BEVACIZUMAB|PACLITAXEL ALBUMIN-STABILIZED NANOPARTICLE FORMULATION|YERVOY|BRIGATINIB|GEMZAR|MUSTARGEN|TREXALL|VIZIMPRO|VINOR ELBINE TARTRATE|ALUNBRIG|GEMCITABINE HYDROCHLORIDE|TOPOTECAN|DACOMITINIB|ROZLYTREK|TECENTRIQ|LORBRENA|METH OTREXATE|PEMETREXED|PEMETREXED DISODIUM|ATEZOLIZUMAB|ABRAXANE|PEMBROLIZUMAB|MVASI|DOXORUBICIN HYDROCHLORIDE|PARAPLATIN|LORLATINIB|RETEVMO|RAMUCIRUMAB|MECHLORETHA MINE HYDROCHLORIDE|GEFITINIB|AFATINIB|CAPMATINIB HYDROCHLORIDE|DURVALUMAB|NIVOLUMAB|CRIZOTINIB|KEYTRUDA|TABRECTA|ZYKAD IA|AFATINIB DIMALEATE|NECITUMUMAB|ALECTINIB|ERLOTINIB

**LUSC** CYRAMZA|IRESSA|AVASTIN|TARCEVA|OS|MERTINIB MESYLATE|AFINITOR|ALECTINIB HYDROCHLORIDE|PORTRAZZA|SELPERCATINIB|VINORELBINE|TOPOTECAN HYDROCHLORIDE|PORFIMER|METHOTREXATE SODIUM|ENTRECTINIB|IPILIMUMAB|CERITINIB|IMFINZI|GILOTRIF|TAFINLAR|ALIMTA|ER LOTINIB HYDROCHLORIDE|XALKORI|EVEROLIMUS|ALECENSA|GEMCITABINE|MEKINIST|PACLITAX EL|TAXOTERE|TRAMETINIB|CARBOPLATIN|PARAPLAT|AFINITOR DISPERZ|DOCETAXEL|DABRAFENIB MESYLATE|OPDIVO|TAGRISSO|BEVACIZUMAB|PACLITAXEL ALBUMIN-STABILIZED NANOPARTICLE FORMULATION|YERVOY|BRIGATINIB|GEMZAR|MUSTARGEN|TREXALL|VIZIMPRO|VINOR ELBINE TARTRATE|ALUNBRIG|GEMCITABINE HYDROCHLORIDE|TOPOTECAN|DACOMITINIB|ROZLYTREK|TECENTRIQ|LORBRENA|METH OTREXATE|PEMETREXED|PEMETREXED DISODIUM|ATEZOLIZUMAB|ABRAXANE|PEMBROLIZUMAB|MVASI|DOXORUBICIN HYDROCHLORIDE|PARAPLATIN|LORLATINIB|RETEVMO|RAMUCIRUMAB|MECHLORETHA MINE HYDROCHLORIDE|GEFITINIB|AFATINIB|CAPMATINIB HYDROCHLORIDE|DURVALUMAB|NIVOLUMAB|CRIZOTINIB|KEYTRUDA|TABRECTA|ZYKAD IA|AFATINIB DIMALEATE|NECITUMUMAB|ALECTINIB|ERLOTINIB

**PRAD** PROVENGE|RADIUM 223 DICHLORIDE|RUBRACA|ELIGARD|XTAND|FIRMAGON|FLUTAMIDE|ENZALUTAMIDE|NILU TAMIDE|LYNPARZA|ERLEADA|DEGARELIX ACETATE|CABAZITAXEL|MITOXANTRONE|ABIRATERONE|NILANDRON|JEVTANA|LEUPROLI DE ACETATE|BICALUTAMIDE|LUPRON DEPOT|RUCAPARIB CAMSYLATE|GOSERELIN|GOSERELIN ACETATE|XOFIGO|MITOXANTRONE HYDROCHLORIDE|LUPRON|DEGARELIX|MELPHALAN|ESTRAMUSTINE|TAXOTERE|ZOLADE X|OLAPARIB|DAROLUTAMIDE|ESTRAMUSTINE PHOSPHATE SODIUM|DOCETAXEL|NUBEQA|CASODEX|ABIRATERONE ACETATE|APALUTAMIDE|SIPULEUCEL-T|ESTRAMUSTINE PHOSPHATE|ZYTIGA

**SKCM** SONIDEGIB|FLUOROURACIL-TOPICAL|COBIMETINIB|VINBLASTINE SULFATE|ERIVEDGE|VISMODEGIB|PACLITAXEL|VORINOSTAT|TRAMETINIB|ODOMZO|ALD ARA|COBIMETINIB FUMARATE|DACARBAZINE|DABRAFENIB MESYLATE|VEMURAFENIB|1218778-77-8|ERISMODEGIB|DABRAFENIB|1187431-43-1|5-FU|VINBLASTINE|EFUDEX|ALITRETINOIN|IMIQUIMOD|AVELUMAB

**STAD** CYRAMZA|DOXORUBICIN HYDROCHLORIDE|TRIFLURIDINE AND TIPIRACIL HYDROCHLORIDE|PEMBROLIZUMAB|EVEROLIMUS|5-FU|RAMUCIRUMAB|TRASTUZUMAB|TAXOTERE|HERCEPTIN|MITOMYCIN|SUNITINIB|SU NITINIB MALATE|LANREOTIDE|DOCETAXEL|MITOMYCIN C|KEYTRUDA|LONSURF|REGORAFENIB|FLUOROURACIL INJECTION

**UCEC** HYDROXYPROGESTERONE CAPROATE|DACTINOMYCIN|VINBLASTINE|METHOTREXATE SODIUM|LENVIMA|PEMBROLIZUMAB|MEGESTROL ACETATE|KEYTRUDA|METHOTREXATE|VINBLASTINE SULFATE|LENVATINIB MESYLATE

**UCS** HYDROXYPROGESTERONE CAPROATE|DACTINOMYCIN|VINBLASTINE|METHOTREXATE SODIUM|LENVIMA|PEMBROLIZUMAB|MEGESTROL ACETATE|KEYTRUDA|METHOTREXATE|VINBLASTINE SULFATE|LENVATINIB MESYLATE

[Target of approved drugs]

**BLCA** ERDAFITINIB|ENFORTUMAB VEDOTIN-EJFV|MITOMYCIN|VALSTAR|ATEZOLIZUMAB|KEYTRUDA|METHOTREXATE SODIUM|BALVERSA|OPDIVO|BAVENCIO|VALRUBICIN|IMFINZI|PEMBROLIZUMAB|TECEN TRIQ|PADCEV|METHOTREXATE|DOXORUBICIN HYDROCHLORIDE|DURVALUMAB|CISPLATIN|THIOTEPA|NIVOLUMAB|JELMYTO|AVELUM AB FKBP15|SALL3|NQO1|DUSP28|B2M|CD226|ATP9B|S100A12|GPR35|FGFR4|C1 8orf63|HDAC4|TAT|NHLRC1|SMIM21|CCDC102B|NTRK1|FGFR3|ANKMY1|NDUFA10|ZN F236|FAM132B|SCLY|CDH7|AC016757.3|XPC|ALO"5|RA'P1|CLCN61|PASK|FAM69C|PER2 |SOCS6|PMS2|FBXO15|FGFR2|CNDP1|CNDP2|KRAS|ATIC|RBM44|CYP2B6|CD3EAP|ZNF 516|ZADH2|KIT|PTEN|GALR1|NR1I2|NETO1|FOLH1|TMEM43|SLC19A1|CDKN1B|FGFR1 |ANO7|MSH2|SLC31A1|AC110619.2|XRCC3|RP11-162A12.2|UBE2F-SCLY|RP11-861L17.3|MLH1|ASXL1|STK25|RP11-321M21.3|JAK2|JAK3|JAK1|CX3CL1|TRAF3IP1|RP11-723G8.2|PRR21|TOP2A|DSEL|CTLA4|FARP2|KLHL30|TSHZ1|PPP1R7|ESPNL|ASB1|AFP|IL1RN|TP53|ESR1|CD274|UBE2F|TIMM21|BAIAP3|CCND1|OTOS|HES6|TMX3|CBLN2|AC062017.1|RNPEPL1|POLE|RP11-41O4.1|ITIH1|AGXT|ITIH3|GLS|FLT3|KCNA1|RTTN|TPM3|CDH19|ICAM3|S100A8|LRRFIP1|KIF1A|BIRC5|SNED1|PDCD1|ACVR2A|ALDH3A1|ARVCF|MTHFR|MECP2|TPMT|COMT|BAX|GTSCR1|ADA|LINC00908|LRP2|GALNT14|ALK|GSTM1|GSTM4|CAPN10|IL2|C2orf54|ZNF407|SLAMF1|RP11-17M16.1|TOP2B|DHFR|ERCC2|MSH3|EGFR|MSH6|FCGR3B|SLCO1B1|AC093802.1|AC104809.3|ALDH1A1|OR6B2|OR6B3|HLA-DRA|AC079612.1|TWIST2|NRAS|RB1|DOK6|AQP12B|AQP12A|SEPT2|MBP|KRT20|SULT2A1|NR3C1|HDLBP|HSPB2|RP11-94B19.4|RP11-169F17.1|GPC1|ILKAP|WRAP53|HGF|CYB5A|MTERFD2|E2F1|NOTCH1|TYMS|GSTP1|KDR|MYEOV2|BRAF|ABCC4|PPP1R13L

**BRCA** EXEMESTANE|FEMARA|ERIBULIN|ADO-TRASTUZUMAB EMTANSINE|EPIRUBICIN|LYNPARZA|IBRANCEITRASTUZUMAB AND HYALURONIDASE-OYSK|ATEZOLIZUMAB|TAXOTERE|METHOTREXATE SODIUM|PERJETA|PHESGO|ZOLADEX|TALZENNA|KISQALI|KADCYLA|METHOTREXATE|ELLENCE|TAMOXIFEN CITRATE|TUKYSA|ABRAXANE|VINBLASTINE|NERATINIB MALEATE|FARESTON|PIQRAY|VERZENIO|AFINITOR|HERCEPTIN|TOREMIFENE|ANASTROZOLE|PACLITAXEL|TAMOXIFEN|AFINITOR DI"PERZ'AREDIA|GOSERELIN ACETATE|TREXALL|PERTUZUMAB|MEGESTROL ACETATE|ENHERTU|SACITUZUMAB GOVITECAN-HZIY|5-FU|OLAPARIB|PALBOCICLIB|THIOTEPA|LAPATINIB DITOSYLATE|AROMASIN|PACLITAXEL ALBUMIN-STABILIZED NANOPARTICLE FORMULATION|TALAZOPARIB TOSYLATE|RUCAPARIB (CAMSYLATE)|IXEMPRA|HERCEPTIN HYLECTA|FAM-TRASTUZUMAB DERUXTECAN-NXKI|ALPELISIB|ARIMIDEX|MITOXANTRONE|EPIRUBICIN HYDROCHLORIDE|FULVESTRANT|GEMZAR|XELODA|GEMCITABINE HYDROCHLORIDE|RUCAPARIB|TECENTRIQ|TUCATINIB|IXABEPILONE|PAMIDRONATE DISODIUM|RIBOCICLIB|NERLYNX|VINBLASTINE SULFATE|EVEROLIMUS|FASLODEX|TOREMIFENE CITRATE|ABEMACICLIB|TRODELVY|HALAVEN|DOCETAXEL|CAPECITABINE|ERIBULIN MESYLATE|LAPATINIB|CYCLOPHOSPHAMIDE|MITOXANTRONE HYDROCHLORIDE|MELPHALAN|DOXORUBICIN HYDROCHLORIDE|LETROZOLE|PERTUZUMAB, TRASTUZUMAB, AND HYALURONIDASE-ZZXF|NERATINIB|RIBOCICLIB SUCCINATE|TRASTUZUMAB|FLUOROURACIL INJECTION|TYKERB STK11|PCSK6|DUSP28|CD226|SLC50A1|TAT|PIK3CA|PIK3CB|PIK3CD|PIK3CG|IRAK2|SCLY|CBLC|IL2RA|IFNG|CAMKV|PI"3C2B'PIK3C2A|PIK3C2G|MUC16|CYP3A4|CYP3A5|CNDP1|CNDP2|F5|POU2AF1|CD3EAP|T"BB4B'CCND3|CCND2|PTEN|TUBE1|DSTYK|CDKN1B|CDKN1A|ALDH3A1|EDNRB|SNORD68|RP11-162A12.2|RP11-321M21.3|JAK2|NF2|NF1|ZNF516|DSEL|ALPK2|DST|BLM|PPARD|CHST3|RTTN|LRMDA|PSIP1|CSNK1A1|TOP1|ITGAL|DCST2|HES6|SMAD4|RET|ITPR2|XIAP|TAF15|FH|GLS|TSC2|TSC1|NCOA3|NCOA1|PRAME|AFP|CKS1B|NR2F2|SPARC|S100A8|TFPI|NAT2|LTK|SRPK2|CYP2C9|SLCO1B1|F2|ALK|MAP2K1|KRTCAP2|PBRM1|ERBB2|TOP2B|MAP2K5|FLCN|DHFR|FLAD1|EGFR|BAX|CTNNB1|OR6B2|OR6B3|HLA-DRA|MYODI|DPYD|CDK12|TWIST2|CXCR2|PGR|PGP|FOLH1|NOS1|ITGB2|AC110619.2|RP11-169F17.1|HGF|MTERFD2|EIF4E2|E2F1|BNC2|PRCC|HEXB|CYB5A|PPP1R13L|LIF|NQO2|NQO1|FCGR1A|PIK3C3|TUBA3E|TUBA3D|AD"MTS2'SIX4|ROR2|NDUFA10|CDH1|CXCR7|CDH7|CDKN2B|AC093802.1|HLA-DRB1|RAMP1|FAM69C|KLK3|ARID1A|C1QC|C1QB|C1QA|CCND1|NETO1|APC|ACTB|FOXP3|NOS3|CNTN5|SMARCB1|ATP7A|MYCN|RP11-861L17.3|CYP2D6|CCNE1|COL18A1|STK25|ITK|TUBD1|TRAF3IP1|PRR21|IGF2|FARP2|TUBB2A|TUBB2B|TUBB|PCDH15|GAS6|EPHX1|PDGFRA|HDAC2|HDAC4|ZNF236|ATM|CYP4B1|TIGD1|ATR|RP11-41O4.1|FLT3|CYP1B1|POLD1|PSEN1|TPM3|RP11-201K10.3|FANCC|AC104809.3|FANCA|LRRFIP1|FANCI|FBXW7|NTF3|TET2|MED24|DLL1|MTOR|GTSCR1|LINC00908|NPAT|KIF1A|SLC22A16|IRS1|CDK2|CDK4|CDK6|SLAMF1|TGFB1|POLA1|MSH2|MSH3|MSH6|MCL1|BGLAP|AC079612.1|RB1|SERPINE1|ETS2|ABCB1|AURKA|ABCB4|SULT2A1|PRKCA|FCGR2B|FCGR2C|FCGR2A|CES1|ABCC4|CES2|TUBA4A|CTNNA2|TOPBP1|NOTCH1|PARP3|HLA-DQA1|TUBG1|TYMS|GSTP1|MYEOV2|ABCC3|MMP2|TGM2|FBXO15|GPER1|GPR35|PPP1R7|MAP3K2|DNMT1|RNPEPL1|BRDT|JAK3|BRCA1|ERBB3|BRCA2|CETP|SHC1|ERBB4|IDH2|IDH1|CLCN6|PASK|PER2|RPL13|BRD4|CTSS|FGFR2|FGFR3|FGFR1|ATIC|RBM44|PLD1|SYK|CXCL8|GALR1|FKBP1A|ALPK1|GRIA3|HRAS|RRAS2|GAPDH|CRTC3|XRCC1|XRCC3|XRCC4|SEPT2|UBE2F-

SCLY|PPM1D|GLDN|FOS|JUN|PPARGC1A|RRM2B|NFE2|CX3CL1|CHEK2|CHEK1|CTLA4|TUBB8|SULT1C4|TUBB6|TSHZ1|TUBB3|PARP2|TUBB1|SFPQ|ASB1|ESPNL|TP53|GNAQ|ESR1|CD274|PALB2|CYP19A1|UBE2F|HERC2|EIF4"BP1'|'TOS|AC062017.1|SNED1|KCNA1|SLC10A2|JMJD6|ANKMY1|CD80|CBLN2|ICAM3|RRM2|DPM3|ERCC2|KDR|SRC|PRDX4|ACACA|EFNA4|EFNA3|EFNA1|PIP|SALL3|ESR2|CYP1A2|RPSA|GSTM1|TNFRSF1A|AGXT|IL15|CAPN10|IL5|IL2|C2orf54|ZNF407|RIPK4|CDH19|BIRC5|FCGR3A|BIRC2|FCGR3B|MAP2|SLCO1B3|DDIT3|MAP4|UGT1A5|ALDH1A1|FAS|MLLT6|AMHR2|PTHLH|NRG1|MBP|KRT20|ANXA1|HSPB2|ZBTB7B|MTR|ILKAP|CYP2A6|WRAP53|PARP1|EPCAM|CASP3|DRD4|DRD2|EBP|DCST1|MAPT|C1S|C1R|ABCC6|ABCC1|BCL2|PTGS2|GSR|FAM132B|AKT1|AKT2|AKT3|ATP9B|BDNF|S100A12|PBXIP1|C18orf63|CCDC102B|NTRK1|TLE3|CDKN2A|SFTPA1|EDN1|MYC|MYB|                                 AC016757.3|SOD2|ALOX5|MGMT|PMS2|SMIM21|KRAS|FDPS|SOCS6|POLE|ZADH2|NR1I2|YSK4|SLC19A1|VDR|STMN1|TRAF2|CSF2|CLTC|ARID4B|CHD1|PTH|TUBA1C|TUBA1B                     |TUBA1A|PIK3R6|PIK3R5|PIK3R4|PIK3R3|PIK3R2|PIK3R1|RP11-723G8.2|TOP2A|TRH|KLHL30|STAG2|TRIM46|FGF2|IL1RN|ABCG2|MPL|SIK2|HOXD13|           PRKCZ|EPHA4|EPHA3|GNRHR|ABL1|CGA|CACNB2|CYP2B6|MAPK3|FASN|PDCD4|TBXA          2R|PYGO2|MTHFR|HDLBP|ARFGEF1|ADA|ADK|RASGRF2|MET|PRKACG|TIMM21|RP11-17M16.1|SPG7|KALRN|TFF3|VHL|LENEP|ANO7|ADAM15|RRM1|DSCAM|OPLAH|NRAS|MLH1|DOK6|AQP12B|AQP12A|CYP2C19|LDHA|NR3C1|RP11-94B19.4|PLAT|GPC1|GGT1|TMX3|SDHC|ABCC10|PMVK|ASXL1|PTPRC|BRAF|GNA11|B AP1

CESC PEMBROLIZUMAB|MVASI|AVASTIN|KEYTRUDA|BEVACIZUMAB|TOPOTECAN|TOP OTECAN HYDROCHLORIDE|BLEOMYCIN                                           SULFATE|HYCAMTIN NRAS|HRAS|MSH3|FCGR3A|MSH2|FCGR3B|VHL|POLE|COL18A1|RB1|FCGR1A|     ATRX|BDNF|HTRA1|HLA-DRA|TYMS|DPYD|PIK3CA|THBS2|MLH1|CXCR2|JAK2|NF2|NF1|JAK1|ANXA11|GGH|TOP1|PDCD1|ERBB2|PTEN|MTHFR|EGFR|IDH1|TOP1MT|FCGR2B|FCGR2C|FCGR2A|VEGFA|MSH6|VEGFC|VEGFB|MUC16|PMS2|B2M|SHMT1|KRAS|ARMS2|TP53|ALK|CXCL8|C1QC|C1QB|C1QA|KIT|MET|BRAF|MMP9|C1R|CD274|MMP2|SLC19A1

COADREAD YERVOY|AVASTIN|LONSURF|ZIV-AFLIBERCEPT|TIPIRACIL HYDROCHLORIDE|TRIFLURIDINE AND TIPIRACIL HYDROCHLORIDE|KEYTRUDA|IRINOTECAN HYDROCHLORIDE|IPILIMUMAB|VECTIBIX|IRINOTECAN|ERBITUX|OPDIVO|CAPECITABINE        |PANITUMUMAB|5-FU|BEVACIZUMAB|XELODA|CAMPTOSAR|PEMBROLIZUMAB|MITOMYCIN       C|TIPIRACIL|LEUCOVORIN       CALCIUM|OXALIPLATIN|CYRAMZA|MVASI|CETUXIMAB|RAMUCIRUMAB|ELOXATIN|ZALT        RAP|NIVOLUMAB|STIVARGA|REGORAFENIB|FLUOROURACIL                                                        INJECTION PTGS2|NT5E|AKT1|B2M|ARAF|FCGR1A|LGALS2|BDNF|EGF|TAT|PIK3CA|NTRK1|PTP4A3|CDH1|JAK2|FGFR1|BRCA1|ERBB3|BRCA2|ERBB4|IDH1|BGLAP|MGMT|CXCL10|PMS2|FGFR2|SHMT1|KRAS|CXCL8|C1QC|C1QB|C1QA|KIT|PTEN|APC|KLC3|SLC19A1|CDKN1A|MSH2|BCHE|XRCC1|ATP7A|ATP7B|RUNX3|THBS2|VPS37A|XDH|ISG15|NF2|NF1|JAK1|ANXA11|CTLA4|FRK|VEGFA|VEGFC|VEGFB|TP53|DDR2|CD274|PALB2|TOP1|V     WF|KRT18|PDGFRB|PDGFRA|SMAD4|ATM|EPHA2|RET|POLE|MAPK10|MAPK11|ABL1|I       L1A|FLT4|RAF1|HTRA1|FLT1|AREG|TEK|FANCC|GGH|KDR|FBXW7|MTHFR|FCGR3A|TOP1MT|CGB5|AGAP1|AC064874.1|MSH6|ATRX|TJP2|ARMS2|ALK|GSTM1|MET|EREG|PARD3B|RAD50|PDCD1|ERBB2|DHFR|ERCC1|ERCC2|MSH3|EGFR|BAX|FCGR3B|VHL|MAP2K1|CDKN2A|UGT1A1|BIRC5|HLA-DRA|TYMSOS|DPYD|NRAS|TYMP|MLH1|CXCR2|ABCB1|ABCB4|NRG1|PIGF|COL18A1|FCGR2B|FCGR2C|FCGR2A|CES1|CES2|CYP7B1|HRAS|E2F1|DKK1|TYMS|GSTP1|BRAF|MMP9|C1S|C1R|MMP2|ABCC1|BCL2

ESCA                KEYTRUDA|RAMUCIRUMAB|OPDIVO|TRASTUZUMAB               ERBB2|PDGFRA|BIRC5|FCGR3A|FCGR3B|AKT2|FCGR1A|MET|PIK3CA|PGR|KDR|               ANXA1|ERBB3|IFNG|EGFR|FCGR2B|FCGR2C|FCGR2A|C1R|HGF|EPCAM|KRAS|ESR1|ARID1A|C1QC|C1QB|C1QA|PTEN|BRAF|C1S|APC

GBM    MVASI|AVASTIN|AFINITOR   DISPERZ|GLIADEL   WAFER|PROCARBAZINE|TEMODAR|CARMUSTINE|AFINITOR|TEMOZOLOMIDE|EVEROLI   MUS|CARMUSTINE   IMPLANT|BICNU|BEVACIZUMAB|LOMUSTINE          BRCAL1|GSR|SYNE1|STMN4|THBS2|HRAS|ATM|EGFR|PRL|RET|C1QB|CTNNB1|VHL|AKT2|AKT3|COL18A1|CSF2|FCGR1A|MMP9|MGMT|KIT|TSC2|HTRA1|TSC1|H2AFX|C1R|DPYD|PIK3CA|ATR|NRAS|RB1|CXCR2|AKT1|NF2|NF1|ANXA11|MYC|GGH|ERBB21                  FLCN

|BRCA2|STAG2|MTHFR|FCGR3A|IDH1|GNA11|FCGR2B|FCGR2C|PLAU|FCGR2A|MT OR|MSH6|VEGFC|VEGFB|ATRX|FKBP1A|ADK|SHMT1|KRAS|ARMS2|TP53|GNAQ|E2F1| ESR1|CXCL8|MPL|G6PD|C1QC|NOTCH1|C1QA|STK11|PTEN|MAOA|FCGR3B|BRAF|BAX |PBRM1|VEGFA|BAP1|SLC19A1|MMP2|MET

**HNSC** CETUXIMAB|KEYTRUDA|TREXALL|HYDREA|TAXOTERE|PEMBROLIZUMAB|NIVOL UMAB|HYDROXYUREA|METHOTREXATE|ERBITUX|OPDIVO|DOCETAXEL|BLEOMYCIN SULFATE SALL3|STK11|FBXO15|DUSP28|AKT1|B2M|CD226|RB1|FCGR1A|BDNF|S100A12 |GPR35|EGF|C18orf63|TAT|PPP1R7|SMIM21|CCDC102B|PIK3CA|DNMT1|NTRK1|SMAD 4|NDUFA10|PTP4A3|ZNF236|FAM132B|RRM2B|SCLY|CDH7|ERBB2|ERBB3|AC016757.3 |AQP12A|ERBB4|ALOX5|RAMP1|AC062017.1|FAM69C|PER2|KLK3|RPL13|BRD4|MUC1 6|CYP3A4|CYP3A5|FGFR2|CNDP1|CNDP2|KRAS|ATIC|RBM44|CCND1|CXCL8|C1QC|C1 QB|C1QA|KIT|PTEN|GALR1|NETO1|SULT2A1|SLC19A1|DSEL|CDKN1B|HRAS|ANO7|SNE D1|ZADH2|XRCC1|AC110619.2|ARID4B|ATP7A|XRCC4|RP11-162A12.2|UBE2F-SCLY|RP11-861L17.3|NR1I2|PASK|NT5E|VPS37A|STK25|RP11-321M21.3|JAK21|NF2|JAK1|TRAF3|P1|RP11-723G8.2|PRR21|IGF2|FARP2|KLHL30|SULT1C4|TSHZ1|TUBB3|TUBB1|BLM|PPARD|ASB1 |EIF4E|CHST3|ESPNL|AFP|C2orf54|IL1RN|TP53|DNTT|GPC1|CD274|ATP9B||TUBB|UBE2 F|TIMM21|OTOS|AREG|HES6|GAS6|EPHX1|PMS2|IC4|TLE3|CBLN2|CYP4B1|RET|RNPE PL1|POLE|RP11-4104.1|FH|GLS|FLT3|KCNA1|SLC10A2|ANKMY1|RTTN|TPM3|CDH19|ICAM3|S100A8|L RRFIP1|KIF1A|AC093802.1|DUSP1|MSH2|KDR|FBXW7|MSH3|MTHFR|EGFR|HDLBP|MS H6|GTSCR1|ADA|LINC00908|CP|TJP2|SLC01B1|ALK|GSTM1|AGXT|MAP2K1|CAPN10| MET|IL2|EREG|ZNF407|PDCD1|SLAMF1|RRM2|RP11-17M16.1|RRM1|SPG7|DHFR|BIRC5|PRDX4|FCGR3A|BAX|FCGR3B|MAP2|SLC01B3|ZNF 516|MCL1|MAP4|XRCC3|OR6B2|OR6B3|HLA-DRA|AC104809.3|DPYD|AC079612.1|TWIST2|NRAS|MLH1|SNORD68|ABCB1|DOK6|AQ P12B|PGP|NAT2|NRG1|SEPT2|MBP|FOLH1|NR3C1|HSPB2|RP11-94B19.4|RP11-169F17.1|FCGR2B|FCGR2C |ABL1|FCGR2A|ABCC4|SOCS6|ILKAP|CYP2A6|TMX3|HGF|CY B5A|MTERFD2|E2F1|PTH|NOTCH1|TYMS|GST"1|MY'OV2|BRAF|MAPT|C1S|C1R|ABCC6 |BCL2

**KIRC** AVASTIN|AFINITOR|IL-2|SORAFENIB|INTERLEUKIN-2|PROLEUKIN|NEXAVAR|EVEROLIMUS|PAZOPANIB HYDROCHLORIDE|KEYTRUDA|IPILIMUMAB|SUNITINIB|SUNITINIB MALATE|AFINITOR DISPERZ|OPDIVO|BAVENCIO|TEMSIROLIMUS|YERVOY|RELATED RESOURCES|TORISEL|BEVACIZUMAB|AXITINIB|PEMBROLIZUMAB|MITOMYCIN|PAZOPAN IB|ALDESLEUKIN|JELMYTO|SUTENT|MVASI|CABOMETYX|CABOZANTINIB-S-MALATE|LENVIMA|NIVOLUMAB|SORAFENIB TOSYLATE|VOTRIENT|INLYTA|AVELUMAB|LENVATINIB MESYLATE STK11|PLK4|AKT1|AKT2|AKT3|ARAF|MLH1|FCGR1A|PMS2|B2M|PIK3CA|PHC1 |FGFR1|BRCA1|FLCN|BRCA2|IL2RA|IDH1|KRAS|FGFR2|FGFR3|SHMT1|ITK|CXCL8|C1QC |C1QB|C1QA|KIT|PTEN|XRN1|FKBP1A|SLC19A1|HLA-B|HRAS|HIF1A|CSF1|ASXL1|RAF1|CCNE1|THBS2|FLT3|JAK2|NF2|NF1|JAK1|ANXA11|TS C1|CTLA4|ERP27|VEGFA|VEGFC|VEGFB|FGF3|FGF1|TP53|GNAQ|ESR1|DDR2|CD274|M PL|HMOX1|LCK|PDGFRB|PDGFRA|MKI67|ATM|RET|POLE|ABL1|FLT4|TSC2|HTRA1|FLT 1|CSF1R|GGH|KDR|FBXW7|ACVR2A|MTHFR|FCGR3A|MTOR|SH2B3|ARMS2|IL2RB|ALK |IL2RG|MET|PBRM1|CDK8|PDCD1|ERBB2|MSH2|MSH3|EGFR|MSH6|FCGR3B|CTNNB1 |VHL|UGT1A1|HLA-DRA|CDK19|DPYD|NRAS|RB1|CXCR2|PGR|AURKB|PTPRB|PDGFA|PDGFB|PDGFC|PDGF D|COL18A1|GNA11|FCGR2B|FCGR2C|FCGR2A|CA9|NOTCH1|U2AF1|BRAF|MMP9|C1R| BAP1|MMP2

**LGG** MVASI|AVASTIN|AFINITOR DISPERZ|GLIADEL WAFER|PROCARBAZINE|TEMODAR|CARMUSTINE|AFINITOR|TEMOZOLOMIDE|EVEROLI MUS|CARMUSTINE IMPLANT|BICNU|BEVACIZUMAB|LOMUSTINE BRCAL1|GSR|SYNE1|STMN4|THBS2|HRAS|ATM|EGFR|PRL|RET|C1QB|CTNNB1| VHL|AKT2|AKT3|COL18A1|CSF2|FCGR1A|MMP9|MGMT|KIT|TSC2|HTRA1|TSC1|H2AFX |C1R|DPYD|PIK3CA|ATR|NRAS|RB1|CXCR2|AKT1|NF2|NF1|ANXA11|MYC|GGH|ERBB21 FLCN|BRCA2|STAG2|MTHFR|FCGR3A|IDH1|GNA11|FCGR2B|FCGR2C|PLAU|FCGR2A|MT OR|MSH6|VEGFC|VEGFB|ATRX|FKBP1A|ADK|SHMT1|KRAS|ARMS2|TP53|GNAQ|E2F1| ESR1|CXCL8|MPL|G6PD|C1QC|NOTCH1|C1QA|STK11|PTEN|MAOA|FCGR3B|BRAF|BAX |PBRM1|VEGFA|BAP1|SLC19A1|MMP2|MET

**LIHC** PEMIGATINIB|CYRAMZA|REGORAFENIB|ATEZOLIZUMAB|CABOMETYX|KEYTRUD A|BEVACIZUMAB|STIVARGA|CABOZANTINIB-S-MALATE|LENVIMA|PEMBROLIZUMAB|AVASTIN|TECENTRIQ|SORAFENIB TOSYLATE|FLOXURIDINE|RAMUCIRUMAB|NIVOLUMAB|NEXAVAR|OPDIVO|PEMAZYRE|L ENVATINIB MESYLATE PDGFRB|PDGFRA|NRAS|HRAS|MSH3|FCGR3A|EPHA2|RET|FCGR3B|PTEN|VHL|POLE|COL18A1|MAPK11|FCGR1A|BCL2|ABL1|FLT4|RAF1|HTRA1|FLT1|JAK3|HLA-DRA|TEK|MLH1|DPYD|PIK3CA|DNMT1|THBS2|NTRK1|ARAF|FLT3|CXCR2|JAK2|NF2|NF1|JAK1|ANXA11|MSH2|TYMS|FGFR1|GGH|KDR|FBXW7|ERBB2|CTLA4|CD274|APC|MTHFR|EGFR|IDH1|FCGR2B|FCGR2C|FCGR2A|FRK|CX3CL1|ENO2|VEGFA|MSH6|VEGFC|VEGFB|PMS2|B2M|FGFR2|SHMT1|KRAS|ARMS2|TP53|ALK|DDR2|CXCL8|C1QC|C1QB|C1QA|KIT|MET|FAS|BRAF|MMP9|C1R|PDCD1|MMP2|SLC19A1

**LUAD** CYRAMZA|IRESSA|AVASTIN|TARCEVA|OSIMERTINIB MESYLATE |AFINITOR|ALECTINIB HYDROCHLORIDE|PORTRAZZA|SELPERCATINIB|VINORELBINE|TOPOTECAN HYDROCHLORIDE|PORFIMER|METHOTREXATE SODIUM|ENTRECTINIB|IPILIMUMAB|CERITINIB|IMFINZI|GILOTRIF|TAFINLAR|ALIMTA|ER LOTINIB HYDROCHLORIDE|XALKORI|EVEROLIMUS|ALECENSA|GEMCITABINE|MEKINIST|PACLITAXEL|TAXOTERE|TRAMETINIB|CARBOPLATIN|PARAPLAT|AFINITOR DISPERZ|DOCETAXEL|DABRAFENIB MESYLATE |OPDIVO|TAGRISSO|BEVACIZUMAB|PACLITAXEL ALBUMIN-STABILIZED NANOPARTICLE FORMULATION|YERVOY|BRIGATINIB|GEMZAR|MUSTARGEN|TREXALL|VIZIMPRO|VINOR ELBINE TARTRATE|ALUNBRIG|GEMCITABINE HYDROCHLORIDE|TOPOTECAN|DACOMITINIB|ROZLYTREK|TECENTRIQ|LORBRENA|METH OTREXATE|PEMETREXED|PEMETREXED DISODIUM|ATEZOLIZUMAB|ABRAXANE|PEMBROLIZUMAB|MVASI|DOXORUBICIN HYDROCHLORIDE|PARAPLATIN|LORLATINIB|RETEVMO|RAMUCIRUMAB|MECHLORETHA MINE HYDROCHLORIDE|GEFITINIB|AFATINIB|CAPMATINIB HYDROCHLORIDE|DURVALUMAB|NIVOLUMAB|CRIZOTINIB|KEYTRUDA|TABRECTA|ZYKAD IA|AFATINIB DIMALEATE|NECITUMUMAB|ALECTINIB|ERLOTINIB

ERRF1|CRKL|STK10|STK11|MOCS3|B2M|CD226|LIFR|TAT|SMG1|PIK3CA|CAMK4|PIK3CG|RNF114|ZC3H15|WEE1|SCLY|IGF1R|XPC|MUC16|CYP3A4|CYP3A5|GATA2|CNDP1|CNDP2|DUSP28|CCND1|TUBB4B|DDX43|PTEN|STK38L|CDKN1B|CDKN1A|ALDH3 A1|HSP90B1|EDNRB|SNORD68|RP11-162A12.2|SOX10|RP11-321M21.3|JAK2|NF2|NF1|TTL|CX3CL1|ZNF516|JAK1|DSEL|DST|NCAPD3|PPARD|VEGFA |VEGFC|VEGFB|GART|RTTN|FSIP2|BNIP3|TNK2|RPS6KA3|RPS6KA6|CBL|FLNC|HES6|TL E3|RET|PRKDC|SMO|XIAP|EML4|FH|GLS|TSC2|HTRA1|TSC1|CIITA|SPARC|S100A8|ITG AV|GGH|IL17F|GSG2|RXRA|LDLR|LTK|UBE2V1|IL2RA|ALK|SLCO1B3|TP73|PBRM1|BRC A1|MYC|MAP2K5|ERBB3|DHFR|EGFR|BAX|CTNNB1|OR6B2|OR6B3|HLA-DRA|DPYD|CDK12|TWIST2|RNPEPL1|CXCR2|HNF1A|PGP|FOLH1|NOS3|AC110619.2|RP11-169F17.1|HGF|TRIP11|TRIP13|MTERFD2|EIF4E2|E2F1|BTRC|CYB5A|PPP3CB|LIF|KIAA1549|STYK1|ARAF|FCGR1A|EGF|PIK3C3|TUBA3E|TUBA3D|FGFR3|NDUFA10|ZNF236|CDH1|FGFR1|CDH7|CDKN2A|RAMP1|SCD5|FAM69C|KLK3|TPR|SNAI1|C1QC|C1QB|CSF21 NETO1|APC|ADNP|CCL4|KCNG1|COPS5|TGM2|TRIB1|SLC29A1|HSPB2|ATP7A|ATP7B|R P11-861L17.3 |CYP2D6|NEK1|STK25|EMP1|CEACAM5|TRAF3IP1|TNKS2|PRR21|IGF2|FARP21 IGFBP5|TUBB2A|TUBB2B|STK32A|TUBB|TOP1|CLK1|CLK3|GAS61EPHX1|PDGFRA|IC4|PI4KA|TPM3|ATM|CYP4B1|TIGD1|ATR|RP11-4104.1|FANCD2|FLT3|AREG|CYP1B1|POLD1|MYH9|TMEM200A|CDKL2|FANCG|FANCC| AC104809.3|FASN|FBXW7|ACVR2A|ANO7|RALG"S|MT'R11|NUDT10|MTOR|GTSCR1|LINC00908|EP300|MAPK8|FAM65C|KIF1A|PIP5K1B|C"K2|S'AMF1|POLA1|TGFB3|PTK7|ERCC2|PRDX4|ARMS2|MSH6|MCL1|HIP1|BAZ2B|CEBPB|"EBPA'AXIN1|RGL1|MAP2|RB1| ETS2|ABCB1|AURKA|SULT2A1|PRKCH|TOP1MT|FCGR2B|FCGR2C|FCGR2A|SKIL|TNFRSF11B|TUBA4A|BA1|NOTCH1|NOTCH3|TYMS|GSTP1|MYEOV2|MMP9|BCL2|MMP2|FES| TSSK1B|PMAIP1|AGER|FBXO15|GPR35|PPP1R7|LDHA|PARD6B|CBFA2T3|JAK3|ERBB2|FLCN|CIP2A|BRCA2|SHC1|ERBB4|TRIM24|IDH1|WNK1|CLCN6|PASK|PER2|PER1|RPL13|BRD4|PATZ1|FGFR4|FGFR2|ROR2|SHMT1|LRRFIP1|ATIC|RBM44|ZFYVE9|SYK|CXCL8|KIT|SPEN|GALR1|FOXA2|FKBP1A|HRAS|MED24|XRCC1|XRCC3|XRCC4|SEPT3|SEPT2|UBE 2F-SCLY|FOS|SLC9A8|ROS1|RRM2B|NFE2|ANXA11|CHEK1|CTLA4|TUBB8|SULT1C4|TUBB6 |TSHZ1|TUBB3|TUBB1|ASB1|PTPN1|ESPNL|TP53|GNAQ|ESR1|DDR2|CD274|UBE2F|EIF4EBP1|STK38|TIE1|RP11-17M16.1|AC062017.1|NTRK1|SNED1|BCAS4|NTRK3|CHST3|KCNA1|STATS|ATXN1L|ANK MY1|CBLN2|ICAM3|AC093802.1|MSH2|DPM1"SRC|'SH3|TSSK2|ACACB|MAST2|SALL3|ATRX|LYN|CDA|SGK1|VHL|GSTM1|GNAS|AGXT|CAPN10|IL2|C2orf54|ZNF407|C18orf63 |TMPO|MAP4K5|CAMK1G|CDH19|BIRC5|FCGR3A|FCGR3B|SLCO1B1|MAP2K2|FOX01| AC016757.3|MAP2K7|MAP2K6|MAP4|MAP2K4|ALDH1A1|MLLT4|SMARCA4|NIPBL|HNRNPA2B1|NRG1|MBP|ALOX5|MALT1|SMARCB1|COL18A1|HSPB8|MCM4|ILKAP|CYP2A6 |OTOS|CASP3|INSR|BCL9|MAPT|ABCC4|C1R|ABCC6|AC079612.1|ABCC3|FAM132B|AKT1|AKT2|AKT3|ATP9B|NIM1|BDNF|S100A12|MS4A2|ENOSF1|CCDC102B|GRK5|YWHA H|NTRK2|GRK1|SMAD4|ARHGAP29|MYB|BLNK|DDIT3|P4HB|SERPINB1|PMS2|SMIM21|UBR5|KRAS|SOCS6|ROBO2|GSK3A|POLE|ZADH2|GSK3B|NR1I2|TMEM189-UBE2V1|TLR9|KDM5A|SLC19A1|STMN1|HLA-A|DCLK1|FMN2|DCP1B|ARID4B|MAPKAPK3|MAPKAPK2|CHD1|TUBA1C|TUBA1B|TUBA1A|THBS2|AFP|ETV5|TMEM189|RP11-723G8.2|TOP2A|ACVRL1|KLHL30|SLC10A2|STAG2|MAP2K3|PKHD1|ITPR1|FGF2IPBK|ET V1|IL1RN|ABCG2|TLK1|ETV4|ETV6|MPL|G"PD|N'P153|UNC79|MATK|MAP2K1|SACS|ABL1|ABL2|CACNB2|MAPK3|MAPK1|KDM6A|PDCD4|DUSP6|PDCD1|MTHFR|HDLBP|FER|UNC13C|ADA|PRB2|CRNKL1|KRT20|MET|KDR|WWOX|TIMM21|CIC|SPG7|BCL11A|LATS2|LATS1|RRM2|RRM1|DSCAM|UBASH3B|TXK|PTPRF|CMPK1|TAL2|NRAS|GCLC|MLH1|DOK6|AQP12B|AQP12A|NAT2|C1QA|DAPK1|ULK2|NR3C1|RP11-94B19.4|GNA11|PLAU|GPC1|TMX3|JARID2|NPM1|ABCC10|TAB2|CHD9|GRIN2A|BRAF| CHD8|SPATA2|BAP1|ATF1

**LUSC** CYRAMZA|IRESSA|AVASTIN|TARCEVA|OSIMERTINIB MESYLATE|AFINITOR|ALECTINIB HYDROCHLORIDE|PORTRAZZA|SELPERCATINIB|VINORELBINE|TOPOTECAN HYDROCHLORIDE|PORFIMER|METHOTREXATE SODIUM|ENTRECTINIB|IPILIMUMAB|CERITINIB|IMFINZI|GILOTRIF|TAFINLAR|ALIMTA|ER LOTINIB HYDROCHLORIDE|XALKORI|EVEROLIMUS|ALECENSA|GEMCITABINE|MEKINIST|PACLITAX EL|TAXOTERE|TRAMETINIB|CARBOPLATIN|PARAPLAT|AFINITOR DISPERZ|DOCETAXEL|DABRAFENIB MESYLATE|OPDIVO|TAGRISSO|BEVACIZUMAB|PACLITAXEL ALBUMIN-STABILIZED NANOPARTICLE FORMULATION|YERVOY|BRIGATINIB|GEMZAR|MUSTARGEN|TREXALL|VIZIMPRO|VINOR ELBINE TARTRATE|ALUNBRIG|GEMCITABINE HYDROCHLORIDE|TOPOTECAN|DACOMITINIB|ROZLYTREK|TECENTRIQ|LORBRENA|METH OTREXATE|PEMETREXED|PEMETREXED DISODIUM|ATEZOLIZUMAB|ABRAXANE|PEMBROLIZUMAB|MVASI|DOXORUBICIN HYDROCHLORIDE|PARAPLATIN|LORLATINIB|RETEVMO|RAMUCIRUMAB|MECHLORETHA MINE HYDROCHLORIDE|GEFITINIB|AFATINIB|CAPMATINIB HYDROCHLORIDE|DURVALUMAB|NIVOLUMAB|CRIZOTINIB|KEYTRUDA|TABRECTA|ZYKAD IA|AFATINIB DIMALEATE|NECITUMUMAB|ALECTINIB|ERLOTINIB
ERRFI1|CRKL|STK10|STK11|MOCS3|B2M|CD226|LIFR|TAT|SMG1|PIK3CA|CAM K4|PIK3CG|RNF114|ZC3H15|WEE1|SCLY|IG"1R|X'C|MUC16|CYP3A4|CYP3A5|GATA2|C NDP1|CNDP2|DUSP28|CCND1|TUBB4B|DDX43|PTEN|STK38L|CDKN1B|CDKN1A|ALDH3 A1|HSP90B1|EDNRB|SNORD68|RP11-162A12.2|SOX10|RP11-321M21.3|JAK2|NF2|NF1|TTL|CX3CL1|ZNF516|JAK1|DSEL|DS T|NCAPD3|PPARD|VEGFA |VEGFC|VEGFB|GART|RTTN|FSIP2|BNIP3|TNK2|RPS6KA3|RPS6KA6|CBL|FLNC|HES6|TL E3|RET|PRKDC|SMO|XIAP|EML4|FH|GLS|TSC2|HTRA1|TSC1|CIITA|SPARC|S100A8|ITG AV|GGH|IL17F|GSG2|RXRA|LDLR|LTK|UBE2V1|IL2RA|ALK|SLCO1B3|TP73|PBRM1|BRC A1|MYC|MAP2K5|ERBB3|DHFR|EGFR|BAX|CTNNB1|OR6B2|OR6B3|HLA-DRA|DPYD|CDK12|TWIST2|RNPEPL1|CXCR2|HNF1A|PGP|FOLH1|NOS3|AC110619.2|RP 11-169F17.1|HGF|TRIP11|TRIP13|MTERFD2|EIF4E2|E2F1|BTRC|CYB5A|PPP3CB|LIF|KIAA1 549|STYK1|ARAF|FCGR1A|EGF|PIK3C3|TUBA3E|TUBA3D|FGFR3|NDUFA10|ZNF236|CD H1|FGFR1|CDH7|CDKN2A|RAMP1|SCD5|FAM69C|KLK3|TPR|SNAI1|C1QC|C1QB|CSF21 NETO1|APC|ADNP|CCL4|KCNG1|COPS5|TGM2|TRIB1|SLC29A1|HSPB2|ATP7A|ATP7B|R P11-861L17.3|CYP2D6|NEK1|STK25|EMP1|CEACAM5|TRAF3IP1|TNKS2|PRR21|IGF2|FARP21 IGFBP5|TUBB2A|TUBB2B|STK32A|TUBB|TOP1|CLK1|CLK3|GAS6|EPHX1|PDGFRA|IC4|P I4KA|TPM3|ATM|CYP4B1|TIGD1|ATR|RP11-41O4.1|FANCD2|FLT3|AREG|CYP1B1|POLD1|MYH9|TMEM200A|CDKL2|FANCG|FANCC| AC104809.3|FASN|FBXW7|ACVR2A|ANO7|RALGDS|MTMR11|NUDT10|MTOR|GTSCR1| LINC00908|EP300|MAPK8|FAM65C|KIF1A|PIP5K1B|CDK2|SLAMF1|POLA1|TGFB3|PTK7 |ERCC2|PRDX4|ARMS2|MSH6|MCL1|HIP1|BAZ2B|CEBPB|CEBPA|AXIN1|RGL1|MAP2|R B1|ETS2|ABCB1|AURKA|SULT2A1|PRKCH|TOP1MT|FCGR2B|FCGR2C|FCGR2A|SKIL|TNF RSF11B|TUBA4A|BAI1|NOTCH1|NOTCH3|TYMS|GSTP1|MYEOV2|MMP9|BCL2|MMP2|F ES|TSSK1B|PMAIP1|AGER|FBXO15|GPR35|PPP1R7|LDHA|PARD6B|CBFA2T3|JAK3|ERBB 2|FLCN|CIP2A|BRCA2|SHC1|ERBB4|TRIM24|IDH1|WNK1|CLCN6|PASK|PER2|PER1|RPL 13|BRD4|PATZ1|FGFR4|FGFR2|ROR2|SHMT1|LRRFIP1|ATIC|RBM44|ZFYVE9|SYK|CXCL8 |KIT|SPEN|GALR1|FOXA2|FKBP1A|HRAS|MED24|XRCC1|XRCC3|XRCC4|SEPT3|SEPT2|U BE2F-SCLY|FOS|SLC9A8|ROS1|RRM2B|NFE2|ANXA11|CHEK1I|CTLA4|TUBB8|SULT1C4|TUBB6 |TSHZ1|TUBB3|TUBB1|ASB1|PTPN1|ESPNL|TP53|GNAQ|ESR1|DDR2|CD274|UBE2F|EIF 4EBP1|STK38|TIE1|RP11-17M16.1|AC062017.1|NTRK1|SNED1|BCAS4|NTRK3|CHST3|KCNA1|STAT6|ATXN1 L|ANK MY1|CBLN2|ICAM3|AC093802.1|MSH2|DPM1|SRC|MSH3|TSSK2|ACACB|MAST2|SALL3 |ATRX|LYN|CDA|SGK1|VHL|GSTM1|GNAS|AGXT|CAPN10|IL2|C2orf54|ZNF407|C18orf6 3|TMPO|MAP4K5|CAMK1G|CDH19|BIRC5|FCGR3A|FCGR3B|SLCO1B1|MAP2K2|FOXO1 |AC016757.3|MAP2K7|MAP2K6|MAP4|MAP2K4|ALDH1A1|MLLT4|SMARCA4|NIPBL|HNRNPA2B1|NRG1|MBP|ALOX5|MALT1|SMARCB1|COL18A1|HSPB8|MCM4|ILKAP|CYP2A6 |OTOS|CASP3|INSR|BCL9|MAPT|ABCC4|C1R|ABCC6|AC079612.1|ABCC3|FAM132B|AKT1|AKT2|AKT3|ATP9B|NIM1|BDNF|S100A12|MS4A2|ENOSF1|CCDC102B|GRK5|YWHAH|NTRK2|GRK1|SMAD4|ARHGAP29|MYB|BLNK|DDIT3|P4HB|SERPINB1|PMS2|SMIM21|UBR5|KRAS|SOCS6|ROBO2|GSK3A|POLE|ZADH2|GSK3B|NR1I2|TMEM189-UBE2V1|TLR9|KDM5A|SLC19A1|STMN1|HLA-A|DCLK1|FMN2|DCP1B|ARID4B|MAPKAPK3|MAPKAPK2|CHD1|TUBA1C|TUBA1B|TUBA1A|THBS2|AFP|ETV5|TMEM189|RP11-723G8.2|TOP2A|ACVRL1|KLHL30|SLC10A2|STAG2|MAP2K3|PKHD1|ITPR1|FGF2|PBK|ET V1|IL1RN|ABCG2|TLK1|ETV4|ETV6|MPL|G6PD|NUP153|UNC79|MATK|MAP2K1|SACS|ABL1|ABL2|CACNB2|MAPK3|MAPK1|KDM6A|PDCD4|DUSP6|PDCD1|MTHFR|HDLBP|FER|UNC13C|ADA|PRB2|CRNKL1|KRT20|MET|KDR|WWOX|TIMM21|CIC|SPG7|BCL11A|LATS2|LATS1|RRM2|RRM1|DSCAM|UBASH3B|TXK|PTPRF|CMPK1|TAL2|NRAS|GCLC|MLH1|DOK6|AQP12B|AQP12A|NAT2|C1QA|DAPK1|ULK2|NR3C1|RP11-94B19.4|GNA11|PLAU|GPC1|TMX3|JARID2|NPM1|ABCC10|TAB2|CHD9|GRIN2A|BRAF | CHD8|SPATA2|BAP1|ATF1

**PRAD** PROVENGE|RADIUM 223 DICHLORIDE|RUBRACA|ELIGARD|XTANDI|FIRMAGON|FLUTAMIDE|ENZALUTAMIDE|NILU TAMIDE|LYNPARZA|ERLEADA|DEGARELIX ACETATE|CABAZITAXEL|MITOXANTRONE|ABIRATERONE|NILANDRON|JEVTANA|LEUPROLI DE ACETATE|BICALUTAMIDE|LUPRON DEPOT|RUCAPARIB CAMSYLATE|GOSERELIN|GOSERELIN ACETATE|XOFIGO|MITOXANTRONE HYDROCHLORIDE|LUPRON|DEGARELIX|MELPHALAN|ESTRAMUSTINE|TAXOTERE|ZOLADE X|OLAPARIB|DAROLUTAMIDE|ESTRAMUSTINE PHOSPHATE SODIUM|DOCETAXEL|NUBEQA|CASODEX|ABIRATERONE ACETATE|APALUTAMIDE|SIPULEUCEL-T|ESTRAMUSTINE PHOSPHATE|ZYTIGA GSR|STK11|PEBP1|FST|TUBA3E|TUBA3D|CDH1|PIK3CA|DNMT1|PZP|IRAK2|CB LC|BRCA2|TH|IL2RA|IFNG|IDH2|IDH1|AQP9|KLK3|RPL13|BRD4|MUC16|CYP3A4|CYP3A5|ROR2|KRAS|OPLAH|TUBB4B|AHR|PTEN|VIP|ATR|TOP2A|VDR|MAP1A|HRAS|ABL1| MGMT|XRCC1|ARID4B|ATP7A|XRCC41LHCGR|NFE2L2|NR1I2|ASXL1|TUBA1B|TUBA1A|TUBA1C|PPARGC1A|TUBB6|CDKN1A|CHEK2|CHEK1|IGF2|"TLA4'TUBB8|SULT1C4|STAG2| PARP1|PARP2|TUBB3|PPARD|CHST3|TUBB1|TUBB2A|TUBB"B|AB'G2|TP53|CSNK1A1|ESR1|MPO|PALB2|TUBB|PCDH15|GAS6|EPHX1|HDAC2|HDAC4|TLE3|EPHA4|CYP4B1|SM AD3|GNRHR|FH|SLC10A2|PRAME|CACNB2|CYP2B6|CFLAR|FANCC|FANCA|MSH2|FBXW 7|GNRHR2|MTHFR|NAT2|HDLBP|DLL1|ESR2|ADK|SLCO1B1|ACPP|RPSA|CYP17A1|GST M1| MET|GNRH1|RIPK4|BRCA1|TOP2B|RRM1|SPG7|KALRN|BIRC5|PRDX4|BAX|MAP2| VHL|SLCO1B3|MCL1|MAP4|UGT1A5|XRCC3|MYOD1|ATM|MLLT6|SNORD68|ABCB1|PG P"LDHA'FOLH1|TRAF2|TET2|KMT2D|PRL|PLAT|AR|CYP2A6|TUBA4A|ERBB2|HEXB|NOTCH1|PARP3|CDK12|GSTP1|PTPRC|MAPT|ABCC6|MMP1|ABCC1|BCL2

**SKCM** SONIDEGIB|FLUOROURACIL-TOPICAL|COBIMETINIB|VINBLASTINE SULFATE|ERIVEDGE|VISMODEGIB|PACLITAXEL|VORINOSTAT|TRAMETINIB|ODOMZO|ALD ARA|COBIMETINIB FUMARATE|DACARBAZINE|DABRAFENIB MESYLATE|VEMURAFENIB|1218778-77-8|ERISMODEGIB|DABRAFENIB|1187431-43-1|5-FU|VINBLASTINE|EFUDEX|ALITRETINOIN|IMIQUIMOD|AVELUMAB PDE4DIP|LIF|CRKL|STK11|PCSK6|HDAC3|PTHLH|AKT1|ARAF|TNRC6B|HIPK4|B DNF|PI4KB|RARA|RARB|TUBA3E|TUBA3D|RARG|BRCA1|PIK3CA|LRP1B|SYNE1|PIK3CG| SMAD4|PHC1|EPHA6|PRKG1|MYC|MYB|ERBB2|DDIT3|TPO|IDH2|KLK3|MGMT|CYP3A4 |CYP3A5|FGFR2|FGFR3|TBXA2R|KRAS|SMO|SYK|CXCL8|DDX43|ROBO2|STAG2|CSF2|KIT|PTEN|TLR7|TUBB4B|C11|IC10|TLR8|TUBE1|BRCA2|STMN1|VKORC1|CDKN1A|DCLK2 |ABL1|CRTC3|EDNRB|NEK11|NPRL2|MYCN|TCF3|NR1I2|TUBA1C|TUBA1B|TUBA1A|JUN|MKL1|EPHA7|TIAM1|CTNNB1|JAK2|NFE2|NF1|TUBD1|TRPM6|MYD88|TOP2A|TRH|EPHB1|TUBB8|FOS|TUBB6|STAG3|TUBB3|HDAC9|TUBB1|BLM|MAP2K3|RHOH|HRAS|ATXN1L|PLAT|TUBB2A|ING1|TUBB2B|ETV1|TP53|GNAQ|RPS6KA6|CD274|G6PD|NYNRIN|TUBB|EIF4EBP1|IC1|PDGFRB|PDGFRA|HDAC2|HDAC5|HDAC4|HDAC7|HDAC6|TLE3|HDAC8|ATM|SRGAP3|TIGD1|AKAP12|SACS|FH|WDHD1|RAF1|NCOA3|CYP1B1|BTG1|CGA|CSF1R|NR2F2|SPARC|FASN|NBN|TBX18|PDCD4|DUSP6|ERCC2|FANCI|FBXW7|SRC| MTHFR|NAT2|RXRG|RXRA|RXRB|ETV5|ADK|ETV4|IL2RA|ITK|MAP2K2|IFNA1|MET|CDK2|EZH2|FGFR1|CIC|POLA2|MAP4|MAP2K5|ACVR2A|KALRN|BIRC5|LIMK1|EGFR|BAX|PTCH1|MAP2|VHL|MAP2K1|CDKN2A|MAP2K7|MAP2K6|RRM1|MAP2K4|MITF|DSCAM|ARID4B|TEAD4|PGD|SIK1|NRAS|CDK17|RB1|ETS2|ABCB1|AURKA|PGP|ABCB4|NRG1|C15orf2|EPM2AIP1|ULK2|PRKCH|SMARCB1|RAC1|PLAU|FCGR2A|MCM3|SUFU|TUBA4A| NPM1|ABCC10|EIF4E2|CASP3|E2F1|NOTCH1|NOTCH2|TUBG1|SOX10|GSTP1|BRAF|MAPT|GNA11|BAP1|ABCC3|MMP2|BCL2

**STAD** CYRAMZA|DOXORUBICIN HYDROCHLORIDE|TRIFLURIDINE AND TIPIRACIL HYDROCHLORIDE|PEMBROLIZUMAB|EVEROLIMUS|5-FU|RAMUCIRUMAB|TRASTUZUMAB|TAXOTERE|HERCEPTIN|MITOMYCIN|SUNITINIB|SU NITINIB MALATE|LANREOTIDE|DOCETAXEL|MITOMYCIN C|KEYTRUDA|LONSURF|REGORAFENIB|FLUOROURACILINJECTION ERBB3|PDGFRB|STK11|AKT1|AKT2|AKT3|ARAF|MLH1|FCGR1A|PMS2|TAT|B2M|PIK3CA|NTRK1|PIK3CG|EDN1|FGFR1|BRCA1|FLCN|BRCA2|AURKB|IFNG|KRT20|EPHA2|BGLAP|KLK3|RPL13|BRD4|MUC16|CYP3A4|CYP3A5|FGFR2|KRAS|ARID1A|C1QC|C1QB|C1QA|KIT|NR1I2|XRN1|FKBP1A|APC|HRAS|HIF1A|ABL1|PTPRB|XRCC1|HSPB2|ARID4B|ATP7A|XRCC4|PTEN|U2AF1|RAF1|XDH|JAK2|NF2|NF1|JAK1|FLT1|TOP2A|IGF2|SULT1C4|TUBB3|TUBB1|PPARD|CHST3|TP53|GNAQ|ESR1|DDR2|CD274|MPL|HMOX1|TUBB|VWF|GAS6|EPHX1|PDGFRA|MKI67|TLE3|ATM|CYP4B1|RET|POLE|MAPK10|MAPK11|FH|IL1A|FLT4|TSC2|FLT3|TSC1|SLC10A2|TEK|CSF1R|FANCC|MSH2|KDR|FBXW7|MSH3| MTHFR|NAT2|CA9|CGB5|AGAP1|AC064874.1|MTOR|MSH6|LGALS2|SLCO1B1|ALK|GSTM1|SSTR1|MET|SSTR3|SSTR2|SSTR5|PBRM1|PDCD1|PALB2|ERBB2|MAP4|SPG7|DHFR|BIRC5|PRDX4|EGFR|BAX|FCGR3B|CTNNB1|VHL|SLCO1B3|MCL1|RRM1|FCGR3A|XRCC 3|HLADRA|DPYD|MAP2|NRAS|RB1|SNORD68|FRK|ABCB1|PGR|PGP|ABCB4|FOLH1|ANXA1|P DGFA|PDGFB|PDGFC|PDGFD|FCGR2B|FCGR2C|FCGR2A|CYP2A6|HGF|EPCAM|BAP1|CYP7B1|NOTCH1|TYMS|GSTP1|BRAF|MAPT|GNA11|C1S|C1R|ABCC6|KRT18|BCL2

**UCEC** HYDROXYPROGESTERONE CAPROATE|DACTINOMYCIN|VINBLASTINE|METHOTREXATE SODIUM|LENVIMA|PEMBROLIZUMAB|MEGESTROL ACETATE|KEYTRUDA|METHOTREXATE|VINBLASTINE SULFATE|LENVATINIB MESYLATE SALL3|PCSK6|FBXO15|DUSP28|B2M|CD226|RB1|BDNF|S100A12|GPR35|C18orf63|TAT|PPP1R7|ASB1|TUBA3E|TUBA3D|CCDC102B|PIK3CB|NTRK1|ANKMY1|NDUFA10|ZNF236|FAM132B|JAK2|MYC|SCLY|CDH7|DDIT3|IDH2|ALOX5|RAMP1|CLCN6|PASK|FAM69C|BGLAP|KLK3|COIL|PMS2|SMIM21|CNDP1|CNDP2|KRAS|ATIC|RBM44|CCND1|ZNF516|CSF1|CSF2|KIT|PTEN|GALR1|GRB2|TUBB4B|NETO1|SULT2A1|TUBE1|SLC19A1|CDKN1B|CDKN1A|ANO7|SNED1|CRTC3|ZADH2|AC110619.2|ARID4B|RP11-162A12.2|UBE2F-SCLY|RP11-861L17.3|TUBB8|NR1I2|TUBA1C|TUBA1B|TUBA1A|CEL|JUN|RP11-94B19.4|STK25|RP11-321M21.3|TNFRSF8|NF1|JAK1|TUBD1|TRAF3IP1|FLT1|RP11-723G8.2|PRR21|TOP2A|DSEL|TRH|FARP2|KLHL30|TUBB6|TSHZ1|TUBB3|ESPNL|BLM |TUBB1|TUBB2A|TUBB2B|IL1RN|TP53|CD274|ATP9B|TUBB|UBE2F|TIMM21|OTOS|HES6|HDAC4|CBLN2|AC062017.1|RNPEPL1|CD5|POLE|RP11-4104.11|FH|FLT4|GLS|FLT3|KCNA1|CGA|AFP|TPM3|NR2F2|CDH19|ICAM3|S100A8|TFPI |KIF1A|MSH2|FANCI|PDCD1|TBXA2R|NTF3|MTHFR|HDLBP|RTTN|BAX|GTSCR1|ADA|LINC00908|TNFRSF1B|IL2RA|ALK|GSTM1|AGXT|IL15|CAPN10|PGR|IL2|C2orf54|ZNF4071 SLAMF1|RP11-17M16.1|DHFR|BIRC5|MSH3| EGFR|MSH6|FCGR3B|SLCO1B1|AC016757.3|AC104809.31 OR6B2|OR6B3|HLA-DRA|AC079612.1|TWIST2|NRAS|PTHLH|MLH1|ABCB1|DOK6|AQP12B|AQP12A|ABCB41 NRG1|SEPT2|MBP|LRRFIP1|FOLH1|NR3C1|HSPB2|MTR|RP11-169F17.1|GPC1|SOCS6|ILKAP|TMX3|HGF|TUBA4A|CYB5A|MTERFD2|E2F1|PER2|NOTCH1|TUBG1|TYMS|AC093802.1|KDR|MYEOV2|BRAF|ABCC4|MMP2

**UCS** HYDROXYPROGESTERONE CAPROATE|DACTINOMYCIN|VINBLASTINE|METHOTREXATE SODIUM|LENVIMA|PEMBROLIZUMAB|MEGESTROL ACETATE|KEYTRUDA|METHOTREXATE|VINBLASTINE SULFATE|LENVATINIB MESYLATE SALL3|PCSK6|FBXO15|DUSP28|B2M|CD226|RB1|BDNF|S100A12|GPR35|C18orf63|TAT|PPP1R7|ASB1|TUBA3E|TUBA3D|CCDC102B|PIK3CB|NTRK1|ANKMY1|NDUFA10|ZNF236|FAM132B|JAK2|MYC|SCLY|CDH7|DDIT"|IDH'|ALOX5|RAMP1|CLCN6|PASK|FAM69C|BGLAP|KLK3|COIL|PMS2|SMIM21|CNDP1|CNDP2|KRAS|ATIC|RBM44|CCND1|ZNF516|CSF1|CSF2|KIT|PTEN|GALR1|GRB2|TUBB4B|NETO1|SULT2A1|TUBE1|SLC19A1|CDKN1B|CDKN1A|ANO7|SNED1|CRTC3|ZADH2|AC110619.2|ARID4B|RP11-162A12.21UBE2F-SCLY|RP11-861L17.3|TUBB8|NR1I2|TUBA1C|TUBA1B|TUBA1A|CEL|JUN|RP11-94B19.4|STK25|RP11-321M21.3|TNFRSF8|NF1|JAK1|TUBD1|TRAF3IP1|FLT1|RP11-723G8.2|PRR21|TOP2A|DSEL|TRH|FARP2|KLHL30|TUBB6|TSHZ1|TUBB3|ESPNL|BLMT UBB1|TUBB2A|TUBB2B|IL1RN|TP53|CD274|ATP9B|TUBB|UBE2F|TIMM21|OTOS|HES6| HDAC4|CBLN2|AC062017.1|RNPEPL1|CD5|POLE|RP11-41O4.1|FH|FLT4|GLS|FLT3|KCNA1|CGA|AFP|TPM3| NR2F2|CDH19|ICAM3|S100A8|TFPI    |KIF1A|MSH2|FANCI|PDCD1|TBXA2R|NTF3|MTHFR|HDLBP|RTTN|BAX|GTSCR1|ADA|LI NC00908|TNFRSF1B|IL2RA|ALK|GSTM1|AGXT|IL15|CAPN10|PGR|IL2|C2orf54|ZNF407| SLAMF1|RP11-17M16.1|DHFR|BIRC5|MSH3|EGFR|MSH6|FCGR3B|SLCO1B1|AC016757.3|AC104809.31 OR6B2|OR6B3|HLA-DRA|AC079612.1|TWIST2|NRAS|PTHLH|MLH1|ABCB1|DOK6|AQP12B|AQP12A|ABCB41 NRG1|SEPT2|MBP|LRRFIP1|FOLH1|NR3C1|HSPB2|MTR|RP11-169F17.1|GPC1|SOCS6|ILKAP|TMX3|HGF|TUBA4A|CYB5A|MTERFD2|E2F1|PER2|NOTC H1|TUBG1|TYMS|AC093802.1|KDR|MYEOV2|BRAF|ABCC4|MMP2

### 1-24. Calculation of TC score

For 17 cancer types, the present inventors assigned a therapeutic candidate (TC) for each drug. The TC score is aggregated from the network propagation value of the drug's target and biomarker genes by taking their root-mean-square (RMS) as follows:

$$S_{(d,c)} = \sqrt{\frac{\sum P_{target}^2}{n_{target}}}$$

, where $S_{(d,c)}$ is the TC score of the drug d in cancer type c, $P_{target}$ is the propagation score of the drug targets in cancer type c and $n_{target}$ is the number of drug targets.

### 1-25. Performance of predicting the cytotoxic effect of drugs

To obtain drug response data of human cancer cell-line, the present inventors downloaded 'secondary-screen-dose-response-curve-parameters' from PRISM database, which included 1,448 compounds screened against 499 cell lines. Among the 17 cancer types, the present inventors used 15 cancer types, including cancer cell lines, which have secondary screen data in PRISM. The cytotoxic effect of the drug on each cancer type was calculated by taking the median $IC_{50}$ values of the drug in cancer cell lines belonging to the corresponding cancer.

The network's performance in predicting the cytotoxic effect of drugs on cancer was measured using the Spearman correlation coefficient between TC score and negative log base ten of the drug's median $IC_{50}$ values for the cancer. The synonyms of drugs between PRSIM and PanDrugs were mapped with PubChem ID using the Pubchempy Python module.

### 1-26. Performance of predicting reversal gene expression(RGE) effect of drugs in COADREAD

To estimate the efficacy of the drug by altering the gene expression to be a reversal of that in cancer conditions, the present inventors used the L1000 dataset from CMap[9]. From the L1000 dataset, the present inventors obtained 6,056 compounds with PubChem IDs and expression profiles for 978 genes directly measured in 15 cancer cell lines whose primary site was the large intestine: CL34, HCT116, HELA, HT115, HT29, LOVO, MDST8, NCIH508, NCIH716, RKO, SNU1040, SNUC5, SW480, SW620, and SW948. The present inventors initially computed the RGE effect between the differential gene expression of COADREAD (obtained via CREEDS[10], signature ID: dz552) and the compound's transcriptional profile using Zhang's connection score [11], that is, the absolute value of the anti-correlated, standardized ranked connection score. Since drugs have multiple transcriptional profiles for each cell line depending on the treatment dose and time, the present inventors calculated the max RGE effect for each cell line and used the median RGE effect across cell lines as a representative value. Finally, the performance of the network in predicting the drug's RGE effect was measured using the Spearman correlation coefficient between the TC score and the RGE effect of drugs that exist in both PanDrugs and CMap.

### 1-27. Selection of repurposing candidates

Among the 1,702 approved drugs in Pandrugs [12] with drug target information, the present inventors selected drugs with a rank percentile of TC score above 0.9. This resulted in 333 approved drugs with new therapeutic indications across the 17 cancer types.

For further experimental validation in LIHC, the present inventors selected ixazomib citrate, which has the highest TC score among repurposing candidates that were only predicted by the co-essentiality network.

### 1-28. Cell culture

The human LIHC cell lines SNU398, SK-HEP-1, and Huh7 were obtained from the Korean Cell Line Bank (Seoul, Korea), and HepG2 cells were obtained from the American Type Culture Collection (Manassas, VA, USA). SNU398 and Huh7 were cultured in Roswell Park Memorial Institute (RPMI) 1640 medium (Welgene) supplemented with 10% fetal bovine serum (FBS, Merck), 100 IU/mL penicillin, and 100 $\mu$g/mL streptomycin (Welgene). SK-HEP-1 and HepG2 were maintained in Eagle's Minimum Essential medium (EMEM, Lonza) supplemented with 10% FBS. All cells were incubated at 37 °C in a humidified incubator with 5% $CO_2$.

### 1-29. Cell viability assay

To assess cell viability, SNU398, SK-HEP-1, Huh7, and HepG2 cells were plated in 96-well plates at a density of $8 \times 10^3$ or $1 \times 10^4$ cells per well. After incubation for 24 h, the medium was removed, and the cells were maintained in 100 $\mu$L of the medium supplemented with different concentrations of ixazomib citrate (MedChemExpress). After incubation for 72 h, cell viability was determined using the phenazine methosulfate (PMS)/3-(4,5-dimethylthiazol-2-yl)-5-(3-carboxymethoxyphenyl)-2-(4-sulfophenyl)-2H-tetrazolium, inner salt (MTS) assay using the Cell Titer 96 Aqueous Non-Radioactive Cell Proliferation Assay kit (Promega, Madison, WI, USA) according to the manufacturer's instructions. The absorbance at 490 nm was measured using a microplate reader (Multiskan SkyHigh Microplate Spectrophotometer, Thermo Scientific). $IC_{50}$ values were calculated from dose-response curves using GraphPad Prism 9 (GraphPad Software).

### 1-30. Colony formation assay

SNU398, SK-HEP-1, Huh7, and HepG2 cells were plated in 24-well plates at a density of $4 \times 10^2$ or $6 \times 10^2$ cells per well. After incubation for 24 h, the medium was removed, and the cells were maintained in the presence of 500 $\mu$L of medium supplemented with different concentrations of ixazomib citrate. The medium was changed every two days. After seven days, colonies were fixed in 10% formalin and stained with 1% crystal violet.

### Example 2. Construction of the co-essentiality network for drug discovery in cancer

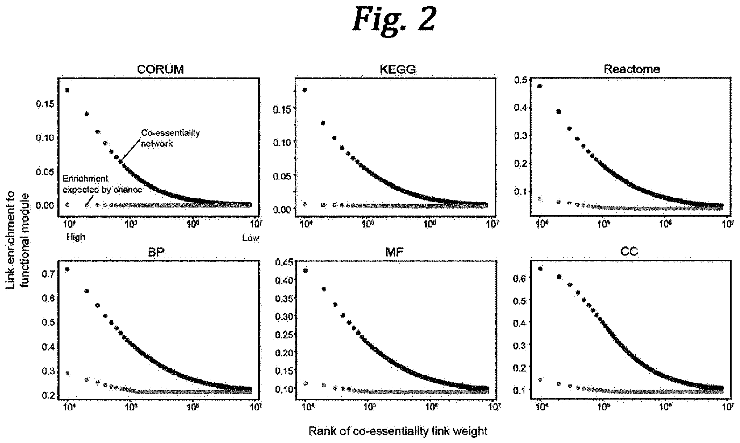

To construct a network for the identification of therapeutic targets and drug repurposing candidates of each cancer type, co-essentiality links were inferred from the correlation of the gene essentiality profiles of cancer cells **(Fig. 1A).** A co-essentiality network was constructed with 18,119 genes and 8,105,180 co-essentiality links based on gene-level essentiality scores using a CRISPR screening dataset from the dependency map (Depmap) project. Co-essentiality links represent the similarity of essentiality profile between two genes across cancer cells and are measured using the context likelihood of relatedness (CLR) algorithm. To validate that the co-essentiality links from our method are relevant to biological function, the present inventors tested whether co-essentiality links are enriched in curated pathways. The present inventors found that the co-essentiality links indicate a strong functional relationship between the two genes **(Fig. 1B, Fig. 2),** resulting in similar growth phenotypes across various cancer cells. For example, in KEGG pathways, gene pairs with greater link weights were likely within the same pathways **(Fig. 1B, black points),** and this tendency was higher than expected by chance **(Fig. 1B, gray points).** Similarly, gene pairs with greater link weights likely reside within the same biological modules from five independent datasets: protein complexes (CORUM), molecular pathways(REACTOME), and Gene Ontology annotations [GO: BP (biological process), MF (molecular function), and CC (cellular components)].

### Example 3. Effectiveness of the co-essentiality network in identifying cancer-related pathways

The present inventors found that the co-essentiality network depicts cancer-related pathways (CRPs) related to oncogenesis and hallmarks of cancer. To investigate the advantages of using the co-essentiality network, the present inventors compared the co-essentiality network with three other molecular networks: a PPI network (BioGRID; 18,708 nodes; 434,527 links) a co-expression network (19,120 nodes; 12,759,793 links), and a co-methylation network (16,333 nodes; 10,193,089 links). The co-essentiality network had a higher enrichment of CRPs **(Fig. 1C, left panel)** with an odds ratio of 4.65, compared with the PPI network (odds ratio = 0.225), the co-expression network (odds ratio = 0.133), and the co-methylation network (odds ratio = 1.50). Co-essentiality links showed significant enrichment to CRPs (hypergeometric test p-value = $3.12 \times 10^{-3}$), whereas other molecular links did not show significant enrichment to CRPs. Co-essentiality links had the highest relative modularity among the 15 CRPs and were greater than 6.83 CRPs, which was expected by chance **(Fig. 1C right, Table 3).**

For example, in 1 the of 15 CRPs, the mTOR signaling pathway (KEGG: hsa04150), targeted by many anticancer drugs, the co-essentiality network showed higher modularity than the other networks **(Fig. 1D,** rank percentile = 0.898, 0.697, 0.177, and 0.109 of relative modularity for the co-essentiality network, BioGRID, co-expression network, and co-methylation network, respectively). To determine whether co-essentiality links were likely to be found between gene pairs in CRPs, the present inventors measured the relative modularity of genes in each KEGG pathway and compared it with three other molecular networks **(Table 3).** Since modularity is biased to degree of the nodes, the present inventors used degree-controlled modularity measure normalized by permutation test with degree matched random nodes. These results suggest that co-essentiality links can detect more oncogenic relationships compared with other molecular links, leading to improve identifying genes critical for cancer treatment.

**Example 4. High modularity of cancer driver genes in the co-essentiality network**

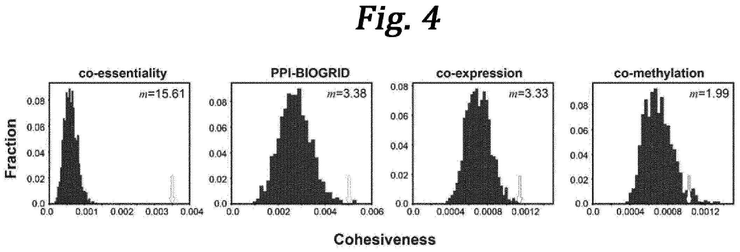

To verify the effectiveness of co-essentiality links for identifying therapeutic targets in cancer, the present inventors investigated the modularity of disease genes in the network, since the modularity of disease genes is indicative of the relevance underlying information to a particular disease phenotype. The present inventors used cancer driver genes whose mutations cause cancer as disease genes. The co-essentiality links resulted in a denser module of cancer driver genes compared with other molecular links. For 17 out of 19 cancer types, the modularity (m) of driver genes was the highest in the co-essentiality network compared with the other three molecular networks **(Fig. 3A).** The present inventors used degree-controlled modularity measure normalized by permutation test (see Methods). For instance, in the case of lung squamous cell carcinoma (LUSC), the co-essentiality network showed the highest modularity for driver genes ($m_{\text{co-essentiality network}}$ = 15.61) among all molecular networks ($m_{\text{PPI-BioGRID}}$ = 3.38, $m_{\text{co-expression network}}$ = 3.33, and $m_{\text{co-methylation network}}$ = 1.99) **(Fig. 3B to Fig. 3E, Fig. 4).** For example, comparing the driver genes of LUSC **(Fig. 3B)** among the networks **(Fig. 3C to Fig. 3E),** the subnetwork of driver genes in the co-essentiality network was more densely connected. *FAT1* and *FGFR2* connect to the subnetwork of LUSC driver genes in the co-essentiality network but are not connected to other molecular networks. *FAT1* has co-essentiality interactions with *RASA1, CUL3,* and *ARHGAP35. FGFR2* has co-essentiality interaction with *FAT1.* Indeed, *FAT1* is a biomarker of immune checkpoint blockade for lung cancer, and inhibition of *FGFR2* is also effective for the treatment of lung cancer. The 17 cancer types with the highest modularity in the co-essentiality network were selected for further analyses of precision oncology tasks.

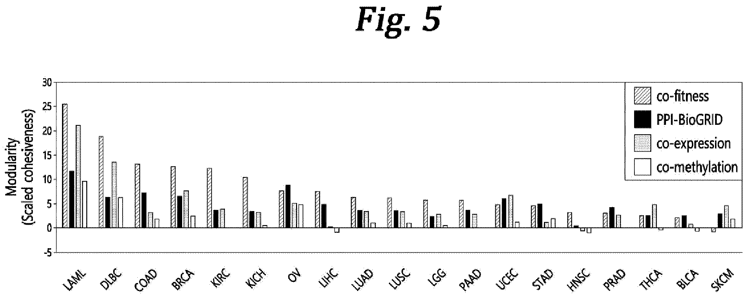

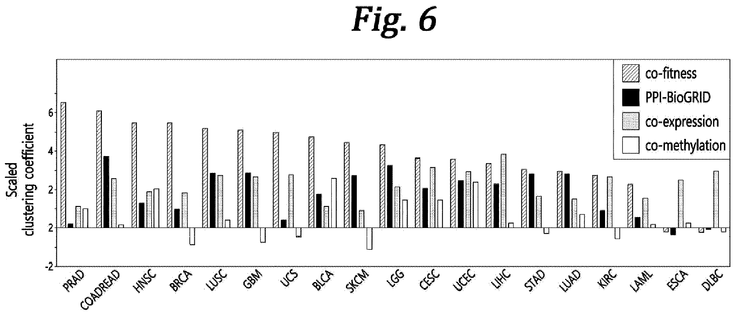

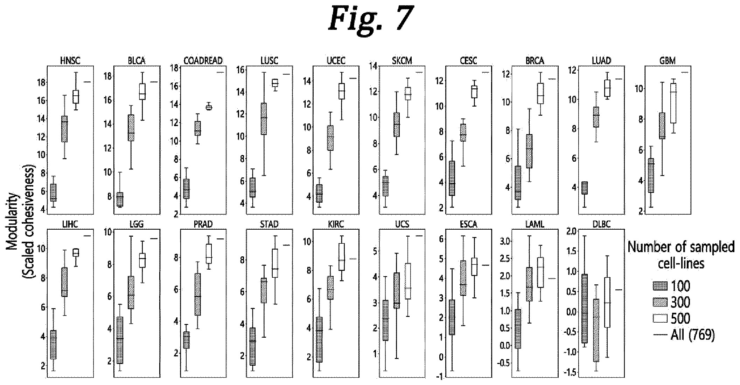

To investigate robustness of the results, the present inventors leveraged a different driver gene resource, cancer gene census Cancer Gene Census (CGC), the modularity (*m*) of driver genes was the highest in the co-essentiality network in 12 out of the 19 cancer types **(Fig. 5).** The present inventors also used a different modularity measurement, the clustering coefficient, which focuses more on link connectivity. For 16 out of the 19 cancer types, the clustering coefficient of driver genes in the co-essentiality network was higher than that in other molecular networks **(Fig. 6).** Furthermore, whether the modularity of driver genes depends on the number of cell-lines used to construct the co-essentiality network should also be determined. The present inventors found that as more cancer cell lines were compiled, co-essentiality networks were improved, and found more disease modules which can be used as potential therapeutic targets **(Fig. 7).**

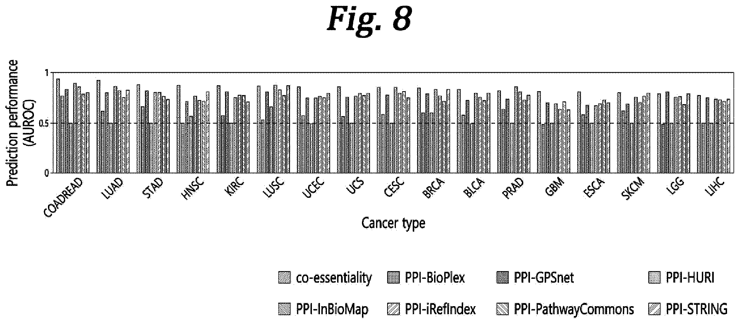

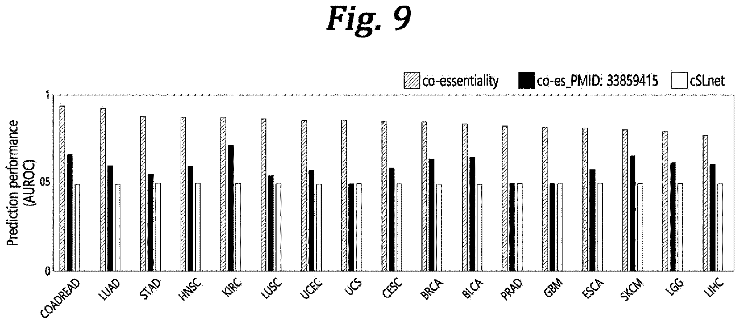

The present inventors further investigated whether the high modularity of co-essentiality links could be utilized to identify the driver genes of each cancer type via connection with known driver genes based on guilt-by-association **(Fig. 3F).** The present inventors found that co-essentiality links are more suitable than other molecular links for identifying cancer driver genes. The co-essentiality network outperformed the three other molecular networks in discovering driver genes using guilt-by-association in 15 of the 17 cancer types **(Fig. 3G).** For example, in the case of head and neck cancer, co-essentiality links improved driver gene discovery by 17%, 16%, and 33% than other networks, respectively. Similarly, the co-essentiality network with guilt-by-association showed higher performance in 13 of the 17 cancer types compared with seven other PPI networks **(Fig. 8):** BioPlex[13], GPSnet [14], HURI [15], Inbiomap [16], iRefIndex [17], Pathway Commons [18], and STRING [19]. In addition, the co-essentiality network showed a higher performance in identifying driver genes than another co-essentiality network in a previous study (PMID: 33859415) [20], and the genetic interaction network based on synthetic-lethal relationship **(Fig. 9)** [21].

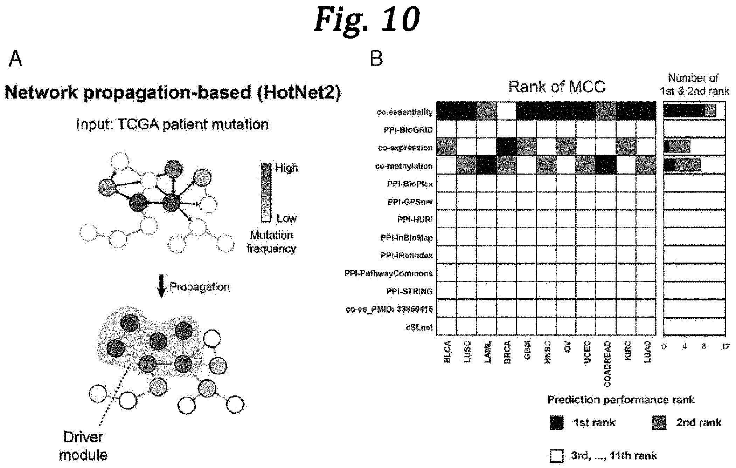

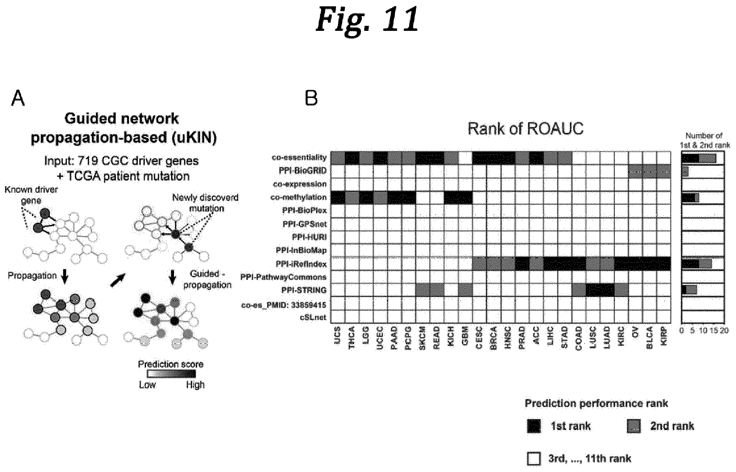

Using two additional reported methods for driver gene identification, the present inventors found that co-essentiality links resulted in the best performance improvements and had a robust benefit for the identification of cancer driver genes. The performance for driver genes identification was measured using network propagation methods such as Hotnet2 [22] **(Fig 10A)** and uKIN [23] **(Fig. 11A).** In both methods, co-essentiality links identified driver genes better than any other molecular links. For 8 out of 11 cancer types used in the Hotnet2 study, the co-essentiality networks with the Honet2 algorithm performed better in identifying driver genes compared with 12 networks **(Fig. 10B).** Likewise, when using the uKIN algorithm, the co-essentiality network showed at least 2nd ranked performance in 16 of the 24 cancer types used in the uKIN study **(Fig. 11B).** Taken together, our results that the co-essentiality network captures more relationships between cancer driver genes suggest that co-essentiality links are more relevant to cancer phenotypes than other molecular links.

**Example 5. Driver modules of the co-essentiality network can differentiate patient survival.**

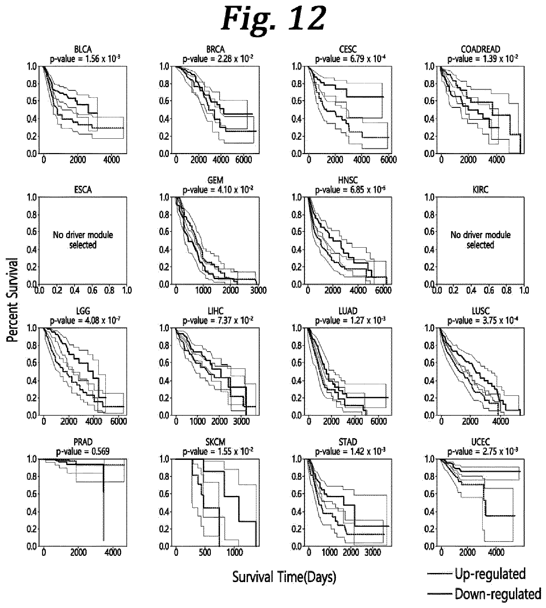

To examine the clinical relevance of the modularity of driver genes in the co-essentiality network, the present inventors evaluated whether the network modules derived from these driver genes improve our understanding of cancer prognosis in terms of patient survival **(Fig. 3H).** Among the 17 TCGA cancer types, 16 with enough patients' survival data were tested. The present inventors found that driver modules from the co-essentiality network were more informative for stratifying patients along with overall survival than other molecular networks **(Fig. 3I, Fig. 12).** In each network, driver modules representing biological pathways in significant network proximity to the driver genes were tested to discern patient survival. Specifically, patients were divided into two groups based on the overall expression of the driver module, and the significance of the survival difference between the two groups was measured using the log-rank test.

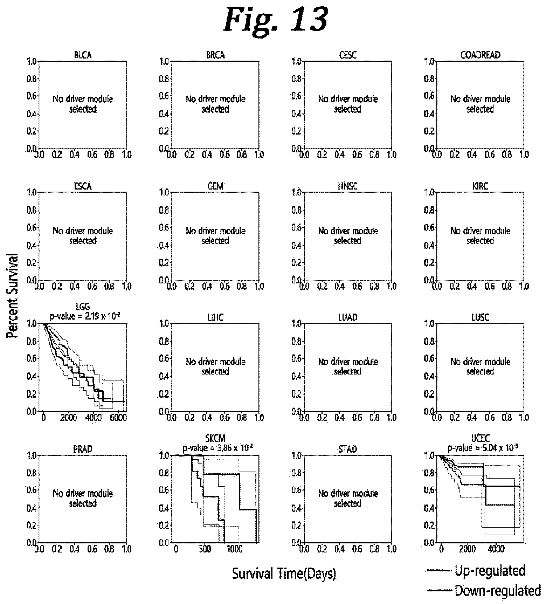

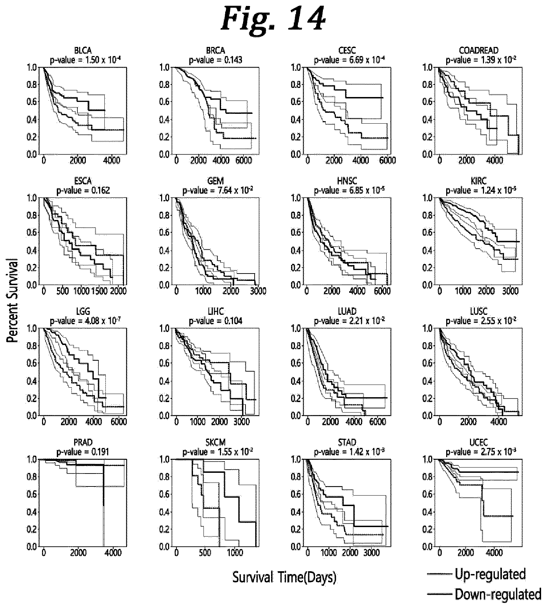

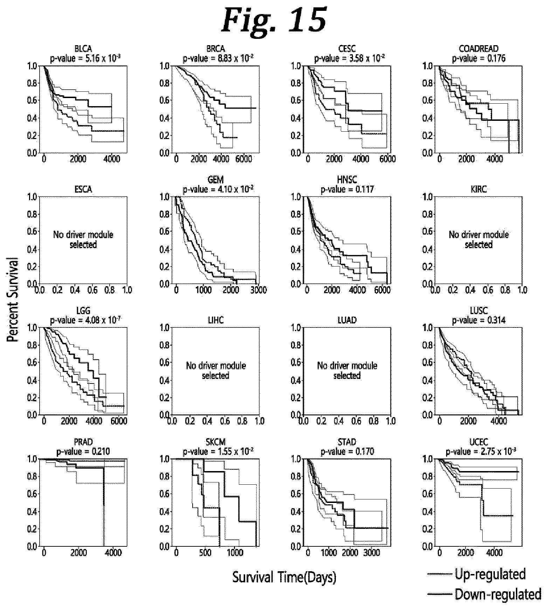

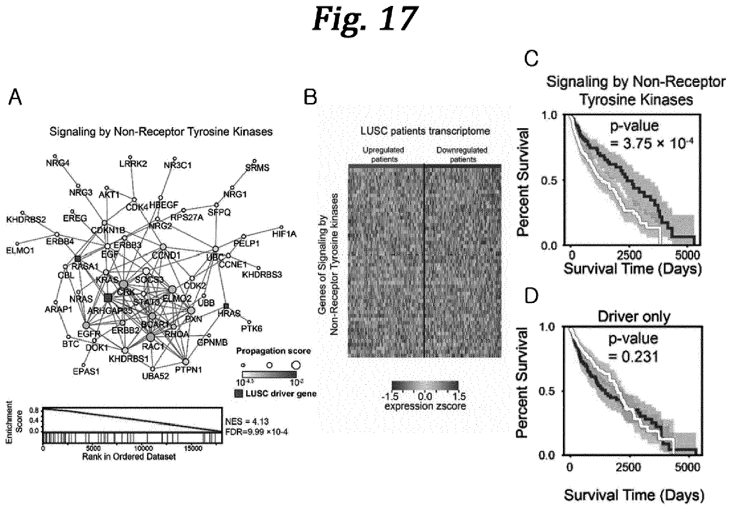

The co-essentiality links provided the best driver modules that stratified patients into groups with different survival rates in 12 of 16 cancer types. In contrast, the driver modules from other molecular networks failed to stratify patients in 13, 6, and 10 cancer types for PPI (BioGRID), co-expression, and co-methylation networks, respectively. For example, in LUSC, the pathway of Signaling by Non-Receptor Tyrosine Kinases (R-HSA-9006927), which is significantly proximal to driver genes in the co-essentiality network (normalized enrichment score [NES] = 4.13, false discovery rate [FDR] = $9.99 \times 10^{-4}$; **Fig. 17A**), can stratify patients with its down-regulation, exhibiting longer overall survival than the others (p-value = $3.75 \times 10^{-4}$, log-rank test; **Fig. 17B, Fig. 17C**). In contrast, co-expression and co-methylation links lined up different driver modules, Ovarian tumor domain proteases (R-HSA-5689896) and Signaling by FGFR2 in disease (R-HSA-5655253), respectively, and failed to differentiate the overall survival of patients (p-value = 0.0255, co-expression; p-value = 0.314, co-methylation, **Fig. 14, Fig. 15**). PPIs provided no driver module because no biological pathway was significantly proximal to driver genes **(Fig. 13).**

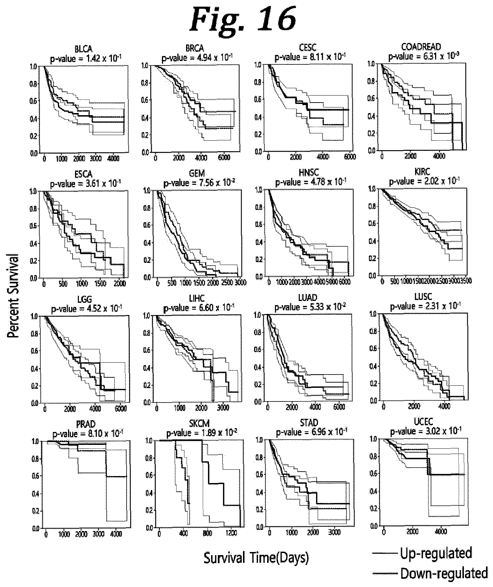

The present inventors also found that driver modules based on co-essentiality links were more informative for patient survival than the driver genes themselves. The driver genes alone, without module identification using co-essentiality links, were unable to differentiate survival outcomes in 15 out of 16 cancer types **(bottom line of Fig. 3I, Figure 16).** In LUSC, for example, the patient groups stratified by expression levels of the driver module using co-essentiality links, Non-Receptor Tyrosine Kinases pathway, showed a significant difference in the overall survival (**Fig. 17C,** p-value = $3.75 \times 10^{-4}$); in contrast, the patient groups distinguished by the expression level of driver genes themselves showed no significant difference in overall survival (**Fig. 17D,** [LUSC]; p-value = 0.231).

**Example 6. Co-essentiality network can prioritize therapeutic targets of specific cancer types.**

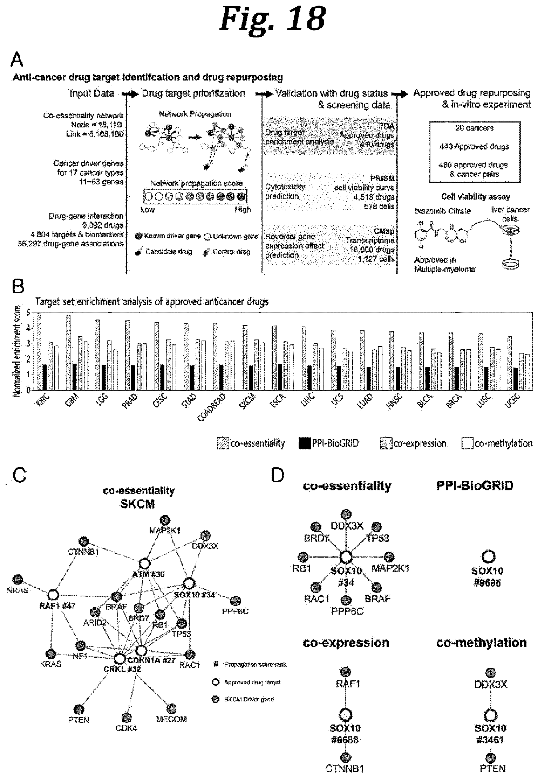

To explore the therapeutic use of the co-essentiality network, the present inventors examined the potential of co-essentiality links for drug repurposing tasks in cancer. Using network propagation, the present inventors examined genes closely located to driver genes in the co-essentiality network, which can be the targets of anticancer drugs, and utilized them for repurposing of approved drugs **(Fig. 18A).** The present inventors verified the performance of the co-essentiality network in identifying anticancer targets by three ways: (a) prioritization of FDA-approved drug targets; (b) prediction of cytotoxicity of drugs on cancer cells ($IC_{50}$); (c) prediction of reversal gene expression (RGE) effect of drugs on cancer cells. Finally, the present inventors experimentally validated ixazomib citrate, one of the repurposing candidates for liver hepatocellular carcinoma (LIHC) predicted by co-essentiality links, using cell viability assay in human LIHC cell lines.

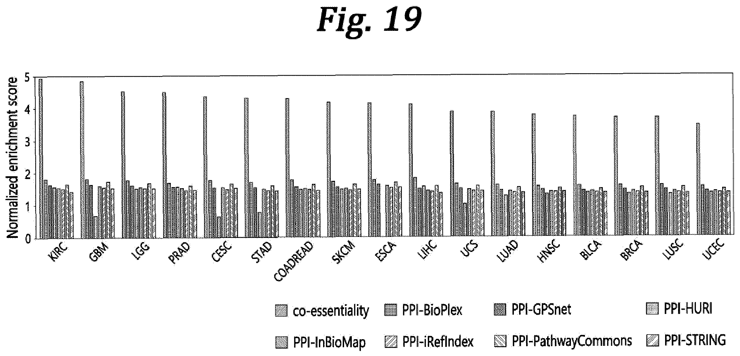

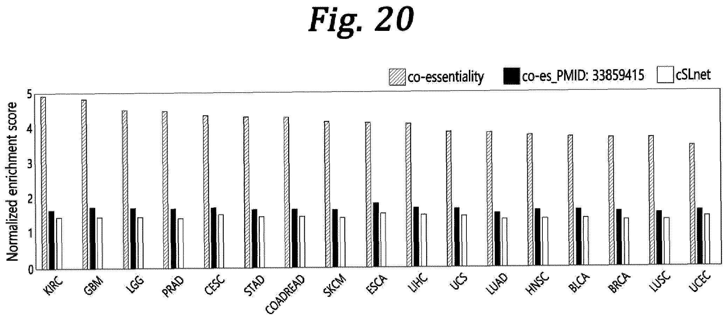

Network propagation with co-essentiality links better prioritized target genes of FDA-approved anticancer drugs compared with 12 other networks **(Fig. 18B, Fig. 19, Fig. 20).** Among the 17 cancer types examined, propagation values using co-essentiality links showed the most positive enrichment for the targets of FDA-approved anticancer drugs. In this analysis, the present inventors used both "direct target" and "biomarker" of drugs as drug targets. The average normalized enrichment score (NES) of co-essentiality links was 4.14, 39% higher than that of co-expression links that had the second-highest NES **(Fig. 18B).** Having greater NES to propagation values of co-essentiality links suggests that the co-essentiality network prioritizes targets of anticancer drugs better. For example, in skin cutaneous melanoma (SKCM), the co-essentiality network had an NES value of 4.18, which was higher than those of PPI-BioGRID, co-expression network, and co-methylation network (1.59, 3.25, and 3.08 respectively). Among the top 50 genes with the highest propagation value from the co-essentiality network, 19 were identified as approved targets, which were higher than the number of PPI-BioGRID, co-expression network, and co-methylation network (17, 14, and 14 genes in the top 50 genes with the highest propagation value, respectively). Among these 19 genes, 5 (*CDKN1A, ATM, CRKL, SOX10,* and *RAF1*) were detected since they had several connections with SKCM driver genes used as inputs for propagation **(Fig. 18C).** Specifically, Dabrafenib, an FDA-approved drug for melanoma, is known to inhibit the RAF proto-oncogene serine/threonine-protein kinase (*RAF1*). In the co-essentiality network, *RAF1* showed a high propagation value ($1.37 \times 10^{-4}$ and ranked 47th) because RAF1 is linked to five known SKCM driver genes: *CTNNB1, NRAS, BRAF, NF1,* and *KRAS.*

The present inventors observed that co-essentiality links could identify the approved drug targets that were not captured by other molecular links. For example, in SKCM, a Vemurafenib target, *SOX10*, ranked 34th in the co-essentiality network, but 9,695th in the PPI network, 6,688th in the co-expression links, and 3,461st in the co-methylation network **(Fig. 18D).** Co-essentiality network can identify more interactions between *SOX10* and known driver genes of SKCM that are not captured in other molecular links. *SOX10* has co-essentiality links with seven driver genes of SKCM: *TP53,*

*MAP2K1, BRAF, PPP6C, RAC1, RB1,* and *BRD7,* which are not connected to other molecular networks. This suggested that co-essentiality links could help to facilitate the identification of new therapeutic targets which are not covered by other molecular links.

**Example 7. Co-essentiality network finds candidates for drug repurposing.**

Having confirmed the capability of co-essentiality links to find targets of approved drugs, the present inventors further investigated whether they could facilitate network-based prediction of drug responses assessed in large-scale pharmacogenomic screenings. For each drug, the present inventors assigned a therapeutic candidate (TC) score aggregated from the propagation value of its targets by taking their root mean square (RMS). Therefore, the present inventors considered the average effect of drug targets on specific cancer types to eliminate bias to the number of drug targets.

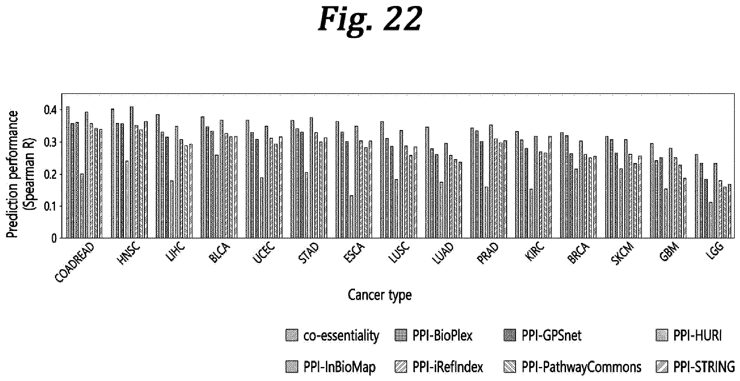

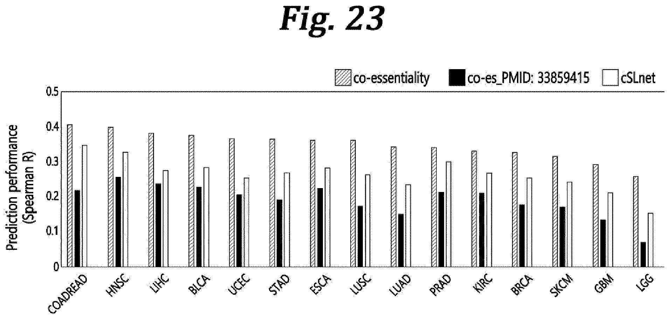

The present inventors found that the TC score calculated from co-essentiality links predicted the cytotoxicity of drugs for cancer cells better than the other molecular links. For each cancer type, drugs with a high TC score from the co-essentiality links were highly cytotoxic to cancer cell lines from that cancer type. Co-essentiality links showed the highest correlation between TC score and the $IC_{50}$ in 14 out of 15 cancer types which have drug screen data **(Fig. 21A),** and the average increase in the Spearman correlation coefficient (Spearman R) was 9.8%, 8.8%, and 43% for the PPI, co-expression, and co-methylation links, respectively. For example, TAK-733, an MEK1/2 inhibitor under clinical trials for advanced non-hematologic malignancies and advanced metastatic melanoma [60,61], was predicted to have an antitumor effect on colorectal cancer (COADREAD; **Fig. 21B;** TC score = $5.39 \times 10^{-3}$, rank percentile = 98.57%), and indeed showed high cytotoxicity in the cancer cell lines of COADREAD (median $IC_{50}$ = $1.03 \times 10^{-3}$ μmo!). In the co-essentiality network, TAK-733 had seven targets, *MAP2K1, MAP2K2, BRAF, KRAS, NRAS, PIK3CA,* and *GNA11* **(Fig. 21C, thick circles),** which are interconnected by nine driver genes of colorectal cancer, namely *CTNNB1, SOX9, TP53, APC, TCF7L2, TGF1, FBXW7, PTEN,* and *ARID1A* **(Fig. 21C, gray filled).** Similarly, the co-essentiality network showed a higher correlation between TC score and $IC_{50}$ in 12 out of 15 cancer types than seven other PPI networks **(Fig. 22).** Also, the co-essentiality network showed a higher correlation than another co-essentiality network and genetic interaction network across all 15 cancer types **(Fig. 23).**

One might ask whether driver genes alone, without the assistance of network propagation, can predict drug responses. The present inventors found that the driver genes alone were not as predictive as the TC score of the co-essentiality network **(Fig. 21A, driver only).** The TC score showed a greater correlation with $IC_{50}$ for all 17 cancer types than driver genes, and the increase in the Spearman correlation coefficient was 42%. This is because driver genes cover only 4.4% of drug targets, whereas using network propagation, all the genes in networks were assigned propagation scores.

Focusing on COADREAD, as it showed the greatest correlation between TC score and $IC_{50}$, the present inventors further discovered that drugs with a high TC score could alter gene expression to revert to that in cancer conditions **(Fig. 21D).** The present inventors used the degree of reversal effect of the drugs on cancer-associated gene expression to confirm the validity of the TC score **(Fig. 21D).** The present inventors measured the reversal gene expression (RGE) effect, which takes the dot product of ranked differential expression between cancer and drug-treatment conditions **(Fig. 21D)** and investigated its association with the TC score. The present inventors observed that the TC score of the co-essentiality network was positively correlated with the RGE effect **(Fig. 21E;** Spearman R = 0.303, p-value = $1.98 \times 10^{-3}$). For example, TAK-733, which exhibited a high TC score and - $\log_{10}(IC_{50})$ in **Fig. 21B,** showed a strong reversal effect on the expression pattern (RGE effect = 0.123, rank percentile = 91.18 %). Additionally, the TC score from the co-essentiality network exhibited a greater correlation with the RGE effect than other molecular networks **(Fig. 21F).** Overall, these results indicate that the co-essentiality network is a better platform than any other molecular network for finding driver-associated candidates of drug targets, which holds potential for drug repurposing.

**Example 8. Experimental validation of drug repurposing using the co-essentiality network**

To identify the novel therapeutic potential of the approved drugs for other purposes, the present inventors conducted *in-silico* drug repurposing using the TC score from the co-essentiality network. Among a total of 1,702 approved drugs in Pandrugs that have known target information from publicly available databases, 333 were predicted to have high anticancer activities in at least one of the 17 cancer types. Specifically, 19 repurposed drugs were assigned new indications that were not covered by other networks **(Fig. 24A).** For example, a drug approved for endocrine diseases, Rosiglitazone maleate, may have new therapeutic potential for COADREAD.

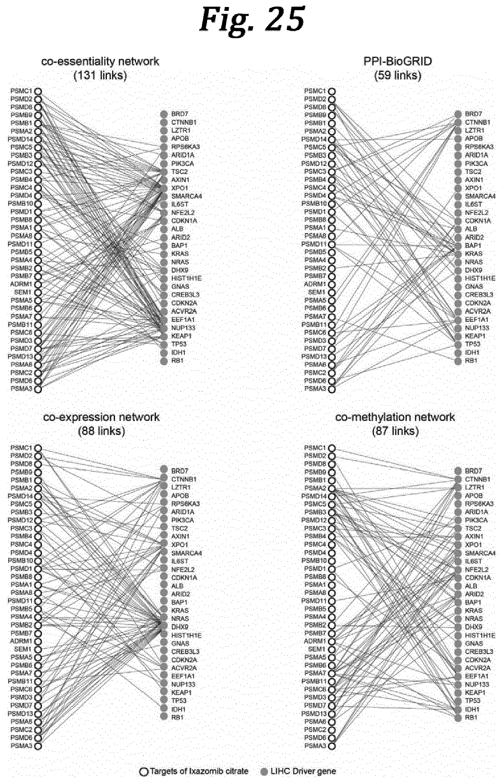

To examine the efficacy of *in-silico* drug repurosing using the co-essentiality network, the present inventors conducted experimental validation of ixazomib citrate approved for acute myeloma in LIHC. In LIHC, the present inventors found five repurposing candidates predicted by the co-essentiality network but not by other network to validate benefit of co-essentiality network. Among five repurposing candidates, ixazomib citrate with the highest TC score was selected to experimental validation. (**Fig. 24B**; TC score = $8.38 \times 10^{-5}$, rank percentile = 92.4% in the co-essentiality network). In the co-essentiality network, the 38 drug targets of ixazomib citrate had 131 co-essentiality links with 31 LIHC driver genes, which formed a denser module than the other molecular networks (**Fig. 24C, Fig. 25;** 59 links in the PPI-BioGRID network, 88 links in the co-expression network, and 87 links in the co-methylation network).

To test the anticancer activity of ixazomib citrate against LIHC, the present inventors performed an MTS assay to investigate the viability of four different cell lines: SNU398, SK-HEP-1, Huh7, and HepG2. ixazomib citrate showed potential antitumor effects in these LIHC cell lines **(Fig. 24D).** Specifically, ixazomib citrate was cytotoxic to all tested LIHC cells in the micromolar ($\mu$M) range: $IC_{50}$ = 137 nM in Huh7 cells, $IC_{50}$ = 197 nM in HepG2 cells, $IC_{50}$ = 270 nM in SK-HEP-1 cells, and $IC_{50}$ = 746 nM in SNU398 cells. Furthermore, a prolonged colony formation assay was performed to determine whether ixazomib citrate caused irreversible growth arrest **(Fig. 24E, left).** ixazomib citrate significantly impaired the growth of four LIHC cell lines, as demonstrated by a decrease in both colony number and colony size in the ixazomib citrate-treated group **(Fig. 24E, right).** This suggests repurposing candidates using network propagation with co-essentiality links can provide new therapeutic options.

[Reference]

1. Kanehisa M, Furumichi M, Tanabe M, Sato Y, Morishima K. KEGG: new perspectives on genomes, pathways, diseases and drugs. Nucleic Acids Res [Internet]. 2017 [cited 2022 Aug 4];45:D353-61. Available from: https://academic.oup.com/nar/article/45/D1/D353/2605697

2. Jassal B, Matthews L, Viteri G, Gong C, Lorente P, Fabregat A, et al. The reactome pathway knowledgebase. Nucleic Acids Res [Internet]. 2019 [cited 2022 Aug 4];48:D498-503. Available from: https://academic.oup.com/nar/article/48/D1/D498/5613674

3. Ashburner M, Ball CA, Blake JA, Botstein D, Butler H, Cherry JM, et al. Gene Ontology: tool for the unification of biology. Nat Genet 2000 251 [Internet]. 2000 [cited 2022 Aug 4];25:25-9. Available from: https://www.nature.com/articles/ng0500_25

4. Liberzon A, Subramanian A, Pinchback R, Thorvaldsdóttir H, Tamayo P, MesirovJP. Molecular signatures database (MSigDB) 3.0. Bioinformatics. 2011;27:1739-40.

5. Ruepp A, Waegele B, Lechner M, Brauner B, Dunger-Kaltenbach I, Fobo G, et al. CORUM: the comprehensive resource of mammalian protein complexes-2009. Nucleic Acids Res [Internet]. 2010 [cited 2018 Dec 14];38:D497-501. Available from: http://www.ncbi.nlm.nih.gov/pubmed/19884131

6. Lee I, Blom UM, Wang PI, Shim JE, Marcotte EM. Prioritizing candidate disease genes by network-based boosting of genome-wide association data. Genome Res [Internet]. 2011 [cited 2022 Aug 8];21:1109-21. Available from: https://genome.cshlp.org/content/21/7/1109.full

7. Guney E, Menche J, Vidal M, Barábasi AL. Network-based in silico drug efficacy screening. Nat Commun 2016 71 [Internet]. 2016 [cited 2022 Jun 30];7:1-13. Available from: https://www.nature.com/articles/ncomms10331

8. McDonald ER, de Week A, Schlabach MR, Billy E, Mavrakis KJ, Hoffman GR, et al. Project DRIVE: A Compendium of Cancer Dependencies and Synthetic Lethal Relationships Uncovered by Large-Scale, Deep RNAi Screening. Cell [Internet]. 2017 [cited 2019 Apr 12];170:577-592.e10. Available from: https://www.sciencedirect.com/science/article/pii/S0092867417308127

9. Lamb J, Crawford ED, Peck D, Modell JW, Blat IC, Wrobel MJ, et al. The connectivity map: Using gene-expression signatures to connect small molecules, genes, and disease. Science (80- ) [Internet]. 2006 [cited 2022 Aug 5];313:1929-35. Available from: https://www.science.org/doi/10.1126/science.1132939

10. Wang Z, Monteiro CD, Jagodnik KM, Fernandez NF, Gundersen GW, Rouillard AD, et al. Extraction and analysis of signatures from the Gene Expression Omnibus by the crowd. Nat Commun 2016 71 [Internet]. 2016 [cited 2022 Aug 5];7:1-11. Available from: https://www.nature.com/articles/ncomms12846

11. Zhang S-D, Gant TW. A simple and robust method for connecting small-molecule drugs using gene-expression signatures. BMC Bioinformatics [Internet]. 2008 [cited 2022 Aug 5];9:258. Available from: /pmc/articles/PMC2464610/

12. Pineiro-Yanez E, Reboiro-Jato M, Gomez-Lopez G, Perales-Patón J, Troule K, Rodriguez JM, et al. PanDrugs: A novel method to prioritize anticancer drug treatments according to individual genomic data. Genome Med [Internet]. 2018 [cited 2020 Jul 7];10:41. Available from: https://genomemedicine.biomedcentral.com/articles/10.1186/s13073-018-0546-1

13. Huttlin EL, Bruckner RJ, Navarrete-Perea J, Cannon JR, Baltier K, Gebreab F, et al. Dual Proteome-scale Networks Reveal Cell-specific Remodeling of the Human Interactome. bioRxiv [Internet]. 2020 [cited 2020 Jul 7];2020.01.19.905109. Available from: https://www.biorxiv.org/content/biorxiv/early/2020/01/19/2020.01.19.905109.full.pdf

14. Cheng F, Lu W, Liu C, Fang J, Hou Y, Handy DE, et al. A genome-wide positioning systems network algorithm for in silico drug repurposing. Nat Commun [Internet]. 2019 [cited 2020 Jul 19];10:1-14. Available from: https://doi.org/10.1038/s41467-019-10744-6

15. Luck K, Kim DK, Lambourne L, Spirohn K, Begg BE, Bian W, et al. A reference map of the human binary protein interactome. Nat 2020 5807803 [Internet]. 2020 [cited 2022 Aug 9];580:402-8. Available from: https://www.nature.com/articles/s41586-020-2188-x

16. Li T, Wernersson R, Hansen RB, Horn H, Mercer J, Slodkowicz G, et al. A scored human protein-protein interaction network to catalyze genomic interpretation. Nat Methods [Internet]. 2017 [cited 2018 Mar 11];14:61-4. Available from: http://www.nature.com/articles/nmeth.4083

17. Turner B, Razick S, Turinsky AL, Vlasblom J, Crowdy EK, Cho E, et al. iRefWeb: interactive analysis of consolidated protein interaction data and their supporting evidence. Database [Internet]. 2010 [cited 2022 Aug 9];2010. Available from: https://academic.oup.com/database/article/doi/10.1093/database/baq023/407721

18. Cerami EG, Gross BE, Demir E, Rodchenkov I, Babur O, Anwar N, et al. Pathway Commons, a web resource for biological pathway data. Nucleic Acids Res [Internet]. 2011 [cited 2019 Jan 28];39:D685-90. Available from: http://www.ncbi.nlm.nih.gov/pubmed/21071392

19. Szklarczyk D, Gable AL, Lyon D, Junge A, Wyder S, Huerta-Cepas J, et al. STRING v11: protein-protein association networks with increased coverage, supporting functional discovery in genome-wide experimental datasets. Nucleic Acids Res [Internet]. 2019 [cited 2019 Jun 14];47:D607-13. Available from: https://academic.oup.com/nar/article/47/D1/D607/5198476

20. Wainberg M, Kamber RA, Balsubramani A, Meyers RM, Sinnott-Armstrong N, Homburg D, et al. A genome-wide atlas of co-essential modules assigns function to uncharacterized genes. Nat Genet 2021 535 [Internet]. 2021 [cited 2022 Jun 16];53:638-49. Available from: https://www.nature.com/articles/s41588-021-00840-z

21. Lee JS, Das A, Jerby-Arnon L, Arafeh R, Auslander N, Davidson M, et al. Harnessing synthetic lethality to predict the response to cancer treatment. Nat Commun [Internet]. 2018;9:1-12. Available from: http://dx.doi.org/10.1038/s41467-018-04647-1

22. Leiserson MDM, Vandin F, Wu HT, Dobson JR, Eldridge J V, Thomas JL, et al. Pan-cancer network analysis identifies combinations of rare somatic mutations across pathways and protein complexes. Nat Genet [Internet]. 2015 [cited 2018 Nov 27];47:106-14. Available from: http://www.nature.com/articles/ng.3168

23. Hristov BH, Chazelle B, Singh M. uKIN Combines New and Prior Information with Guided Network Propagation to Accurately Identify Disease Genes. Cell Syst [Internet]. 2020 [cited 2020 Aug 11];10:470-479.e3. Available from: https://doi.org/10.1016/j.cels.2020.05.008

**Claims**

1. A method of drawing novel anticancer using co-essentiality network by a computing device, comprising:

(1) a process of collecting gene genome data, and constructing co-essentiality network by measuring similarity between genes in the gene genome data;

(2) a process of extracting cancer-related driver module from the co-essentiality network; and

(3) a process of drawing novel anticancer using the cancer-related driver module.

2. The method of Claim 1,
wherein the process (1) comprises:
a process of calculating PCC(Pearson Correlation Coefficient) between a pair of genes in the gene genome data, and measuring the similarity by applying CLR(Context Likelihood Relatedness) algorithm to absolute value of the PCC.

3. The method of Claim 1,
wherein the process (2) comprises:
a process of conducting network propagation, which prioritizes genes in the network in an order associated with a cancer-related driver gene using a page-rank algorithm.

4. The method of Claim 3,
wherein the process (2) further comprises:
a process of identifying a biological pathway associated with the cancer-related driver gene using a network propagation score obtained through the network propagation.

5. The method of Claim 4,
wherein the process (2) further comprises:
a process of extracting a biological pathway, which satisfies FDR(False Discovery Rate) of < 0.001 and NES(Normalized Enrichment Score) of > 0, through GSEA(Gene Set Enrichment Analysis) from among the identified biological pathway and selecting the biological pathway as the driver module.

6. The method of Claim 5,
wherein the driver module is selected from among the biological pathway with lowest p-value through a log-rank test from among the extracted biological pathway.

7. The method of Claim 1,
wherein the novel anticancer includes a repurposed conventional drug.

8. A device of discovering novel anticancer using co-essentiality network by a computing device, comprising:

a data collecting unit configured to collect gene genome data;
a network constructing unit configured to construct co-essentiality network by measuring similarity between genes in the gene genome data;
a module extracting unit configured to extract cancer-related driver module from the co-essentiality network; and
an anticancer drawing unit configured to draw novel anticancer using the cancer-related driver module.

9. The device of claim 8,
wherein the network constructing unit is further configured to calculate PCC(Pearson Correlation Coefficient) between a pair of genes in the gene genome data, and measures the similarity by applying CLR(Context Likelihood Relatedness) algorithm to absolute value of the PCC.

10. The device of claim 8,
wherein the module extracting unit is further configured to conduct network propagation, which prioritizes genes in the network in an order associated with a cancer-related driver gene using a page-rank algorithm.

11. The device of claim 10,
wherein the module extracting unit is further configured to identify a biological pathway associated with the cancer-related driver gene using a network propagation score obtained through the network propagation.

12. The device of claim 11,
wherein the module extracting unit is further configured to extract a biological pathway, which satisfies FDR(False Discovery Rate) of < 0.001 and NES(Normalized Enrichment Score) of > 0, through GSEA(Gene Set Enrichment Analysis) from among the identified biological pathway, and select the biological pathway as the driver module.

13. The device of claim 12,
    wherein the driver module is selected from among the biological pathway with lowest p-value through a log-rank test from among the extracted biological pathway.

14. The device of claim 8,
    wherein the novel anticancer includes a repurposed conventional drug.

# Fig. 1

**A** Construction of co-essentiality network

**B**

**D**

**C**

*Fig. 2*

# Fig. 3

Fig. 4

## Fig. 5

## Fig. 6

## Fig. 7

EP 4 386 759 A1

# Fig. 8

*Fig. 9*

## Fig. 10

A

**Network propagation-based (HotNet2)**

Input: TCGA patient mutation

High

Low

Mutation
frequency

Propagation

Driver
module

B

Rank of MCC

Number of
1st & 2nd rank

co-essentiality
PPI-BioGRID
co-expression
co-methylation
PPI-BioPlex
PPI-GPSnet
PPI-HURI
PPI-InBioMap
PPI-iRefIndex
PPI-PathwayCommons
PPI-STRING
co-es_PMID: 33859415
cSLnet

BLCA LUSC LAML BRCA GBM HNSC OV UCEC COADREAD KIRC LUAD

0  4  8  12

Prediction performance rank

■ 1st rank    ■ 2nd rank

□ 3rd, ..., 11th rank

*Fig. 11*

EP 4 386 759 A1

# Fig. 12

Percent Survival

Survival Time(Days)

——— Up-regulated
——— Down-regulated

## Fig. 13

# Fig. 14

# Fig. 15

## Fig. 16

Percent Survival

Survival Time(Days)

—— Up-regulated
—— Down-regulated

*Fig. 17*

# Fig. 18

## A

**Anti-cancer drug target identifcation and drug repurposing**

## B

Target set enrichment analysis of approved anticancer drugs

co-essentiality    ■ PPI-BioGRID    co-expression    □ co-methylation

## C

co-essentiality
**SKCM**

\# Propagation score rank
O Approved drug target
● SKCM Driver gene

## D

**co-essentiality**      **PPI-BioGRID**

**co-expression**      **co-methylation**

## Fig. 19

## Fig. 20

# *Fig. 21*

A

Correlation between therapeutic candidate(TC) score & drug response(IC₅₀)

co-essentiality · PPI-BioPlex · co-expression · co-methylation · driver only

B

Colorectal cancer (COADREAD)

spearman r = 0.410
p-value = 3.80 x 10⁻²⁶

TAK-733

C

Targets of TAK-733
COADREAD Driver gene

D

Correlation between therapeutic candidate(TC) score & Reversal gene expression(RGE) effect

Differential expressed genes in COADREAD

Gene expression perturbation signatures (CMap)

Tumor

Normal tissue

Reversal gene expression (RGE) effect

E

Colorectal cancer (COADREAD)

spearman r = 0.303
p-value = 1.98 x 10⁻³

TAK-733

F

co-essentiality
PPI-BioGRID
co-expression
co-methylation

*Fig. 22*

## Fig. 23

# Fig. 24

**A**
**Drug repurposing map**

**B**
**Cell-viability screening on candidate drugs for liver cancer(LIHC)**

Ixazomib Citrate

Approved in Multiple-myeloma

co-essentiality 92.4%
PPI-BioGRID 78.2%
co-expression 67.9%
co-methylation 81.3%

50   100
Rank of TC score(%)

**C**

○ Targets of Ixazomib citrate
● LIHC Driver gene

**D**
**Dose-response curve**

**E**
**Prolonged colony formation assay**

| | SNU-398 | Huh7 | SK-Hep-1 | HegG2 |
|---|---|---|---|---|
| | 746nM | 137nM | 270nM | 197nM |

Drug dose   Vehicle   1um   3.16um

SNU-398

Huh7

SK-Hep-1

HegG2

* Colony stained with crystal violet

# Fig. 25

co-essentiality network
(131 links)

PPI-BioGRID
(59 links)

co-expression network
(88 links)

co-methylation network
(87 links)

○ Targets of Ixazomib citrate  ● LIHC Driver gene

# Fig. 26

START

Collect gene genome data — S2610

Construct co-essentiality network by measuring
similarity between genes in the gene genome data — S2620

Extract cancer-related driver module
from the co-essentiality network — S2630

Drawing novel anticancer using
the cancer-related driver module — S2640

END

# Fig. 27

27

2710  Bus  2720

| Data collecting unit | | Network contucting unit |

2730  2740

| Module extracing unit | | Anticancer drawing unit |

Europäisches
Patentamt

European
Patent Office

Office européen
des brevets

# EUROPEAN SEARCH REPORT

Application Number

EP 23 21 6667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KIM EIRU ET AL: "A network of human functional gene interactions from knockout fitness screens in cancer cells", LIFE SCIENCE ALLIANCE, vol. 2, no. 2, 12 April 2019 (2019-04-12), XP093143739, US ISSN: 2575-1077, DOI: 10.26508/lsa.201800278 * whole document, in particular abstract; Materials and methods; Figures; Results and Discussion * | 1-14 | INV. G16B5/00 G16B20/00 |
| X,D | WAINBERG MICHAEL ET AL: "A genome-wide atlas of co-essential modules assigns function to uncharacterized genes", NATURE GENETICS, NATURE PUBLISHING GROUP US, NEW YORK, vol. 53, no. 5, 15 April 2021 (2021-04-15), pages 638-649, XP037448434, ISSN: 1061-4036, DOI: 10.1038/S41588-021-00840-Z [retrieved on 2021-04-15] * whole document, in particular paragraph " Identification of cancer-type-specific pathway dependencies" on page 646; Figures * | 1-14 | |
| | ----- | | |
| | -/-- | | TECHNICAL FIELDS SEARCHED (IPC) G16B |

The present search report has been drawn up for all claims

| Place of search | Date of completion of the search | Examiner |
|---|---|---|
| Munich | 22 March 2024 | Vanmontfort, D |

EPO FORM 1503 03.82 (P04C01)

**page 1 of 2**

Europäisches
Patentamt

European
Patent Office

Office européen
des brevets

**EUROPEAN SEARCH REPORT**

Application Number

EP 23 21 6667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Hart Traver ET AL: "Coessentiality and cofunctionality: a network approach to learning genetic vulnerabilities from cancer cell line fitness screens", bioRxiv, 4 May 2017 (2017-05-04), XP093143755, DOI: 10.1101/134346 Retrieved from the Internet: URL:https://www.biorxiv.org/content/10.1101/134346v1.full.pdf [retrieved on 2024-03-20] * whole document, in particular abstract; Methods and Figures; last paragraph of introduction; paragraphs "Generating the coessentiality network" and "Clusters in the coessentiality network are defined by distinct cell types" and "Core essentials are more sensitive to perturbation at lower expression levels" of Results section * | 1-14 | |
| X | AMICI DAVID R ET AL: "FIREWORKS: a bottom-up approach to integrative coessentiality network analysis", LIFE SCIENCE ALLIANCE, vol. 4, no. 2, 16 December 2020 (2020-12-16), XP093143738, US ISSN: 2575-1077, DOI: 10.26508/lsa.202000882 * whole document, in particular abstract; Materials and methods; Results and Figures * | 1-14 | TECHNICAL FIELDS SEARCHED (IPC) |

The present search report has been drawn up for all claims

| Place of search | Date of completion of the search | Examiner |
|---|---|---|
| Munich | 22 March 2024 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 111128299

- CN 110473591

**Non-patent literature cited in the description**

- **KANEHISA M ; FURUMICHI M ; TANABE M ; SATO Y ; MORISHIMA K.** KEGG: new perspectives on genomes, pathways, diseases and drugs. *Nucleic Acids Res,* 2017, vol. 45, D353-61, https://academic.oup.com/nar/article/45/D1/D353/2605697

- **JASSAL B ; MATTHEWS L ; VITERI G ; GONG C ; LORENTE P ; FABREGAT A et al.** The reactome pathway knowledgebase. *Nucleic Acids Res,* 2019, vol. 48, D498-503, https://academic.oup.com/nar/article/48/D1/D498/5613674

- **ASHBURNER M ; BALL CA ; BLAKE JA ; BOTSTEIN D ; BUTLER H ; CHERRY JM et al.** Gene Ontology: tool for the unification of biology. *Nat Genet,* 2000, vol. 25, 25-9, https://www.nature.com/articles/ng0500_25

- **LIBERZON A ; SUBRAMANIAN A ; PINCHBACK R ; THORVALDSDÓTTIR H ; TAMAYO P ; MESIROV JP.** Molecular signatures database (MSigDB) 3.0. *Bioinformatics.,* 2011, vol. 27, 1739-40

- **RUEPP A ; WAEGELE B ; LECHNER M ; BRAUNER B ; DUNGER-KALTENBACH I ; FOBO G et al.** CORUM: the comprehensive resource of mammalian protein complexes-2009. *Nucleic Acids Res,* 2010, vol. 38, D497-501, http://www.ncbi.nlm.nih.gov/pubmed/19884131

- **LEE I ; BLOM UM ; WANG PI ; SHIM JE ; MARCOTTE EM.** Prioritizing candidate disease genes by network-based boosting of genome-wide association data. *Genome Res,* 2011, vol. 21, 1109-21, https://genome.cshlp.org/content/21/7/1109.full

- **GUNEY E ; MENCHE J ; VIDAL M ; BARÁBASI AL.** Network-based in silico drug efficacy screening. *Nat Commun,* 2016, vol. 7, 1-13, https://www.nature.com/articles/ncomms10331

- **MCDONALD ER ; DE WEEK A ; SCHLABACH MR ; BILLY E ; MAVRAKIS KJ ; HOFFMAN GR et al.** Project DRIVE: A Compendium of Cancer Dependencies and Synthetic Lethal Relationships Uncovered by Large-Scale, Deep RNAi Screening. *Cell,* 2017, vol. 170, 577-592, https://www.sciencedirect.com/science/article/pii/S0092867417308127

- **LAMB J ; CRAWFORD ED ; PECK D ; MODELL JW ; BLAT IC ; WROBEL MJ et al.** The connectivity map: Using gene-expression signatures to connect small molecules, genes, and disease. *Science,* 2006, vol. 313, 1929-35, https://www.science.org/doi/10.1126/science.1132939

- **WANG Z ; MONTEIRO CD ; JAGODNIK KM ; FERNANDEZ NF ; GUNDERSEN GW ; ROUILLARD AD et al.** Extraction and analysis of signatures from the Gene Expression Omnibus by the crowd. *Nat Commun,* 2016, vol. 7, 1-11, https://www.nature.com/articles/ncomms12846

- **ZHANG S-D ; GANT TW.** *A simple and robust method for connecting small-molecule drugs using gene-expression signatures,* 2008, vol. 9, 258, BMC Bioinformatics

- **PINEIRO-YANEZ E ; REBOIRO-JATO M ; GOMEZ-LOPEZ G ; PERALES-PATÓN J ; TROULE K ; RODRIGUEZ JM et al.** PanDrugs: A novel method to prioritize anticancer drug treatments according to individual genomic data. *Genome Med,* 2018, vol. 10, 41, https://genomemedicine.biomedcentral.com/articles/10.1186/s13073-018-0546-1

- **HUTTLIN EL ; BRUCKNER RJ ; NAVARRETE-PEREA J ; CANNON JR ; BALTIER K ; GEBREAB F et al.** Dual Proteome-scale Networks Reveal Cell-specific Remodeling of the Human Interactome. *bioRxiv,* 07 July 2020, https://www.biorxiv.org/content/biorxiv/early/2020/01/19/2020.01.19.905109.full.pdf

- **CHENG F ; LU W ; LIU C ; FANG J ; HOU Y ; HANDY DE et al.** A genome-wide positioning systems network algorithm for in silico drug repurposing. *Nat Commun,* 2019, vol. 10, 1-14, https://doi.org/10.1038/s41467-019-10744-6

- **LUCK K ; KIM DK ; LAMBOURNE L ; SPIROHN K ; BEGG BE ; BIAN W et al.** A reference map of the human binary protein interactome. *Nat,* 09 August 2022, vol. 580, 402-8, https://www.nature.com/articles/s41586-020-2188-x

- **LI T ; WERNERSSON R ; HANSEN RB ; HORN H ; MERCER J ; SLODKOWICZ G et al.** A scored human protein-protein interaction network to catalyze genomic interpretation. *Nat Methods,* 2017, vol. 14, 61-4, http://www.nature.com/articles/nmeth.4083
- **TURNER B ; RAZICK S ; TURINSKY AL ; VLASBLOM J ; CROWDY EK ; CHO E et al.** iRefWeb: interactive analysis of consolidated protein interaction data and their supporting evidence. *Database,* 2010, https://academic.oup.com/database/article/doi/10.1093/database/baq023/407721
- **CERAMI EG ; GROSS BE ; DEMIR E ; RODCHENKOV I ; BABUR O ; ANWAR N et al.** Pathway Commons, a web resource for biological pathway data. *Nucleic Acids Res,* 2011, vol. 39, D685-90, http://www.ncbi.nlm.nih.gov/pubmed/21071392
- **SZKLARCZYK D ; GABLE AL ; LYON D ; JUNGE A ; WYDER S ; HUERTA-CEPAS J et al.** STRING v11: protein-protein association networks with increased coverage, supporting functional discovery in genome-wide experimental datasets. *Nucleic Acids Res,* 14 June 2019, vol. 47, D607-13, https://academic.oup.com/nar/article/47/D1/D607/5198476
- **WAINBERG M ; KAMBER RA ; BALSUBRAMANI A ; MEYERS RM ; SINNOTT-ARMSTRONG N ; HOMBURG D et al.** A genome-wide atlas of co-essential modules assigns function to uncharacterized genes. *Nat Genet,* 2021, vol. 53, 638-49, https://www.nature.com/articles/s41588-021-00840-z
- **LEE JS ; DAS A ; JERBY-ARNON L ; ARAFEH R ; AUSLANDER N ; DAVIDSON M et al.** Harnessing synthetic lethality to predict the response to cancer treatment. *Nat Commun,* 2018, vol. 9, 1-12, http://dx.doi.org/10.1038/s41467-018-04647-1
- **LEISERSON MDM ; VANDIN F ; WU HT ; DOBSON JR ; ELDRIDGE J V ; THOMAS JL et al.** Pan-cancer network analysis identifies combinations of rare somatic mutations across pathways and protein complexes. *Nat Genet,* 27 November 2018, vol. 47, 106-14, http://www.nature.com/articles/ng.3168
- **HRISTOV BH ; CHAZELLE B ; SINGH M.** uKIN Combines New and Prior Information with Guided Network Propagation to Accurately Identify Disease Genes. *Cell Syst,* 11 August 2020, vol. 10, 470-479, https://doi.org/10.1016/j.cels.2020.05.008